# EUROPÄISCHE PATENTSCHRIFT

(11) **EP 0 807 629 B1**
(45) Veröffentlichungstag und Bekanntmachung des Hinweises auf die Patenterteilung: **03.03.2004**
(21) Anmeldenummer: 97107775.5
(22) Anmeldetag: 13.05.1997
(51) Int. Cl.: C07D 311/68, A61K 31/35, C07D 311/70, C07D 405/12

(54) **Sulfonamid-substituierte Chromane, Verfahren zu ihrer Herstellung, ihre Verwendung als Medikament oder Diagnostikum sowie sie enthaltendes Medikament**
Sulfonamido substituted chromane derivatives, method for their preparation, their use as medicaments or diagnostic agents aswell as medicaments containing them
Dérivés sulfonamide de chromane, procédé pour leur préparation, leur utilisation comme médicament ou agent diagnostique insi que médicament les contenant

(30) Priorität: 15.05.1996 DE 19619614; 26.09.1996 DE 19639462
(43) Veröffentlichungstag der Anmeldung: 19.11.1997
(73) Patentinhaber: Aventis Pharma Deutschland GmbH, 65929 Frankfurt am Main (DE)
(72) Erfinder: Lang, Hans Jochem, Dr., 65719 Hofheim (DE); Gerlach, Uwe, Dr., 65795 Hattersheim (DE); Brendel, Joachim, Dr., 61118 Bad Vilbel (DE); Englert, Heinrich Christian, Dr., 65719 Hofheim (DE); Gögelein, Heinz, Dr., 60528 Frankurt (DE); Hropot, Max, Dr., 65439 Flörsheim (DE); Bohn, Helmut, Dr., 61137 Schöneck (DE); Herling, Andreas, Dr., 65520 Bad Camberg (DE); Busch, Andreas, Prof. Dr., 72076 Tübingen (DE); Greger, Rainer, Prof. Dr., 79423 Heitersheim (DE)

(56) Entgegenhaltungen:
- EP-A- 0 315 009
- EP-A- 0 370 901
- EP-A- 0 389 861
- PFLÜGERS ARCH. - EUR. J. PHYSIOL., Bd. 429, 1995, Seiten 517-530, XP002038768 LOHRMANN ET AL.: "A new class of inhibitors of cAMP-mediated Cl- secretion in rabbit colon, acting by the reduction of cAMP-activated K+ conductance"
- DRUG DEVELOPMENT RESEARCH, Bd. 33, 1994, Seiten 235-249, XP000567177 T.J. COLATSKY; T.M. ARGENTIERI: "Potassium Channel Blockers as Antiarrhytmic Drugs"

## Beschreibung

Die Erfindung betrifft Chromane der Formel I 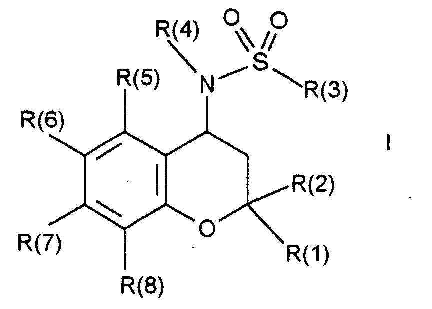 worin bedeuten:
R(1) und R(2)
unabhängig voneinander Wasserstoff, CₚF₂ₚ₊₁, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonylamino und Methylsulfonyl;
p 1, 2 oder 3;
oder
R(1) und R(2)
gemeinsam eine Alkylenkette mit 2, 3, 4, 5, 6, 7, 8, 9 oder 10 C-Atomen;
R(3) R(9)-CₙH₂ₙ[NR(11)]ₘ-;
R(9) Wasserstoff oder Cycloalkyl mit 3, 4, 5, 6, 7 oder 8 C-Atomen;
n Null, 1, 2, 3, 4, 5, 6, 7, 8, 9 oder 10;
m Null oder 1;
R(11) Wasserstoff oder Alkyl mit 1, 2, 3, 4, 5 oder 6 C-Atomen;
oder
R(11) gemeinsam mit R(9) eine Alkylengruppe mit 1, 2, 3, 4, 5, 6, 7 oder 8 C-Atomen;
wobei eine CH₂-Gruppe der Gruppe CₙH₂ₙ durch -O-, -SO_{q} oder -NR(10) ersetzt sein kann;
q Null, 1 oder 2;
R(10) Wasserstoff, Methyl oder Ethyl;
R(4) R(12)-CᵣH₂ᵣ;
R(12)
Wasserstoff, Cycloalkyl mit 3, 4, 5, 6, 7 oder 8 C-Atomen, Piperidyl, 1-Pyrrolidinyl, N-Morpholino, N-Methylpiperazino, CₚF₂ₚ₊₁, Pyridyl, Thienyl, Imidazolyl oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
r 1, 2, 3, 4, 5, 6, 7, 8, 9, 10, 11, 12, 13, 14, 15, 16, 17, 18, 19 oder 20;
wobei eine CH₂-Gruppe der Gruppe CᵣH₂ᵣ ersetzt sein kann durch -O-, -C=C-, -C=C-, -CO-, -CO-O-, -SO_{q}- oder -NR(10)-;
q Null, 1 oder 2;
R(10) Wasserstoff, Methyl oder Ethyl;
R(5), R(6), R(7) und R(8)
unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, -CN, -CF₃, -C₂F₅, -C₃F₇, -N₃, -NO₂, -CONR(13)R(14), -COOR(15), R(16)-CₛH₂ₛ-Y- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
R(13) und R(14)
unabhängig voneinander Wasserstoff oder Alkyl mit 1, 2 oder 3 C-Atomen;
R(15) Wasserstoff, Methyl, Ethyl; Phenyl oder -CᵤH₂ᵤ-NR(13)R(14);
u 2 oder 3;
R(16) Wasserstoff, Cycloalkyl mit 3, 4, 5, 6, 7 oder 8 C-Atomen, -COOR(15), Thienyl, Imidazolyl, Pyridyl, Chinolyl, Isochinolyl, Piperidyl, 1-Pyrrolidinyl, N-Morpholino, N-Methylpiperazino, CₜF₂ₜ₊₁ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl; .
s Null, 1, 2, 3, 4, 5 oder 6;
t 1, 2 oder 3;
Y SO_{q}, -CO-, -SO₂-NR(10)-, -O-, -NR(10)- oder -CO-NR(10);
wobei jedoch R(6) nicht -OCF₃ oder -OC₂F₅ sein kann;
und deren physiologisch verträgliche Salze.

Bevorzugt sind Verbindungen der Formel I, in denen bedeuten:
R(1) und R(2)
unabhängig voneinander Wasserstoff, CF₃, Alkyl mit 1, 2 oder 3 C-Atomen, gemeinsam eine Alkylenkette mit 4 oder 5 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
R(3) R(9)-CₙH₂ₙ[NR(11)]ₘ-;
R(9) Wasserstoff;
n Null, 1, 2, 3, 4, 5 oder 6;
m Null oder 1;
R(11) Wasserstoff oder Alkyl mit 1, 2, 3 oder 4 C-Atomen;
R(4) R(12)-CᵣH₂ᵣ;
R(12)
Wasserstoff, Cycloalkyl mit 5, 6, 7 oder 8 C-Atomen, CF₃, Pyridyl oder Phenyl,
das unsubstituiert ist oder substituiert mit 1 oder 2 Substituenten ausgewählt aus der Gruppe bestehend aus F, Cl, CF₃, Sulfamoyl oder Methylsulfonyl;
r 1, 2, 3, 4, 5, 6, 7, 8, 9 oder 10;
wobei eine CH₂-Gruppe der Gruppe CᵣH₂ᵣ ersetzt sein kann durch -O-, -CO-, -CO-O- oder -SO_{q}-;
q Null, 1 oder 2;
R(5), R(6), R(7) und R(8)
unabhängig voneinander Wasserstoff, F, Cl, Br, I, Alkyl mit 1 oder 2 C-Atomen, -CN, -CF₃, -NO₂, -CONR(13)R(14), -COOR(15), R(16)-CₛH₂ₛ-Y- oder Phenyl,
das unsubstituiert ist oder substituiert mit einem Substituenten ausgewählt aus der Gruppe bestehend aus F, Cl und CF₃;
R(13) und R(14)
unabhängig voneinander Wasserstoff oder Alkyl mit 1, 2 oder 3 C-Atomen;
R(15) Methyl, Ethyl, Phenyl oder -CᵤH₂ᵤ-NR(13)R(14);
u 2 oder 3;
R(16) Wasserstoff, Cycloalkyl mit 5 oder 6 C-Atomen, CₜF₂ₜ₊₁ oder Phenyl,
das unsubstituiert ist oder substituiert mit einem Substituenten ausgewählt aus der Gruppe bestehend aus F, Cl, Br, CF₃, Methyl, Methoxy, Sulfamoyl oder Methylsulfonyl;
t 1, 2 oder 3;
s Null, 1, 2, 3 oder 4;
Y SO_{q}, -CO-, -SO₂-NR(10)-, -O-, -NR(10)- oder -CO-NR(10);
q Null, 1 oder 2;
R(10) Wasserstoff oder Methyl;
wobei jedoch R(6) nicht -OCF₃ oder -OC₂F₅ sein kann;
und deren physiologisch verträgliche Salze.

Besonders bevorzugt sind Verbindungen der Formel I, in denen bedeuten:
R(1) und R(2)
unabhängig voneinander CF₃, Methyl oder Phenyl,
das unsubstituiert ist oder substituiert durch 1 oder 2 Substituenten ausgewählt aus der Gruppe bestehend aus F, Cl, Br, I, CF₃, Methyl, Methoxy, Sulfamoyl und Methylsulfonyl;
R(3) Alkyl mit 1, 2, 3 oder 4 C-Atomen, Dimethylamino oder Diethylamino;
R(4) R(12)-CᵣH₂ᵣ;
R(12)
Wasserstoff, Cycloalkyl mit 5 oder 6 C-Atomen oder CF₃;
r 1, 2, 3, 4, 5, 6, 7 oder 8;
wobei eine CH₂-Gruppe der Gruppe CᵣH₂ᵣ ersetzt sein kann durch -O-, -CO-, -CO-O- oder -SO_{q}-;
q Null, 1 oder 2;
R(5), R(6), R(7) und R(8)
unabhängig voneinander Wasserstoff, F, Cl, Br, I, Alkyl mit 1 oder 2 C-Atomen, -CN, -NO₂, -COOR(15), R(16)-CₛH₂ₛ-Y- oder Phenyl,
das unsubstituiert ist oder substituiert mit einem Substituenten ausgewählt aus der Gruppe bestehend aus F oder Cl;
R(15) Methyl, Ethyl, Phenyl oder -CᵤH₂ᵤ-NR(13)R(14);
u 2 oder 3;
R(13) und R(14)
unabhängig voneinander Wasserstoff oder Alkyl mit 1, 2 oder 3 C-Atomen;
R(16) Wasserstoff, CF₃ oder Phenyl,
s Null, 1, 2, 3 oder 4;
Y SO_{q}, -CO-, -SO₂-NR(10)-, -O-, -NR(10)- oder -CO-NR(10);
q Null, 1 oder 2;
R(10) Wasserstoff oder Methyl;
wobei jedoch R(6) nicht -OCF₃ sein kann; und deren physiologisch verträgliche Salze.

Ganz besonders bevorzugt sind die folgenden Verbindungen der Formel I:
4-(N-Ethylsulfonyl-N-methyl)amino-6-fluor-2,2-dimethylchroman, 6-Cyano-4-(N-ethylsulfonyl-N-methyl)amino-2,2-dimethylchroman, 4-(N-Ethylsulfonyl-N-methyl)amino-6-methoxycarbonyl-2,2-dimethylchroman, 6-Cyano-4-[N-ethylsulfonyl-N-(4,4,4-trifluorbutyl)]amino-2,2-dimethylchroman, 4-(N-Butyl-N-ethylsulfonyl)amino-6-cyano-2,2-dimethylchroman, 4-(N-Ethylsulfonyl-N-methyl)amino-2,2,6-trimethylchroman, 7-Chlor-4-(N-ethylsulfonyl-N-methyl)amino-6-fluor-2,2-dimethylchroman, 6,7-Dichlor-4-(N-ethylsulfonyl-N-methyl)amino-2,2-dimethylchroman, 4-(N-Butyl-N-ethylsulfonyl)amino-6-fluor-2,2-dimethylchroman, 4-(N-Ethylsulfonyl-N-methyl)amino-6-fluor-2,2-tetramethylenchroman, 4-[N-Ethylsulfonyl-N-(4,4,4-trifluorbutyl)]amino-6-fluor-2,2-dimethylchroman, 4-(N-Ethylsulfonyl-N-hexyl)amino-6-fluor-2,2-dimethylchroman, 6-Ethyl-4-[N-ethylsulfonyl-N-(4,4,4-trifluorbutyl)]amino-2,2-dimethylchroman, 4-(N-Ethoxycarbonylmethyl-N-ethylsulfonyl)amino-6-fluor-2,2-dimethylchroman, 4-(N-Ethylsulfonyl-N-methyl)amino-6-(4,4,4-trifluorbutyl)oxy-2,2-dimethylchroman, 6-(4-Bromophenyl)- 4-(N-ethylsulfonyl-N-methyl)amino-2,2-dimethylchroman, 4-[N-Ethylsulfonyl-N-(3-ethoxypropyl)]amino -2,2,6-trimethylchroman.
Enthalten die Verbindungen I eine saure oder basische Gruppe bzw. einen basischen Heterocyclus, so sind auch die entsprechenden, pharmakologisch und toxikologisch verträglichen Salze Gegenstand der Erfindung. So können die Verbindungen I, die eine oder mehrere -COOH-Gruppen tragen, beispielsweise als Alkalisalze, vorzugsweise als Natrium- oder Kaliumsalze verwendet werden. Verbindungen I, die eine basische, protonierbare Gruppe oder einen basischen heterocyclischen Rest tragen, können auch in Form ihrer organischen oder anorganischen, pharmakologisch und toxikologisch verträglichen Säureadditionssalze verwendet werden, beispielweise als Hydrochloride, Methansulfonate, Acetate, Lactate, Maleinate, Fumarate, Malate, Gluconate usw. Enthalten die Verbindungen I eine saure und basische Gruppe im gleichen Molekül, so gehören neben den geschilderten Salzformen auch innere Salze, sogenannte Betaine, zur der Erfindung.

Enthalten die Substituenten der Verbindungen der Formel I Gruppen mit unterschiedlichen stereochemischen Möglichkeiten, so gehören auch die einzelnen möglichen Stereoisomeren zur Erfindung. So enthalten die Verbindungen I ein chirales Zentrum in Position 4 des Chromansystems, so daß die einzelnen reinen optischen Antipoden sowie beliebige Stoffgemische der optischen Isomeren Teil der Erfindung sind.

Die Verbindungen der Formel I sind durch unterschiedliche chemische Verfahren herstellbar, die ebenfalls Teil der Erfindung sind.

So erhält man eine Verbindung der Formel I, indem man
a) eine Verbindung der Formel II 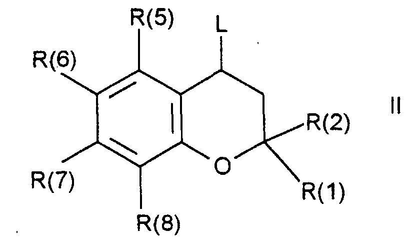 worin R(1), R(2), R(5), R(6), R(7) und R(8) die angegebene Bedeutung besitzen und L die für eine Alkylierung übliche nucleofuge Fluchtgruppe, insbesondere Cl, Br, I, MeSO₂-O-, einen p-Toluolsulfonyloxy-Rest, bedeutet,
   mit einem Sulfonamid oder dessen Salz der Formel III 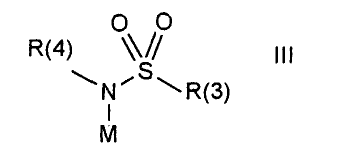 in an sich bekannter Weise umsetzt, worin R(3) und R(4) die angegebene Bedeutung besitzen, jedoch r im Substituenten R(4) auch die Bedeutung Null hat, und M für Wasserstoff oder vorzugsweise für ein Metallatom, besonders bevorzugt für Lithium, Natrium oder Kalium steht;
   oder daß man
b) eine Verbindung der Formel IV 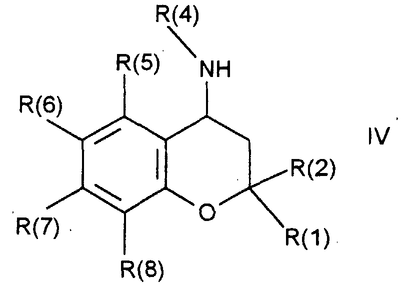 worin R(1), R(2), R(4), R(5), R(6), R(7) und R(8) die angegebene Bedeutung besitzen, jedoch r im Substituenten R(4) auch die Bedeutung Null hat,
   mit einem Sulfonsäure-Derivat der Formel V

   R(3) - SO₂-W V

   umsetzt, worin R(3) die angegebene Bedeutung besitzt und W eine nucleofuge Fluchtgruppe, wie Fluor, Brom, 1-Imidazolyl, insbesondere aber Chlor bedeutet;
   oder daß man
c) eine Verbindung der Formel VI 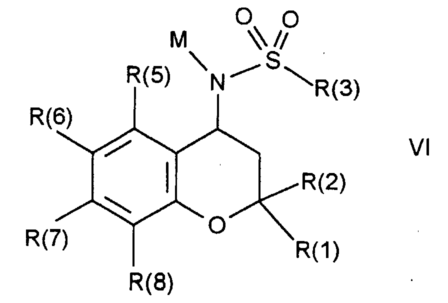 worin R(1), R(2), R(5), R(6), R(7), R(8) und M die angegebene Bedeutung besitzen, in an sich bekannter Weise mit einem Alkylierungsmittel der Formel VII

   R(4)-L VII

   im Sinne einer Alkylierungsreaktion umsetzt, worin R(4) mit Ausnahme von Wasserstoff sowie L die angegebene Bedeutung besitzen;
   oder daß man
d) in einer Verbindung der Formel I 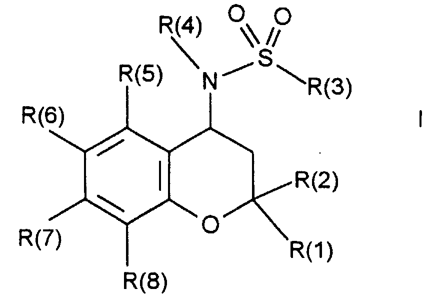
worin R(1) bis R(4) die angegebene Bedeutung besitzen, in mindestens einer Position R(5) bis R(8) eine elektrophile Substitutionsreaktion durchführt, sofern diese Position Wasserstoff bedeutet und die restlichen Substituenten R(5) bis R(8) die angegebene Bedeutung besitzen.
Verfahrensweise a)
   beschreibt die an sich bekannte Alkylierung eines Sulfonamids bzw. eines seiner Salze der Formel III durch ein alkylierend wirkendes Chromanderivat der Formel II. Da die Alkylierung eines Sulfonamides aus der Salzform heraus erfolgt, muß bei Verwendung eines freien Sulfonamids (Formel III, M = H) durch Einwirkung einer Base ein Sulfonamidsalz (Formel III, M = Kation) erzeugt werden, das sich durch höhere Nucleophilie und damit durch höhere Reaktivität auszeichnet. Setzt man freies Sulfonamid (M = H) ein, so erfolgt die Deprotonierung des Sulfonamids zum Salz in situ unter bevorzugter Verwendung solcher Basen, die selbst nicht oder nur wenig alkyliert werden, wie Natriumcarbonat, Kaliumcarbonat, ein sterisch stark gehindertes Amin, z. B. Dicyclohexylamin, N,N,N-Dicyclohexyl-ethylamin oder andere starke Stickstoffbasen mit geringer Nucleophilie, beispielsweise DBU, N,N',N"'-Triisopropylguanidin etc. Es können allerdings auch andere üblich verwendete Basen für die Reaktion eingesetzt werden, wie Kalium-tert.butylat, Natriummethylat, Alkalihydrogencarbonate, Alkalihydroxide, wie beispielweise LiOH, NaOH oder KOH, oder Alkalihydroxide, beispielsweise Ca(OH)₂.
   Dabei arbeitet man vorzugsweise in aprotischen polaren Lösungsmitteln wie Dimethylformamid, Dimethylacetamid, Tetramethylharnstoff, Hexamethylphosphorsäuretriamid, Tetrahydrofuran usw. Prinzipiell kann aber auch in polaren protischen Lösungsmitteln gearbeitet werden, wie Wasser, Methanol, Ethanol, Isopropanol, Ethylenglycol oder dessen Oligomeren und deren entsprechenden Halbethern und Ethern. Die Reaktion wird in einem bevorzugten Temperaturbereich von 20 bis 140°C, besonders bevorzugt von 40 bis 100°C durchgeführt. In günstiger Weise kann Verfahrensweise a) auch unter den Bedingungen einer Zweiphasenkatalyse durchgeführt werden. Die Verbindungen der Formel 11 gewinnt man nach literaturbekannten Methoden, beispielsweise aus den entsprechenden Alkoholen (Formel III, L = -OH) durch Einwirkung von Halogenwasserstoff HL (L = Cl, Br, I) oder durch Einwirkung eines anorganischen Säurehalogenids (POCl₃, PCl₃, PCl₅, SOCl₂, SOBr₂) oder durch radikalische Halogenierung der entsprechenden Chroman-Derivate (Formel II, L = H) mit elementarem Chlor oder Brom, oder mit radikalisch aktivierbaren Halogenierungsmitteln wie N-Bromsuccinimid (NBS) oder SO₂Cl₂ (Sulfurylchlorid) in Gegenwart eines Radikalkettenstarters wie energiereichem Licht des sichtbaren oder ultravioletten Wellenbereiches oder durch Verwendung eines chemischen Radikalstarters wie Azodiisobutyronitril.
Verfahrensweise b)
   beschreibt die an sich bekannte und häufig angewandte Reaktion einer aktivierten Sulfonylverbindung der Formel V, insbesondere einer Chlorsulfonylverbindung (W = Cl), mit einem Amin der allgemeinen Formel IV zum entsprechenden Sulfonamid-Derivat der allgemeinen Formel I. Die Reaktion kann prinzipiell ohne Lösungsmittel durchgeführt werden, jedoch werden derartige Reaktionen in den meisten Fällen unter Verwendung eines Lösungsmittels durchgeführt.
   Die Reaktionsführung geschieht vorzugsweise unter Verwendung eines polaren Lösungsmittels vorzugsweise in Gegenwart einer Base, die selbst als Lösungsmittel verwendet werden kann, z.B. bei Verwendung von Triethylamin, Pyridin und dessen Homologen. Vorzugsweise verwendete Lösungsmittel sind beispielsweise Wasser, aliphatische Alkohole, z.B. Methanol, Ethanol, Isopropanol, sek. Butanol, Ethylenglykol und dessen monomere und oligomere Monoalkyl- und Dialkylether, Tetrahydrofuran, Dioxan, dialkylierte Amide wie DMF, DMA, sowie TMU und HMPT. Man arbeitet dabei bei einer Temperatur von 0 bis 160°C, vorzugsweise von 20 bis 100°C.
   Die Amine der Formel IV erhält man in an sich literaturbekannter Weise bevorzugt aus den entsprechenden Carbonylverbindungen der Formel X 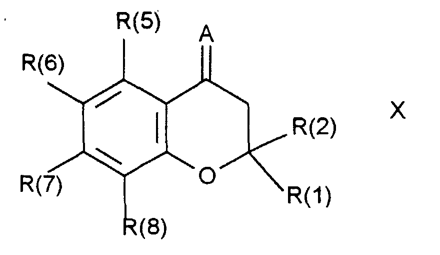 worin R(1), R(2), R(5), R(6), R(7) und R(8) die angegebene Bedeutung besitzen und A Sauerstoff bedeutet, entweder mit Ammoniak oder einem Amin der Formel XI

   R(4)-NH₂ XI

   mit R(4) in der angegebenen Bedeutung, worin jedoch r im Substituenten R(4) auch die Bedeutung Null hat, unter reduktiven katalytischen Bedingungen, vorzugsweise bei höherer Temperatur im Autoklaven. Dabei werden primär durch Kondensationsreaktion der Ketone X (mit A = Sauerstoff) und Aminen (XI) in situ die Schiff-Basen der Formel X mit A gleich R(4)-N = gebildet und unmittelbar ohne deren Isolierung reduktiv in das Amin der Formel IV übergeführt. Entsprechend können die bei dieser Reaktion intermediär aus X und XI entstehenden Schiff-Basen (Formel X, A: R(4)-N =) hergestellt und isoliert werden, um sie in einem separaten Schritt nachfolgend mit einem geeigneten Reduktionsmittel wie NaBH₄, LiAlH₄, NaBH₃CN oder durch katalytische Hydrierung in die Verbindungen der Formel IV umzuwandeln. Die Verbindungen IV mit R(4) gleich Wasserstoff können vorteilhaft in literaturbekannter Weise durch Reduktion von Oximen oder Oximethern (Formel X, A gleich RO-N=), Hydrazonen (Formel X, A: R(18)R(19)N-N=) durch Verwendung eines komplexen Metallhydrides oder durch katalytische Hydrierung erhalten werden. Die erforderlichen Oxime und Hydrazone werden vorzugsweise bequem und in an sich bekannter Weise aus den Ketonen der Formel X (A gleich Sauerstoff) mit Hydrazin oder einem seiner Derivate oder beispielsweise mit Hydroxylamin Hydrochlorid unter wasserabspaltenden Bedingungen hergestellt.
Verfahrensweise c)
   beschreibt wie auch Verfahrensweise a) die an sich bekannte Alkylierungsreaktion eines Sulfonamids bzw. eines seiner Salze VI mit einem Alkylierungsmittel der Formel VII. Entsprechend dieser Reaktionsanalogie gelten für Verfahrensweise c) die bereits ausführlich unter Verfahrensweise a) beschriebenen Reaktionsbedingungen.
   Die Herstellung der Sulfonamid-Derivate VI und deren Vorprodukte wurden bereits bei Verfahrensweise b) beschrieben. Die Herstellung der Alkylantien VII erfolgt nach Analogvorschriften der Literatur bzw. wie unter Verfahrensweise a) beschrieben, vorzugsweise aus den entsprechenden Hydroxyverbindungen (Formel VII mit L gleich -OH).
Verfahrensweise d)
   beschreibt die weitere chemische Umwandlung von erfindungsgemäßen Verbindungen der Formel I in andere Verbindungen der Formel I durch elektrophile Substitutionsreaktionen in einer oder in mehreren der mit R(5) bis R(8) bezeichneten Positionen, die jeweils Wasserstoff bedeuten.
   Bevorzugte Substitutionsreaktionen sind
   1. die aromatische Nitrierung zur Einführung einer oder mehrerer Nitrogruppen, sowie deren nachfolgende Reduktion zu NH₂-,
   2. die aromatische Halogenierung insbesondere zur Einführung von Chlor, Brom oder Jod,
   3. die Chlorsulfonierung zur Einführung einer Chlorsulfonylgruppe durch Einwirkung von Chlorsulfonsäure,
   4. die Friedel-Crafts Acylierungsreaktion zur Einführung eines Acylrestes R(16)-CₛH₂ₛ-CO- oder eines Sulfonylrestes R(16)-CₛH₂ₛ-SO₂- durch Einwirkung der entsprechenden Säurechloride R(16)-CₛH₂ₛ-CO-Cl bzw. R(16)-CₛH₂ₛ-SO₂-Cl in Gegenwart einer Lewis-Säure als Friedel-Crafts-Katalysator, vorzugsweise von wasserfreiem Aluminiumchlorid.

Die Verbindungen I sind verwandt mit der in der letzten Dekade in der Arzneimittelchemie intensiv bearbeiteten Klasse der 4-Acylaminochroman-Derivate, insbesondere von 2,2-Dialkyl-4-acylamino-3-chromanolen. Prominentester Vertreter derartiger 4-Acylaminochromane ist das Cromakalim der Formel XII 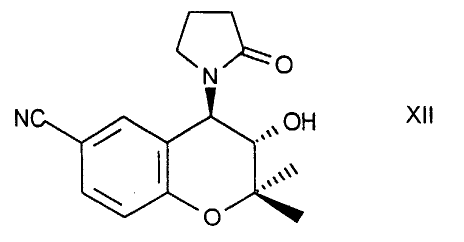 und zahlreiche, sich von diesem Präparat ableitenden Folgepräparate (z.B. Edwards and Weston, TIPS 11, 417-422 (1990), "Structure Activity Relationships of K⁺ channel openers"

Bei Cromakalim und anderen verwandten 4-Acylaminochroman-Derivaten handelt es sich um Verbindungen mit relaxierender Wirkung auf glattmuskuläre Organe, so daß sie zur Senkung des erhöhten Blutdruckes infolge Gefäßmuskelrelaxation und in der Behandlung des Asthmas infolge der Relaxation der glatten Muskulatur der Atemwege verwendet werden. All diesen Präparaten ist gemeinsam, daß sie auf zellulärer Ebene beispielsweise von glatten Muskelzellen wirken und dort zu einer Öffnung bestimmter ATP-sensitiver K⁺-Kanäle führen. Der durch den Austritt von K⁺-Ionen induzierte Anstieg von negativer Ladung in der Zelle ("Hyperpolarisation") wirkt über Sekundärmechanismen der Erhöhung von intrazellulärem Ca²⁺ und damit einer Zellaktivierung, z. B. einer Muskelkontraktion, entgegen.

Im Gegensatz zu diesen 4-Acylaminochroman-Derivaten, die wie erwähnt als Öffner des ATP-sensitiven K⁺-Kanals identifiziert wurden, zeigen die erfindungsgemäßen Verbindungen der Formel I mit der 4-Sulfonylaminostruktur überraschenderweise eine starke und spezifische blockierende (schließende) Wirkung auf einen K⁺-Kanal, der durch cyclisches Adenosinmonophosphat (cAMP) geöffnet wird und sich grundlegend vom erwähnten K⁺(ATP)-Kanal unterscheidet. Neuere Untersuchungen zeigen vielmehr, daß dieser am Dickdarm identifizierte K⁺(cAMP) -Kanal sehr ähnlich, vielleicht sogar identisch mit dem am Herzmuskel identifizierten I_{sK}-Kanal zu sein scheint. Infolge dieser Blockierung des K⁺(cAMP) -Kanals (= I_{sK}-Kanals) entwickeln die Verbindungen im lebenden Organismus pharmakologische Wirkungen von hoher therapeutischer Verwertbarkeit.

So zeichnen sich die Verbindungen I als neue Wirkstoffklasse potenter Inhibitoren der stimulierten Magensäuresekretion aus. Die Verbindungen der Formel I bzw. I a sind somit wertvolle Medikamente zur Behandlung von Ulcera des Magens und des intestinalen Bereiches, beispielsweise des Duodenums, aus. Sie eignen sich ebenfalls infolge ihrer starken magensaftsekretionshemmenden Wirkung als ausgezeichnete Therapeutika zur Behandlung der Refluxoesophagitis.

Die Verbindungen I zeichnen sich weiterhin durch eine antidiarrhoische Wirkung aus und sind deshalb als Arzneimittel zur Behandlung von Durchfall-Erkrankungen geeignet.

Weiterhin können die Verbindungen I als Arzneimittel zur Behandlung und Verhinderung aller Typen von Arrhythmien einschließlich atrialer, ventrikulärer und supraventrikulärer Arrhythmien verwendet werden. Sie können insbesondere angewandt werden zur Kontrolle von Reentry-Arrhythmien und zur Verhinderung des plötzlichen Herztodes infolge von Kammerflimmern.

Inzwischen existieren Publikationen, aus denen eine Korrelation zwischen I_{sK}-Kanal-inhibitorischer Wirkung und dem Unterbinden von lebensbedrohlichen cardialen Arrhythmien beschrieben wird, wie sie beispielsweise durch β-adrenerge Hyperstimulation ausgelöst werden (z. B. T. J. Colatsky, C. H. Follmer und C .F. Starmer: "Channel Specificity in Antiarrhythmic Drug Action; Mechanism of potassium channel block and its role in suppressing and aggravating cardiac arrhythmias", Circulation (1990) 82: 2235 - 2242; A. E. Busch, K. Malloy, W. J. Groh, M. D. Varnum, J. P. Adelman und J. Maylie; "The novel class III antiarrhythmics NE-10064 and NE-10133 inhibit I_{sK} channels in xenopus oocytes and I_{Ks} in guinea pig cardiac myocytes", Biochem. Biophys. Res. Commun. (1994) 202: 265 - 270).

Neben den oben genannten Cromakalim- bzw. Acylaminochroman-Derivaten wurden im Verlauf der letzten Jahre in der Literatur auch Verbindungen mit 4-Sulfonylaminochroman-Struktur beschrieben, die sich entweder in der Struktur oder in der biologischen Wirkung von den erfindungsgemäßen Verbindungen der Formel I deutlich unterscheiden. So beschreibt die Europäische Offenlegungsschrift 315 009 Chromanderivate mit 4-Phenylsulfonylaminostruktur, die sich durch antithrombotische und antiallergische Eigenschaften auszeichnen.

Die Europäische Offenlegungsschrift 370 901 beschreibt 3-Hydroxychromanderivate mit einer 4-Phenylsulfonylaminogruppe, worin die restliche Valenz des N-Atoms ein Wasserstoffatom trägt. Diese Verbindungen sind somit in essentiellen Gruppen von der Formel I bzw. I a unterschiedlich substituiert. Entsprechend werden für diese Verbindungen der Europäischen Offenlegungsschrift 370 901 Wirkungen auf das Zentralnervensystem gefunden, so daß sie sich auch in pharmakologischer Hinsicht unterscheiden.

Die Europäische Offenlegungsschrift 389 861 beschreibt 3-Hydroxychromanderivate mit 4-Sulfonylaminogruppe. Dabei handelt es sich bei den in der EP-OS beschriebenen Benzopyranderivaten um Aktivatoren bzw. Öffner des sogenannten adenosintriphosphat-sensitiven K⁺-Kanals (K⁺(ATP) - Kanal]. Die pharmakologischen Wirkungen von K⁺(ATP) -Kanalöffnern sind nun bekanntermaßen vollständig verschieden von den hier beschriebenen Blockern des I_{sK}-Kanals. So wurden für die K⁺(ATP) -Kanalöffnern für diesen Mechanismus typische vasodilatierende und blutdrucksenkende Eigenschaften nachgewiesen. Die von den Autoren synthetisierten, beschriebenen K⁺(ATP) - Kanalöffner zeigen erwartungsgemäß für diesen Mechanismus der K⁺-Kanalöffnung typische, spezielle antiarrhythmische Eigenschaften. In einer grundlegenden Arbeit konnte von Lucchesi et al. ( J.Cardiovasc. Pharmacol. 15, 452 464 [1990]) eindrucksvoll gezeigt werden, daß K⁺(ATP) -Kanalöffner am sauerstoffunterversorgten kranken Herzen oder bei plötzlichen Ischämien nicht antiarrhythmisch wirken, sondern sogar im Gegenteil lebensbedrohliche profibrillatorische Effekte verursachen. Diese gefährlichen Zustände werden als Konsequenz der aus der Aktivierung des K⁺(ATP) -Kanals resultierenden Verkürzungen der Repolarisationsdauer ausgelöst. Im Gegensatz zu diesen am kranken, mangelversorgten Herzen lebensbedrohlichen profibrillatorischen Effekten durch die Wirkung von K⁺(ATP) -Kanalöffnern sollten Blocker des K⁺(cAMP)-Kanals unter diesen Bedingungen antifibrillatorische Wirkung zeigen. Als prominenter Vertreter der von uns synthetisierten Verbindungen der Formel la fand inzwischen 6-Cyano-4-(n-ethylsulfonyl-N-methyl)amino-2,2-dimethyl-3-chromanol unter der Bezeichnung 293B Eingang in die neueste Literatur als Beispiel eines hochspezifischen I_{Ks}- bzw. I_{sK}-Kanalblockers mit einer entsprechenden Verlängerung des Aktionspotentials am Herzen (Süßbrich et al, Naunyn Schiedebergs Arch.Pharm. [1996] 353 (4,Suppl.), R72; Pflügers Arch.-Eur. J. Physiol. 431 (6) [Suppl], R 22 [1996], A. Busch et al., Pflügers Arch.-Eur. J. Physiol. 432 (6) [Suppl], 1094-1096 [1996]).
Aufgrund spezieller struktureller Kenntnisse wurden von uns einige Verbindungen synthetisiert und untersucht, die zwar aus der genannten Offenlegungsschrift (EP-OS 389 861) bereits bekannt sind, aber von den Autoren nicht beschrieben, synthetisiert bzw. in ihrer therapeutischen Wirkung erkannt wurden. Für diese speziellen, von uns hergestellten und untersuchten 3-hydroxysubstituierten Chromane wurde nun überraschenderweise eine potente Blockade des K⁺(cAMP)- Kanals (Pflügers Arch. - Eur. J. Physiol. [1995] 429: 517 - 530 A new class of inhibitors of cAMP-mediated Cl⁻ secretion in rabbit colon, acting by the reduction of cAMP-activated K⁺ conductance) festgestellt und die Inhibition des I_{Ks}-Kanals am Herzen. Die I_{sK}-kanalblockierende Wirkung der 3-hydroxysubstituierten Chromane ist aber deutlich weniger ausgeprägt als die der entsprechenden hydroxylgruppenfreien Chromane der Formel I. Gegenstand der Erfindung ist deshalb auch die Verwendung von Verbindungen der Formel Ia zur Behandlung des Sudden Cardiac Death, ventrikulärer Fibrillationen und generell von Arrhythmien des kranken Herzen, die auf den I_{Ks}-Kanals zurückzuführen sind.

Die Publikation "N-sulfonamides of benzopyran-related potassium channel openers: conversion of glyburyde insensitive smooth muscle relaxants to potent smooth muscle contractors" in Bioorg. Med. Chem. Lett. (1994) 4: 769 - 773 beschreibt spezielle trifluormethylsubstituierte 4-Sulfonylaminochroman-Derivate, die aber im Gegensatz zu den hier beschriebenden strukturell andersartigen K⁺(cAMP)-Kanalblockern biologisch andersartige pharmakologische Wirkungen und damit andere therapeutische Anwendungsbereiche besitzen.

In jüngster Zeit wurden außerdem Spiro[2H-1-benzopyran-2,4'-piperidine] mit essentieller basischer Seitengruppe in der Literatur beschrieben, z. B. MK-499 "Cardiac electrophysiology and antiarrhythmic actions of two long-acting spirobenzopyran piperidine class III agents, L-702,958 and L-706,000 (MK 499)" J. Pharmakol. Exp. Ther. (1994) 269: 541 - 554; T. J. Colatsky und T. M. Argentieri, "Potassium channel blockers as antiarrhythmic drugs"; Drug Develp. Res. (1994) 33: 235 - 249.

Diese "Spirobenzopyran-Piperidines Class III Agents" werden allerdings in der Literatur hinsichtlich ihrer Wirkungsweise sehr klar charakterisiert [P. S. Spector, M. E. Curran, M. T. Keating, M. C. Sanguinetti, Circulation Res. (1996) 78: 499 - 503; J.J. Lynch et al., J. Pharmacol. Exp. Ther. (1994) 269: 541 - 554]. Dabei wird in der zitierten Literatur eindeutig beschrieben und gezeigt, daß die antiarrhythmische Wirkung dieser Verbindungen durch die Inhibition des HERG-Kanals und der rasch (rapid) aktivierendenden Komponente des delayed rectifier K⁺-Kanals, des I_{Kr}-Kanals, verursacht wird. Damit sind die Spirobenzopyran-Piperidine als Substanzen mit proarrhythmischer Komponente und mit der Gefahr einer erhöhten Mortalität gegen Placebo gekennzeichnet, wie sie für diese Wirkstoffklasse in der Sword Studie gezeigt wurde. Dies steht im klaren Gegensatz zu den erfindungsgemäßen Verbindungen, deren Vorteil in der Blockierung der langsam (slow) aktivierendenden Komponente des delayed rectifier K⁺-Kanals, des I_{Ks}-Kanals, besteht, die diese proarrhythmische Komponente nicht besitzen.

Die Verbindungen der Formel I können zur Erzielung einer vorteilhaften therapeutischen Wirkung auch mit anderen Wirkstoffen kombiniert werden. So sind in der Behandlung von Herz-Kreislauferkrankungen vorteilhafte Kombinationen mit herz-kreislaufaktiven Stoffen denkbar. Als derartige für Herz-Kreislauferkrankungen vorteilhafte Kombinationspartner können beispielsweise andere Antiarrhythmika, so Klasse I, II oder III -Antiarrhythmika infrage kommen, wie beispielsweise sogenannte I_{Kr}-Kanalblocker, z.B. Dofetilide, weiterhin blutdrucksenkende Stoffe wie ACE-Inhibitoren (beispielweise Enalapril, Captopril, Ramipril), Angiotensin-Antagonisten, K⁺-Kanalaktivatoren, sowie alpha- und β-Rezeptorenblocker, aber auch sympathomimetische und adrenerg wirkende Verbindungen, sowie Na⁺/H⁺-Exchange Inhibitoren, Calciumkanalantagonisten, Phosphodiesterasehemmer und andere positiv inotrop wirkende Stoffe wie Digitalisglykoside, Diuretika.

Weiterhin kann eine Kombination mit antibiotisch wirkenden Substanzen und mit Antiulkusmitteln, beispielsweise mit H2-Antagonisten (Ranitidin, Cimetidin, Famotidin etc) vorteilhaft sein, wie insbesondere in der Anwendung zur Behandlung von Magen-Darmerkrankungen.

Arzneimittel, die eine erfindungsgemäße Verbindung I enthalten, können oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen I mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen der Formel I gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln. Als Lösungsvermittler werden beispielsweise auch Oligosaccharide wie Cyclodextrine verwendet.

Als pharmazeutische Formulierung für die Verabreichung in Form von Aerosolen oder Sprays sind geeignet z. B. Lösungen, Suspensionen oder Emulsionen des Wirkstoffes des Formel I in einem pharmazeutisch unbedenklichen Lösungsmittels, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen; aber auch von Art und Stärke der zu behandelnden Krankheit sowie von Geschlecht, Alter, Gewicht und individueller Ansprechbarkeit des zu behandelnden Säugers ab.

Im Durchschnitt beträgt die tägliche Dosis einer Verbindung der Formel I bei einem etwa 75 kg schweren Patienten mindestens 0,1 mg, vorzugsweise 10 mg bis höchstens 100 mg, vorzugsweise höchstens 1 g; bzw. für Verbindungen der Formel I a mindestens 1 mg, vorzugsweise 50 mg, bis höchstens 300 mg, vorzugsweise 1 g.

Erklärung der im Text verwendeten Abkürzungen
- DMA: Dimethylacetamid
- HMPT: Hexamethylphosphorsäuretriamid
- TMU: Tetramethylharnstoff
- hr: Stunde(n)
- M: Mol
- mM: Millimol
- min: Minuten
- TEA: Triethylamin
- THF: Tetrahydrofuran

### Beispiele:

### Beispiel 1: 4-(N-Ethylsulfonyl-N-methyl)amino-2,2-dimethylchroman

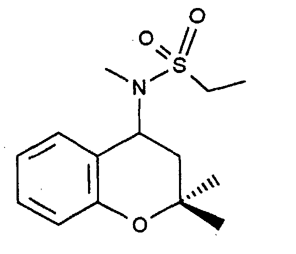

a) 2,2-Dimethyl-4-chromanonoxim
   Ein Reaktionsgemisch hergestellt aus 10 mM 2,2-Dimethyl-4-chromanon, 12 mM Hydroxylamin Hydrochlorid in 5 ml Methanol und 5 ml Pyridin erhitzt man unter Rühren über 2 Stunden auf 80 - 85°C, destilliert das Lösungsmittel am Rotationsverdampfer ab und bringt den öligen Rückstand unter Wasser zur Kristallisation.
   Kristalline Substanz, Schmp. 115 - 118 °C.
b) 4-Amino-2,2-dimethylchroman Hydrochlorid
   Eine Lösung aus 10 mM 2,2-Dimethyl-4-chromanonoxim und 75 ml Methanol wird nach Zugabe von Raney-Nickel als Katalysator im Autoklaven mit Wasserstoff bei 60 °C, 100 atm. Druck über eine Dauer von 6 Stunden hydriert. Nach Filtration und dem Abdestillieren des Lösungsmittels löst man den amorphen Rückstand in Ethylacetat und versetzt mit HCl-gasgesättigtem Diethylether bis zur stark sauren Reaktion. Der kristalline Niederschlag von 2,2-Dimethyl-4-aminochroman Hydrochlorid wird abfiltriert, mehrfach mit Ethylacetat gewaschen und getrocknet.
   Farblose Kristalle, Schmp. 268 °C.
c) 4-N-Ethylsulfonylamino-2,2-dimethylchroman
   Variante 1: Zu einer gerührten Lösung hergestellt aus 4,3 mMol 4-Amino-2,2-dimethylchroman Hydrochlorid, 15 ml THF und 1,25 ml TEA wird bei 0°C in Portionen eine Lösung aus 4,5 mMol Ethansulfonsäurechlorid in 5 ml THF gegeben. Man läßt etwa 2 Stunden bei 0°C und 1 weitere Stunde bei Raumtemperatur rühren, filtriert den Niederschlag und destilliert das Lösungsmittel am Rotationsverdampfer ab. Das zurückbleibende Öl kristallisiert unter Petrolether.
   Farblose Kristalle, Schmp.: 106 - 108°C.
   Variante 2: Zu einer Suspension aus 1,06 g (0,005 M) 4-Amino-2,2-dimethylchroman Hydrochlorid und 2,0 g (0,02 M) TEA in 25 ml DMA gibt man portionsweise zwischen 0 und 5°C 0,83 g ((0,0065 M) Ethansulfonsäurechlorid und läßt 2 Tage bei Raumtemperatur rühren. Nach Abdestillieren des Lösungsmittels am Rotationsverdampfer versetzt man mit Wasser, worauf das sich abscheidende Öl nach kurzer Zeit kristallin erstarrt.
   Schmp. 106 - 109 °C.
d) 4-N-Ethylsulfonyl-N-methylamino-2,2-dimethylchroman:
   In eine Natriummethylat-Lösung, dargestellt aus 0,0166 Tom Natrium in 20 ml wasserfreiem Methanol gibt man langsam eine Lösung von 0,0111 M 4-N-Ethylsulfonylamino-2,2-dimethylchroman in 15 ml wasserfreiem Methanol. Zu dieser Mischung gibt man anschließend in Portionen eine Lösung aus 0,014 M Methyliodid in 5 ml wasserfreiem Methanol und erhitzt für 6 Stunden am Rückflußkühler auf 50°C. Das Lösungsmittel wird am Rotationsverdampfer abdestilliert, der Rückstand mit Essigsäureethylester behandelt und mit 2 N NaOH extrahiert. Man trocknet die organische Phase über wasserfreiem Natriumsulfat und erhält 4-N-Ethylsulfonyl-N-methylamino-2,2-dimethylchroman durch erneutes Abdestillieren des Lösungsmittels.
   Farblose kristalline Substanz, Schmp.: 90 - 92 °C.

### Beispiel 2: 4-(N-Ethyl-N-ethylsulfonyl)amino-2,2-dimethylchroman:

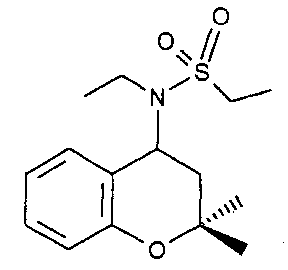

In eine Suspension von 0,0122 M Natriumhydrid in 30 ml wasserfreiem Dimethylacetamid unter Argonatmosphäre gibt man unter Rühren portionsweise 0,0111 M 4-N-Ethylsulfonylamino-2,2-dimethylchroman und rührt eine weitere Stunde bei Raumtemperatur. Nach anschließender Zugabe von 0,0122 M Ethylbromid läßt man bei Raumtemperatur weitere 24 Stunden rühren. Das Lösungsmittel wird unter vermindertem Druck abdestilliert, der Rückstand anschließend mit Essigester behandelt und mit Wasser extrahiert und die organische Phase nach Abtrennung und Trocknung über wasserfreiem Natriumsulfat im Rotationsverdampfer abdestilliert. Man erhält 4-N-Ethyl-N-ethylaminosulfonyl-2,2-dimethylchroman durch Kristallisation unter Petrolether als farblose kristalline Substanz,
Schmp 85 °C.

### Beispiel 3: 4-(N-Benzyl-N-ethylsulfonyl)amino-2,2-dimethylchroman

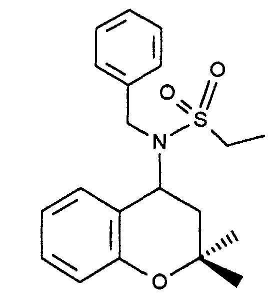

erhält man analog der in Beispiel 2 angegebenen Vorschrift aus 4-N-Ethylsulfonylamino-2,2-dimethylchroman und Benzylbromid.
Farblose Kristalle, Schmp 95 - 97 °C.

### Beispiel 4: 4-[N-Ethylsulfonyl-N-(2-dimethylaminoethyl)]amino-2,2-dimethylchroman

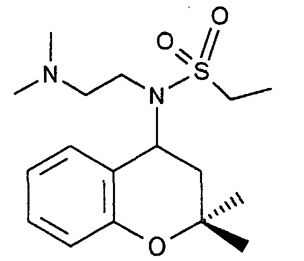

erhält man analog der in Beispiel 2 angegebenen Vorschrift aus 4-N-Ethylsulfonylamino-2,2-dimethylchroman und 2-Chlorethyl-dimethylamin Hydrochlorid unter Anwendung der doppelten Menge an Natriumhydrid.
Farblose Kristalle, Schmp 90 - 93 °C.

### Beispiel 5: 4-N-Ethylsulfonylamino-2,2-dimethyl-6,8-dinitrochroman

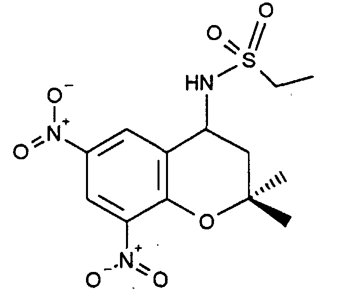

In 4,3 ml auf -15 bis -20°C gekühlte 100%ige Salpetersäure gibt man portionsweise unter Rührung 3,71 mM 4-N-Ethylsulfonylamino-2,2-dimethylchroman und rührt unter Beibehaltung der Kühlung weitere 20 Minuten. Man gießt das Reaktionsgemisch in 50 ml Eiswasser, filtriert und wäscht die gelben Kristalle mehrfach mit Wasser. Die Verbindung wird gereinigt durch Chromatographie an Kieselgel mit einem Gemisch aus 3 Teilen Ethanol und 7 Teilen Ethylacetat und anschließend mit Petrolether kristallisiert.
Gelbe kristalline Verbindung, Schmp. 140-142 °C.

### Beispiel 6: 4-N-Ethylsulfonylamino-2,2-dimethyl-6-nitrochroman

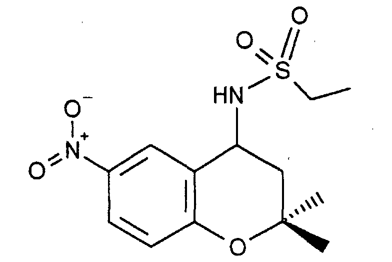

Zu einer Mischung von 3,71 mM 4-N-Ethylsulfonylamino-2,2-dimethylchroman in 2,54 ml Essigsäure gibt man portionsweise bei -20°C 0,54 ml 100%ige Salpetersäure und rührt weitere 5 min bei -20°C. Das Reaktionsgemisch wird in 50 ml Eiswasser gegossen, der violettfarbene Niederschlag filtriert und mehrfach mit kaltem Wasser auf dem Filter gewaschen. Die Kristalle werden in wenig Ethylacetat gelöst und an Kieselgel mit einer Mischung aus 3 Teilen Petrolether und 2 Teilen Ethylacetat chromatografiert.
Hellgelbe Kristalle, Schmp. 198 - 201 °C.

### Beispiel 7: 4-(N-Ethyl-N-ethylsulfonyl)amino-2,2-dimethyl-6-nitrochroman

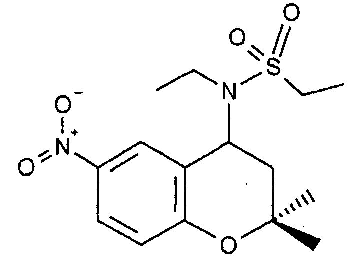

erhält man analog der in Beispiel 2 angegebenen Vorschrift aus 4-N-Ethylsulfonylamino-2,2-dimethyl-6-nitro-chroman und Ethylbromid.
Hellgelbe Kristalle, Schmp 180 - 185 °C.

### Beispiel 8: 4-(N-Ethylsulfonyl-N-methyl)amino-2,2-dimethyl-6-nitrochroman

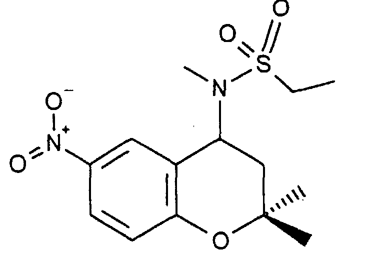

erhält man analog der in Beispiel 2 angegebenen Vorschrift aus 4-N-Ethylsulfonylamino-2,2-dimethyl-6-nitro-chroman und Methyliodid.
Hellgelbe Kristalle, Schmp 190 - 192 °C.

### Beispiel 9: 6-Amino-4-(N-ethylsulfonyl-N-methyl)amino-2,2-dimethylchroman Hydrochlorid

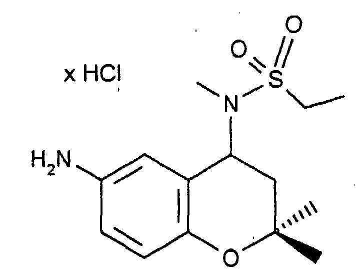

erhält man durch katalytische Hydrierung von 7,21 mM 4-N-Ethylsulfonyl-N-methylamino-2,2-dimethyl-6-nitrochroman mit Wasserstoffgas in 150 ml Methanol mit Raney-Nickel als Katalysator bis zur Aufnahme der theoretischen Menge an Wasserstoff über eine Dauer von etwa 1,5 hr bei 760 Torr. Nach Filtration und Verdampfen des Lösungsmittels löst man den amorphen Rückstand in Essigester und reinigt das Produkt durch Zugabe einer gesättigten Lösung von HCl-Gas in Diethylether durch Ausfällung des Hydrochlorids.
Farblose Kristalle, Schmp. 75 - 78 °C.

### Beispiel 10: 6-Amino-4-(N-ethyl-N-ethylsulfonyl)amino-2,2-dimethylchroman Hydrochlorid

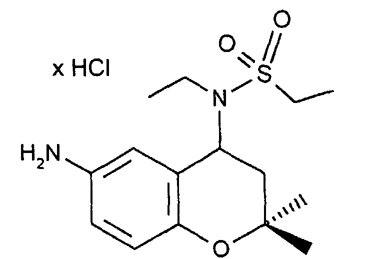

erhält man analog der in Beispiel 9 angegebenen Vorschrift aus 4-N-Ethyl-N-ethylsulfonylamino-2,2-dimethyl-6-nitrochroman durch katalytische Hydrierung mit Raney-Nickel.
Farblose Kristalle, Schmp. 95 - 100 °C.

### Beispiel 11: 4-N-(Dimethylaminosulfonyl)amino-2,2-dimethylchroman

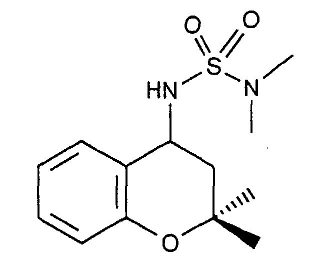

Zu einer Suspension von 10 mM 4-Amino-2,2-dimethylchroman Hydrochlorid in 75 ml wasserfreiem THF gibt man eine Lösung aus 0,03 mM TEA in 30 ml DMA. Man rührt etwa 30 min bei Raumtemperatur und tropft zu der Suspension unter Kühlung bei etwa 10 °C eine Lösung aus 12 mM N,N-Dimethylsulfamoylchlorid in 10 ml wasserfreiem THF. Nach Entfernen des Kühlbades rührt man bei Raumtemperatur weitere 24 hr. Sodann destilliert man am Rotationsverdampfer das Lösungsmittel ab und rührt den Rückstand unter Wasser, wobei nach einiger Zeit Kristallisation erfolgt. Nach Abfiltrieren der Kristalle und waschen mit Wasser erhält man 4-N-(Dimethylaminosulfonyl)-amino-2,2-dimethylchroman als farblose Kristalle,
Schmp. 77 - 79 °C.

### Beispiel 12: 4-N-Methyl-N-(dimethylaminosulfonyl)amino-2,2-dimethylchroman

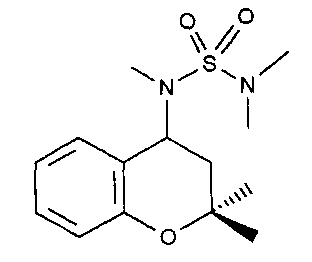

erhält man analog der in Beispiel 2 angegebenen Vorschrift aus 4-N-(Dimethylaminosulfonyl)amino-2,2-dimethylchroman und Methyljodid.
Farblose Kristalle, Schmp. 146 - 148°C.

### Beispiel 13: 4-N-Ethylsulfonylamino-6-fluor-2,2-dimethylchroman

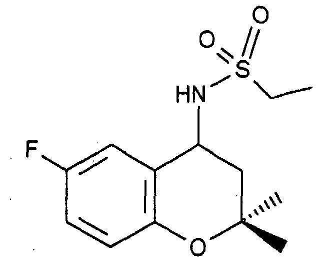

a) Essigsäure-4-fluorphenylester
   erhält man als öligen Rückstand durch Kochen von 4-Fluorphenol in Acetanhydrid und anschließendem Verdampfen des Lösungsmittels.
b) 5-Fluor-2-hydroxy-acetophenon
   erhält man aus 0,0376 Mol Essigsäure-4-fluorphenylester und 0,083 M wasserfreies AlCl₃ (für Friedel-Crafts-Reaktionen) bei 120°C für 2 bis 3 Stunden und durch anschließendes Zersetzen mit Eiswasser nach dem Abkühlen. Man extrahiert mit Essigester und destilliert nach Trocknung über Natriumsulfat das Lösungsmittel ab und bringt den amorphen viskosen Rückstand unter Cyclohexan zur Kristallisation.
   Farblose kristalline Substanz, Schmp. 46 - 47°C.
c) 6-Fluor-2,2-dimethyl-4-chromanon
   erhält man analog der in Beispiel 18 b) angegebenen Vorschrift aus 5-Fluor-2-hydroxy-acetophenon und Aceton in Gegenwart von Pyrrolidin.
   Farbloser bis schwach gelber amorpher Rückstand.
d) 6-Fluor-2,2-dimethyl-4-chromanonoxim
   erhält man analog der in Beispiel 1 a) angegebenen Vorschrift aus 6-Fluor-2,2-dimethyl-4-chromanon und Hydroxylamin Hydrochlorid, Kristallisation des Produkts unter Wasser und Umkristallsation aus Cyclohexan unter Verwendung von Aktivkohle.
   Farblose Kristalle, Schmp. 108 - 110°C.
e) 4-Amino-6-fluor-2,2-dimethyl-4-chroman Hydrochlorid
   erhält man analog der in Beispiel 1 b) angegebenen Vorschrift durch katalytische Hydrierung im Autoklaven aus 6-Fluor-2,2-dimethyl-4-chromanonoxim, Raney-Nickel und Wasserstoff.
   Farblose Kristalle, Schmp. 226 °C, Sublimation ab 296 °C.
f) 4-N-Ethylsulfonylamino-6-fluor-2,2-dimethylchroman
   Zu einer Suspension aus 5 mM 4-Amino-6-fluor-2,2-dimethyl-4-chroman Hydrochlorid in 20 ml DMA und 15 mM TEA gibt man 5,5 mM Ethansulfonsäurechlorid unter Rührung und Kühlung auf 10°C. Man rührt weitere 24 Stunden bei Raumtemperatur, destilliert sodann das Lösungsmittel am Rotationsverdampfer ab und rührt den Rückstand unter 75 ml Wasser. Das sich abscheidende Öl wird mit Ethylacetat extrahiert, abgetrennt, über wasserfreiem Natriumsulfat getrocknet. Nach dem Abdestillieren des Lösungsmittels im Rotationsverdampfer wird 4-Ethylsulfonylamino-6-fluor-2,2-dimethylchroman als amorphes Produkt erhalten.

### Beispiel 14: 4-(N-Ethylsulfonyl-N-methyl)amino-6-fluor-2,2-dimethylchroman

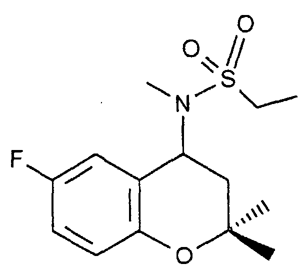

erhält man analog der in Beispiel 2 angegebenen Vorschrift aus 4-N-Ethylsulfonylamino-6-fluor-2,2-dimethylchroman und Methyliodid.
Amorphes öliges Produkt.

### Beispiel 15: 6-Fluor-4-N-(dimethylaminosulfonyl)amino-2,2-dimethylchroman

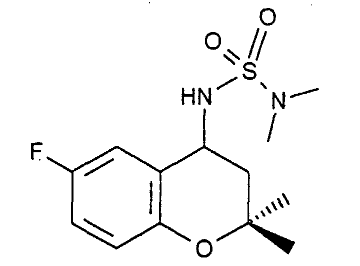

erhält man analog der in Beispiel 11 angegebenen Vorschrift aus 4-Amino-6-fluor-2,2-dimethylchroman und N,N-Dimethylsulfamoylchlorid in wasserfreiem DMA. Farblose Kristalle, Schmp. 86 - 88 °C.

### Beispiel 16: 6-Fluor-4-[N-methyl-N-(dimethylaminosulfonyl)]amino-2,2-dimethylchroman

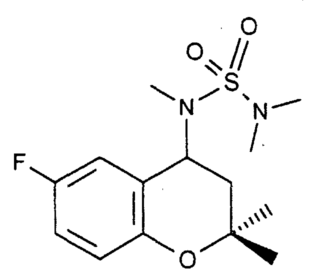

erhält man analog der in Beispiel 2 angegebenen Vorschrift aus 6-Fluor-4-N-(dimethylaminosulfonyl)amino-2,2-dimethylchroman und Methyliodid. Amorphes öliges Produkt.

### Beispiel 17: 6-Cyano-4-(N-ethylsulfonyl-N-methyl)amino-2,2-dimethylchroman

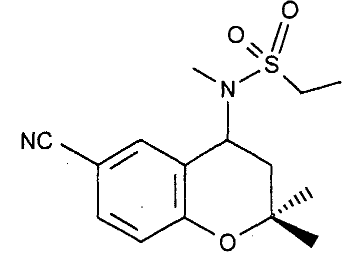

a) 6-Cyano-2,2-dimethylchroman
   Eine Suspension bestehend aus 10 mM 6-Cyano-2,2-dimethyl-3,4-chromen, 50 ml Methanol und ca 500 mg Palladiumkatalysator auf Bariumsulfat (10%-ig) wird in einer Schüttelente unter Wasserstoffatmosphäre bei 1 atm und bei 20°C bis zur Aufnahme der theoretischen Menge Wasserstoff geschüttelt. Nach Filtration vom Katalysator destilliert man das Lösungsmittel am Rotationsverdampfer ab und erhält 6-Cyano-2,2-dimethylchroman als farbloses bis schwach gelbliches Öl.
b) 4-Brom-6-cyano-2,2-dimethylchroman
   Zu einer Lösung von 10 mM 6-Cyano-2,2-dimethylchroman in 30 ml Tetrachlorkohlenstoff gibt man 11 mM N-Bromsuccinimid und 0,22 g Azodiisobutyronitril (Aldrich) und erhitzt die so erhaltene Suspension 3 Stunden am Rückflußkühler zum Sieden. Anschließend filtriert man vom unlöslichen Succinimid ab, destilliert das Lösungsmittel ab und bringt den Rückstand unter einer Mischung aus n-Hexan und Diisopropylether zur Kristallisation.
   Schwach gelbe Kristalle, Schmp. 93 - 94°C.
c) 6-Cyano-4-[N-ethylsulfonyl-N-methyl]amino-2,2-dimethylchroman
   Zu einer Suspension von 11 mM Natriumhydrid (als 80%ige Ölsuspension) in 5 ml wasserfreiem DMA tropft man unter Schutzgasatmosphäre aus Argon eine Lösung von 11 mM Ethansulfonsäure-N-methylamid und rührt ca eine Stunde bei Raumtemperatur nach. Sodann versetzt man mit einer Lösung aus 10 mM 4-Brom-6-cyano-2,2-dimethylchroman in 7 ml wasserfreiem DMA und rührt 72 Stunden bei 70°C. Man gießt das Reaktionsgemisch unter Rührung in 75 ml Wasser, extrahiert den ölig-amorphen Niederschlag mit Ethylacetat und trocknet die organische Phase über wasserfreiem Natriumsulfat. Das Lösungsmittel wird am Rotationsverdampfer abdestilliert und der amorphe Rückstand an der Kieselgelsäule mit einem Lösungsmittelgemisch bestehend aus 1 Teil Toluol und 1 Teil Ethylacetat als Laufmittel säulenchromatografisch getrennt. Nach Abdestillieren der Elutionsflüssigkeit im Rotationsverdampfer erhält man das 6-Cyano-4-[N-ethylsulfonyl-N-methyl]amino-2,2-dimethylchroman als farbloses kristallines Produkt,
   Schmp. 166 - 168 °C.

### Beispiel 18: 4-N-Ethylsulfonylamino-6-methoxycarbonyl-2,2-dimethylchroman

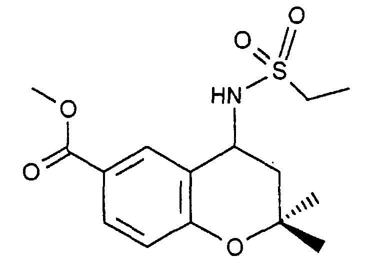

a) 3-Acetyl-4-hydroxybenzoesäure
   36,6 g (0,274 M) AlCl₃ werden in 50 ml 1,2,4-Trichlorbenzol suspendiert und mit 9 g (50 mM) 4-Acetoxybenzoesäure versetzt. Nach Zutropfen von 7,84 g (0,1 M) Acetylchlorid wird das Reaktionsgemisch auf 130 - 140°C erhitzt, wobei ab etwa 60°C die Entwicklung von HCl-Gas eintritt. Man rührt etwa 1 Stunde bei 130°C, läßt sodann auf 60 - 70°C abkühlen und gießt das Gemisch vorsichtig in gerührtes Eiswasser ein. Man extrahiert mehrfach mit Ethylacetat, extrahiert sodann die vereinigten organischen Phasen mit gesättigter wäßriger Natriumbikarbonat-Lösung und stellt die vereinigten wäßrigen Phasen mit konzentrierter HCl vorsichtig auf pH < 1, wobei sich die 3-Acetyl-4-hydroxybenzoesäure schwerlöslich abscheidet.
   Farblose kristalline Substanz, Schmp 228 - 233°C.
b) 6-Carboxy-2,2-dimethyl-4-chromanon
   Zu einer Suspension von 14,7 g (0,0815 M) 3-Acetyl-4-hydroxybenzoesäure in 200 ml Acetonitril gibt man 13,8 g Pyrrolidin und 40 ml Aceton. Man läßt die sich langsam verfärbende Lösung für 2 Tage bei Raumtemperatur stehen, destilliert das Lösungsmittel am Rotationsverdampfer ab, versetzt den Rückstand mit Wasser, stellt mit conc. Salzsäure auf sauren pH < 1 und filtriert die kristalline Substanz ab.
   Farblose Kristalle, Schmp 154 - 160°C.
c) 6-Carboxy-2,2-dimethyl-4-chromanonoxim
   14,9 g 6-Carboxy-2,2-dimethyl-3-chromanon werden in 100 ml Ethanol und 100 ml Pyridin gelöst, und nach Zugabe von 5,16 g Hydroxylamin Hydrochlorid für 6 Stunden unter Rührung auf 80°C erhitzt. Das Lösungsmittel wird am Rotationsverdampfer abdestilliert. Man versetzt den Rückstand mit Wasser, stellt mit conc. Salzsäure auf pH <1 gestellt und filtriert die farblosen Kristalle ab.
   Schmp. 223 - 225 °C.
d) 4-Amino-6-carboxy-2,2-dimethylchroman
   35,2 g (0,15 M) 6-Carboxy-2,2-dimethyl-4-chromanonoxim werden in 300 ml Methanol durch Zugabe von 600 ml konzentriertem wäßrigem Ammoniak in Lösung gebracht und nach Zugabe von einigen Gramm Raney-Nickel-Katalysator unter 100 atm Wasserstoffdruck bei 80°C über 10 Stunden hydriert. Nach Abfiltrieren des Katalysators destilliert man etwa 3/4 des Lösungsmittels im Rotationsverdampfer ab. Der kristalline Niederschlag an 4-Amino-6-carboxy-2,2-dimethylchroman wird abfiltriert.
   Farblose Kristalle, Schmp. 307 - 310°C.
e) 4-Amino-6-methoxycarbony)-2,2-dimethylchroman
   0,05 M 4-Amino-6-carboxy-2,2-dimethylchroman werden in 200 ml Methanol mit 9,5 ml konz. Schwefelsäure versetzt und die dunkle Lösung für 6 Stunden zum Rückfluß erhitzt. Unter Eiskühlung stellt man das Reaktionsgemisch durch portionsweise Zugabe von gesättigter wäßriger Kaliumcarbonatlösung auf pH 9 und filtriert vom abgeschiedenen Salz ab. Das Lösungsmittel wird am Rotationsverdampfer abdestilliert, der ölige Rückstand mit Wasser versetzt und mehrfach mit Diethylether extrahiert. Nach Abdestillieren des Lösungsmittels wird der ölig-amorphe Rückstand unter n-Heptan kristallisiert.
   Farblose kristalline Substanz, Schmp. 62 - 65°C.
f) 4-N-Ethylsulfonylamino-6-methoxycarbonyl-2,2-dimethylchroman
   erhält man analog der in Beispiel 1 c) angegebenen Vorschrift aus 0,0184 M 4-Amino-6-methoxycarbonyl-2,2-dimethylchroman mit 0,021 M Ethansulfonsäurechlorid in THF mit überschüssigem TEA.
   Farblose Kristalle, Schmp. 111 - 113°C.

### Beispiel 19: 4-(N-Ethylsulfonyl-N-methyl)amino-6-methoxycarbonyl-2,2-dimethylchroman

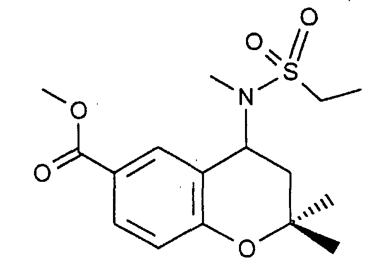

erhält man analog der in Beispiel 2 angegebenen Vorschrift aus 0,0155 M 4-N-Ethylsulfonylamino-6-methoxycarbonyl-2,2-dimethylchroman, 0,0232 M NaH (als 80%ige Suspension in Öl) und 0,0217 M Methyljodid in wasserfreiem DMA.
Farblose kristalline Substanz, Schmp. 184 - 187°C.

### Beispiel 20: 6-Methoxycarbonyl-4-N-(dimethylaminosulfonyl)amino-2,2-dimethylchroman

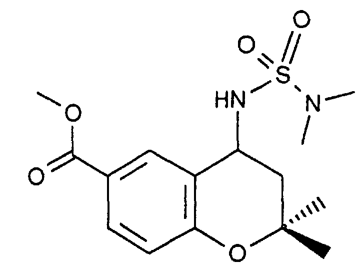

erhält man analog der in Beispiel 11 angegebenen Vorschrift aus 4-Amino-6-carboxy-2,2-dimethylchroman , Dimethylamidosulfonsäurechlorid und Triethylamin in THF.
Farblose Kristalle, Schmp. 127 - 129°C.

### Beispiel 21: 6-Methoxycarbonyl-4-[N-methyl-N-(dimethylaminosulfonyl)]amino-2,2-dimethylchroman

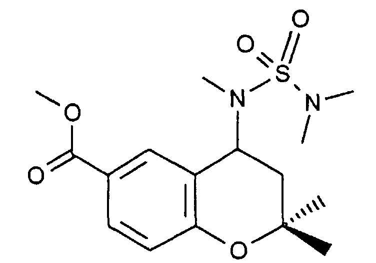

erhält man analog der in Beispiel 2 angegebenen Vorschrift aus 6-Methoxycarbonyl-4-N-(dimethylaminosulfonyl)amino-2,2-dimethylchroman, NaH und Methyliodid in DMA.
Farblose kristalline Substanz, Schmp. 125 - 129 °C

### Beispiel 22: 4-(N-Butyl-N-ethylsulfonyl)amino-6-methoxycarbonyl-2,2-dimethylchroman

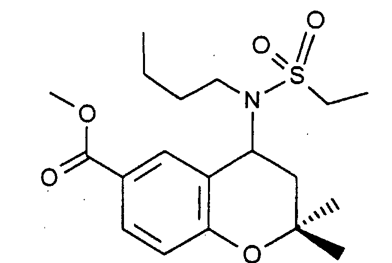

erhält man analog der in Beispiel 2 angegebenen Vorschrift aus 4-N-Ethylsulfonylamino-6-methoxycarbonyl-2,2-dimethylchroman, NaH und 1-Butyliodid in DMA.
Farbloses bis schwach gelbes ölig-amorphes Produkt.

### Beispiel 23: 6-Carboxy-4-(N-ethylsulfonyl-N-methyl)amino-2,2-dimethylchroman

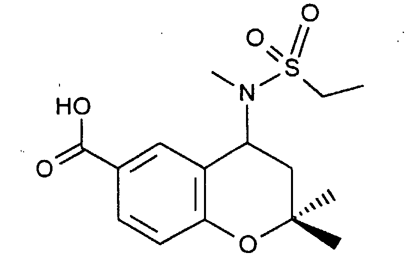

Eine Suspension bestehend aus 1 g (0,00305 M) 4-N-Ethylsulfonyl-N-methylamino-6-methoxycarbonyl-2,2-dimethylchroman, 30 ml Methanol und einer Lösung von 0,36 g (0,0091 M) NaOH in 20 ml Wasser rührt man unter Rückflußbedingungen bis zur Entstehung einer Lösung über etwa 10 Stunden. Man destilliert das Lösungsmittel am Rotationsverdampfer ab, versetzt den Rückstand mit Wasser, stellt mit HCl auf pH 0 bis 1 und filtriert die farblosen Kristalle ab.
Schmp. 235 - 237°C.

### Beispiel 24: 6-Aminocarbonyl-4-(N-ethylsulfonyl-N-methyl)amino-2,2-dimethylchroman

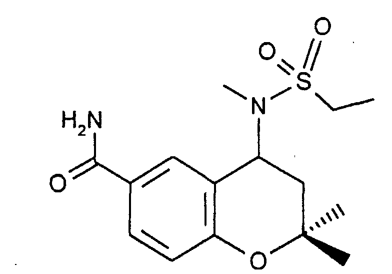

Zu einer Lösung aus 0,7 g 6-Carboxy-4-(N-ethylsulfonyl-N-methyl)amino-2,2-dimethylchroman (0,0021 M) in 25 ml wasserfreiem THF gibt man 0,38 g (0,0023 M) Carbonyldiimidazol, rührt 3 Stunden bei Raumtemperatur und versetzt anschließend mit 10 ml conc. wäßrige Ammoniaklösung (25%ig). Nach dem Rühren bei Raumtemperatur für ca. 15 Stunden destilliert man das Lösungsmittel am Rotationsverdampfer weitgehend ab, versetzt den Rückstand mit Wasser und filtriert die weiße kristalline Substanz ab.
Schmp. 202 - 204 °C.

### Beispiel 25: 6-Cyano-4-(N-ethylsulfonyl-N-methyl)amino-2,2-dimethylchroman

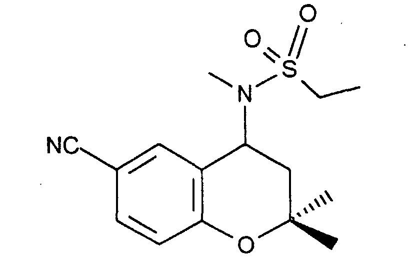

0,5 g (0,0015 M) 6-Aminocarbonyl-4-(N-ethylsulfonyl-N-methyl)amino-2,2-dimethylchroman werden mit einem Gemisch von 0,72 g (0,0045 M) N-Trimethylsilylpyrrolidon und 0,0013 g (0,000075 M) Natriumbis(trimethylsilyl)amid unter Argon-Atmosphäre versetzt und auf 90°C (Badtemperatur) erwärmt. Aus der anfänglichen festen Mischung entsteht eine Lösung, die man 4 Stunden bei 90°C rührt und anschließend über Nacht bei Raumtemperatur stehen läßt. Nach Entfernen des Inertgasschutzes und dem Verrühren mit Wasser kommt es zur Kristallisation des Öls. Man saugt die Kristalle ab und reinigt von noch enthaltenem Ausgangsprodukt durch Chromatographie an Kieselgel mit einem Gemisch aus 10 Teilen Methylenchlorid und 1 Teil Methanol als Elutionsmedium.
Schmp. 164 - 167 °C.

### Beispiel 26: 6-Carboxy-4-N-ethylsulfonylamino-2,2-dimethylchroman

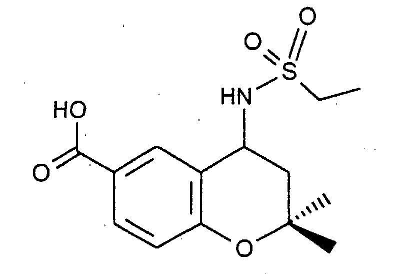

erhält man analog der in Beispiel 1 a) angegebenen Vorschrift aus 4-Amino-6-methoxycarbonyl-2,2-dimethylchroman mit Isopropylsulfonsäurechlorid in THF mit überschüssigem TEA.
Farblose Kristalle, Schmp. 112 - 115°C.

### Beispiel 27: 4-[N-Ethylsulfonyl-N-(4,4,4-trifuorbutyl)amino]-6-methoxycarbonyl-2,2-dimethylchroman

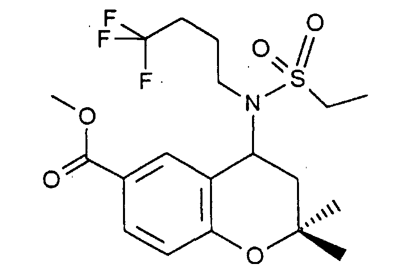

erhält man analog der in Beispiel 2 beschriebenen Vorschrift aus 4-N-Ethylsulfonylamino-6-methoxycarbonyl-2,2-dimethylchroman und 4,4,4-Trifluor-1-iodbutan in DMA.
Blaßgelbes bis farbloses ölig-amorphes Produkt.

### Beispiel 28: 6-Carboxy-4-[N-ethylsulfonyl-N-(4,4,4-trifuorbutyl)amino]-2,2-dimethylchroman

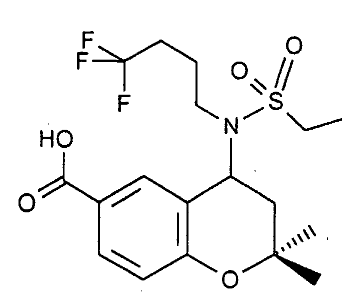

erhält man analog der in Beispiel 23 beschriebenen Vorschrift durch alkalische Hydrolyse von 4-N-Ethylsulfonyl-N-(4,4,4-trifuorbutyl)amino-6-methoxycarbonyl-2,2-dimethylchroman.
Farblose kristalline Substanz. Schmp. 189 - 192°C.

### Beispiel 29: 4-(N-Butyl-N-ethylsulfonyl)amino- 6-methoxycarbonyl-2,2-dimethylchroman

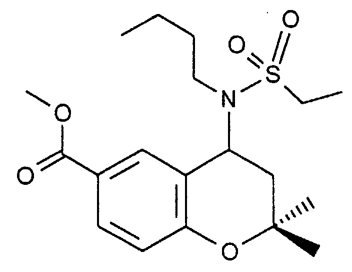

erhält man analog der in Beispiel 2 beschriebenen Vorschrift aus 4-N-Ethylsulfonylamino-6-methoxycarbonyl-2,2-dimethylchroman und Butyljodid in DMA. Farblose kristalline Substanz, Schmp. 81 - 84 °C.

### Beispiel 30: 6-Methoxycarbonyl-4-N-methylsulfonylamino-2,2-dimethylchroman

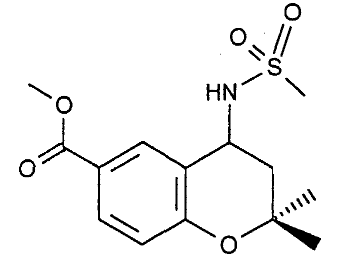

erhält man analog der in Beispiel 1 c) beschriebenen Vorschrift aus 4-Amino-6-methoxycarbonyl-2,2-dimethylchroman mit Methansulfonsäurechlorid.
Farblose kristalline Substanz, Schmp. 159 - 163°C.

### Beispiel 31: 6-Aminocarbonyl-4-[N-ethylsulfonyl-N-(4,4,4-trifuorbutyl)amino]-2,2-dimethylchroman

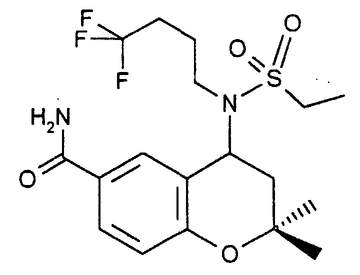

erhält man analog der in Beispiel 24 beschriebenen Vorschrift aus 6-Carboxy-4-N-ethylsulfonyl-N-(4,4,4-trifuorbutyl)amino-2,2-dimethylchroman, Carbonyldiimidazol und Ammoniak.
Farblose kristalline Substanz. Schmp. 170 - 174°C.

### Beispiel 32: 6-Carboxy-4-[N-methyl-N-(dimethylaminosulfonyl)amino]-2,2-dimethylchroman

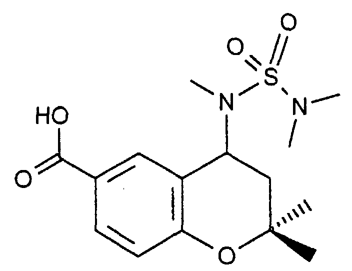

erhält man analog der in Beispiel 23 angegebenen Vorschrift aus 6-Methoxycarbonyl-4-[N-methyl-N-(dimethylaminosulfonyl)amino]-2,2-dimethylchroman.
Farblose kristalline Verbindung, Schmp. 245 - 248°C.

### Beispiel 33: 6-Cyano-4-[N-ethylsulfonyl-N-(4,4,4-trifluorbutyl)amino]-2,2-dimethylchroman

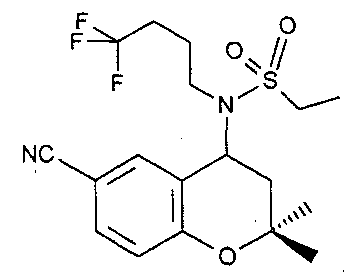

erhält man analog der in Beispiel 25 angegebenen Vorschrift aus 6-Aminocarbonyl-4-N-ethylsulfonyl-N-(4,4,4-trifuorbutyl)amino-2,2-dimethylchroman und anschließende Reinigung durch Säulenchromatografie an Kieselgel mit einem Gemisch aus 10 Teilen Methylenchlorid und 1 Teil Methanol als Elutionsmittel.
Farblose bis blaßgelbe kristalline Substanz, Schmp.: 172 - 176°C.

### Beispiel 34: 6-Aminocarbonyl-4-[N-methyl-N-(dimethylamino)sulfonylamino]-2,2-dimethylchroman

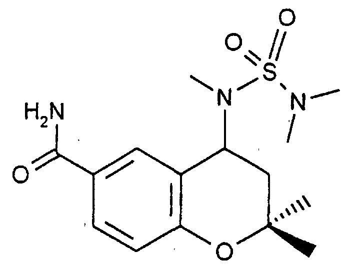

erhält man analog der in Beispiel 24 angegebenen Vorschrift aus 6-Carboxy-4-[N-methyl-N-(dimethylaminosulfonyl)amino]-2,2-dimethylchroman.
Farblose kristalline Substanz, Schmp. 215 - 218°C.

### Beispiel 35: 6-Cyano-4-[N-methyl-N-(dimethylaminosulfonyl)amino]-2,2-dimethylchroman

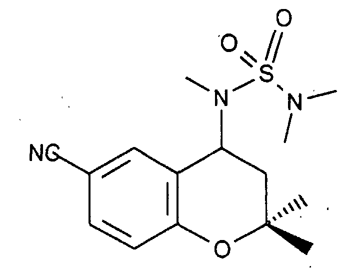

erhält man analog der in Beispiel 25 angegebenen Vorschrift aus 6-Aminocarbonyl-4-[N-methyl-N-(dimethylaminosulfonylamino]-2,2-dimethylchroman.
Farblose kristalline Substanz, Schmp. 100 - 102°C.

### Beispiel 36: 4-(N-Butyl-N-ethylsulfonyl)amino-6-carboxy-2,2-dimethylchroman

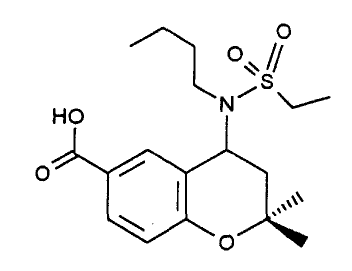

erhält man analog der in Beispiel 23 angegebenen Vorschrift aus 4-N-(Butyl-N-ethylsulfonyl)amino-6-methoxycarbonyl-2,2-dimethylchroman.
Farblose Kristalline Verbindung, Schmp. 148 - 151°C.

### Beispiel 37: 6-Aminocarbonyl-4-(N-butyl-N-ethylsulfonyl)amino-2,2-dimethylchroman

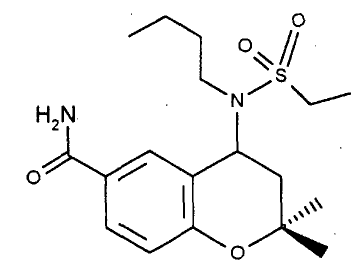

erhält man analog der in Beispiel 24 angegebenen Vorschrift aus 4-(N-Butyl-N-ethylsulfonyl)amino-6-carboxy-2,2-dimethylchroman.
Farblose kristalline Substanz, Schmp. 195 - 199°C.

### Beispiel 38: 4-(N-Butyl-N-ethylsulfonyl)amino-6-cyano--2,2-dimethylchroman

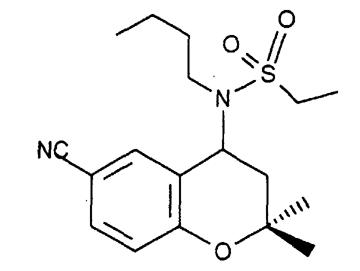

erhält man analog der in Beispiel 25 angegebenen Vorschrift aus 6-Aminocarbonyl-4-N-butyl-N-ethylsulfonylamino-2,2-dimethylchroman.
Farblose kristalline Substanz, Schmp. 96 - 98°C.

### Beispiel 39: 4-[N-Ethylsulfonyl-N-(4-picolyl)amino]-6-methoxycarbonyl-2,2-dimethylchroman

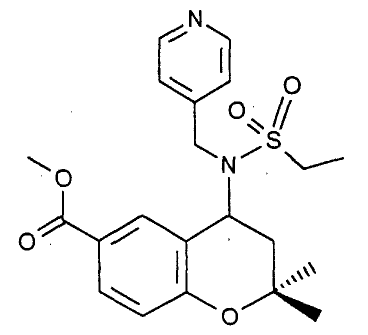

erhält man analog der in Beispiel 2 angegebenen Vorschrift aus 0,005 M 4-N-Ethylsulfonylamino-6-methoxycarbonyl-2,2-dimethylchroman, 0,015 M NaH und 0,007 M 4-Picolylchlorid Hydrochlorid.
Dunkel gefärbte, ölig-amorphe Substanz.

### Beispiel 40: 6-Carboxy-4-[N-ethylsulfonyl-N-(4-picolyl)amino]-2,2-dimethylchroman

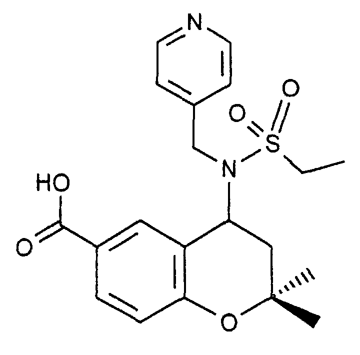

erhält man analog der in Beispiel 23 angegebenen Vorschrift aus 4-[N-Ethylsulfonyl-N-(4-picolyl)amino]-6-methoxycarbonyl-2,2-dimethylchroman.
Farblose kristalline Substanz, Schmp. 210 - 212°C.

### Beispiel 41: 6-Aminocarbonyl-4-[N-ethylsulfonyl-N-(4-picolyl)amino]-2,2-dimethylchroman

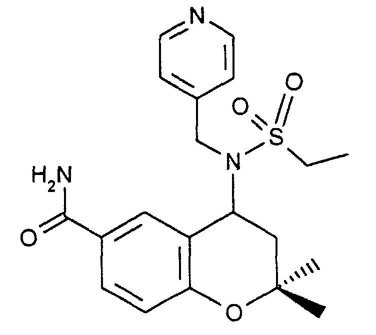

erhält man analog der in Beispiel 24 angegebenen Vorschrift aus 6-Carboxy-4-N-ethylsulfonyl-N-(4-picolyl)amino-2,2-dimethylchroman.
Farblose kristalline Verbindung, Schmp. 193 - 196°C.

### Beispiel 42: 6-Piperidinocarbonyl-4-N-ethylsulfonyl-N-methylamino-2,2-dimethylchroman

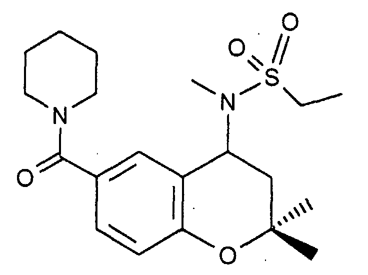

erhält man analog der in Beispiel 24 angegebenen Vorschrift aus 0,003 M 6-Carboxy-4-[N-ethylsulfonyl-N-methylamino]-2,2-dimethylchroman, 0,0033 M N,N-Carbonyldiimidazol und 0,012 M Piperidin.
Farblose Kristalle aus Ethanol, Schmp. 184°C.

### Beispiel 43: 4-N-Isopropylsulfonylamino-6-methoxycarbonyl-2,2-dimethylchroman

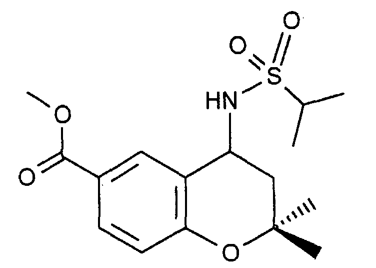

erhält man analog der in Beispiel 1 c) angegebenen Vorschrift aus 0,024 M 4-Amino-6-methoxycarbonyl-2,2-dimethylchroman mit 0,0319 M Ethansulfonsäurechlorid mit überschüssigem TEA in THF unter Rückflußbedingungen über 12 Stunden und durch zusätzliche Reinigung des Produktes durch Säulenchromatografie an Kieselgel mit einem Gemisch aus 1 Teil Ethylacetat und 3 Teilen Toluol als Elutionsmittel.
Farblose Kristalle, Schmp. 111 - 113°C.

### Beispiel 44: 4-(N-Isopropylsulfonyl-N-methyl)amino-6-methoxycarbonyl-2,2-dimethylchroman

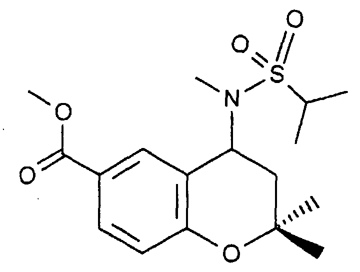

erhält man analog der in Beispiel 2 beschriebenen Vorschrift aus 4-N-Isopropylsulfonylamino-6-methoxycarbonyl-2,2-dimethylchroman und Methyliodid.
Farblose Kristalle, Schmp. 115 - 119°C.

### Beispiel 45: 6-Carboxy-4-(N-isopropylsulfonyl-N-methyl)amino-2,2-dimethylchroman

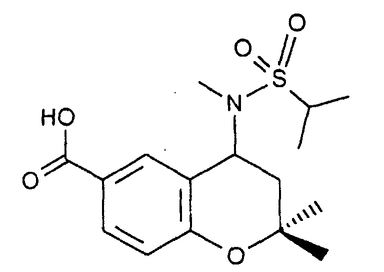

erhält man analog der in Beispiel 23 beschriebenen Vorschrift aus 4-(N-Isopropylsulfonyl-N-methyl)amino-6-methoxycarbonyl-2,2-dimethylchroman.
Farblose Kristalle, Schmp. 228 - 233°C.

### Beispiel 46: 6-Aminocarbonyl-4-(N-isopropylsulfonyl-N-methyl)amino-2,2-dimethylchroman

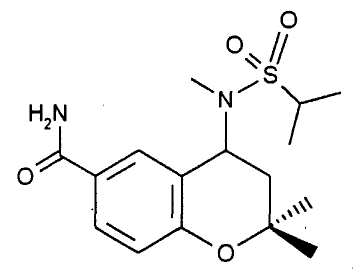

erhält man analog der in Beispiel 24 beschriebenen Vorschrift aus 6-Carboxy-4-(N-isopropylsulfonyl-N-methyl)amino-2,2-dimethylchroman.
Farbloses kristallines Produkt, Schmp. 217 - 220°C.

### Beispiel 47: 6-Cyano-4-(N-isopropylsulfonyl-N-methyl)amino-2,2-dimethylchroman

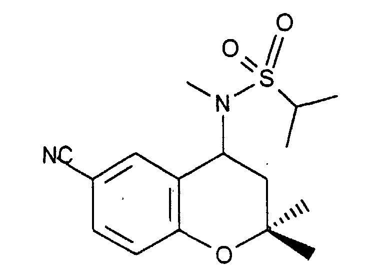

erhält man analog der in Beispiel 25 beschriebenen Vorschrift aus 6-Aminocarbonyl-4-(N-isopropylsulfonyl-N-methyl)amino-2,2-dimethylchroman. Nach Isolation des Produktes durch Filtration reinigt man durch Chromatographie an Kieselgel mit einem Gemisch aus 10 Teilen Methylenchlorid und 1 Teil Methanol und bringt die Substanz nach Abdestillieren des Lösungsmittels unter Diisopropylether zur Kristallisation.
Farblose Kristalle, Schmp. 129 - 135°C.

### Beispiel 48: 4-N-Butylsulfonylamino-6-methoxycarbonyl-2,2-dimethylchroman

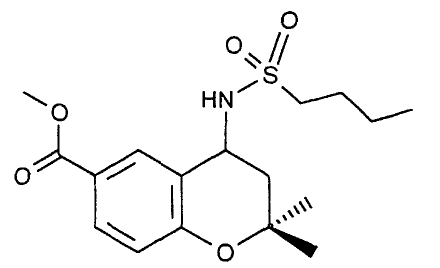

erhält man analog der in Beispiel 1 c) beschriebenen Vorschrift aus 4-Amino-2,2-dimethylchroman Hydrochlorid und 1-Butylsulfonylchlorid.
Schmp. 117 - 120°C.

### Beispiel 49: 4-(N-Butylsulfonyl-N-methyl)amino-6-methoxycarbonyl-2,2-dimethylchroman

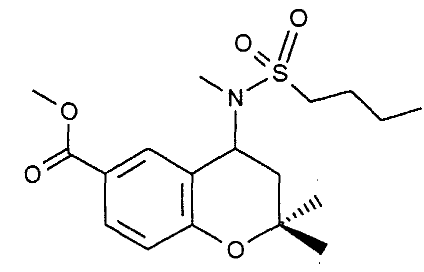

erhält man analog der in Beispiel 2 beschriebenen Vorschrift aus 4-N-Butylsulfonylamino-6-methoxycarbonyl-2,2-dimethylchroman und Methyliodid.
Farblose bis hellgelbes amorph- öliges Produkt.

### Beispiel 50: 4-(N-Butylsulfonyl-N-methyl)amino-6-carboxy-2,2-dimethylchroman

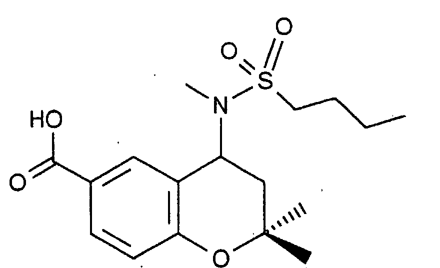

erhält man analog der in Beispiel 23 beschriebenen Vorschrift aus 4-(N-Butylsulfonyl-N-methyl)amino-6-methoxycarbonyl-2,2-dimethylchroman.
Farbloses kristallines Produkt, Schmp. 200 - 205°C.

### Beispiel 51: 6-Aminocarbonyl-4-(N-butylsulfonyl-N-methyl)amino-2,2-dimethylchroman

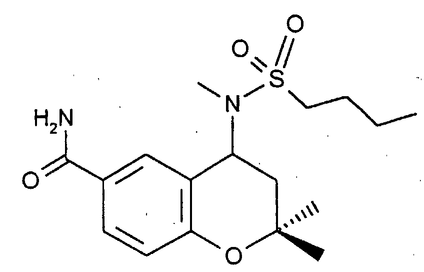

erhält man analog der in Beispiel 24 beschriebenen Vorschrift aus 4-(N-Butylsulfonyl-N-methyl)amino-6-carboxy-2,2-dimethylchroman.
Farbloses kristallines Produkt, Schmp. 162 - 166°C.

### Beispiel 52: 6-Cyano-4-N-butylsulfonyl-N-methylamino-2,2-dimethylchroman

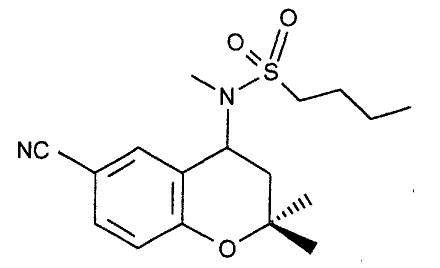

erhält man analog der in Beispiel 25 beschriebenen Vorschrift aus 6-Aminocarbonyl-4-(N-butylsulfonyl-N-methyl)amino-2,2-dimethylchroman. Nach Isolation des Produktes durch Filtration reinigt man durch Chromatographie an Kieselgel mit einem Gemisch aus 10 Teilen Methylenchlorid und 1 Teil Methanol und bringt die Substanz nach Abdestillieren des Lösungsmittels unter Diisopropylether zur Kristallisation.
Farblose Kristalle, Schmp. 57 - 62°C.

### Beispiel 53: 6-Methoxycarbonyl-4-(N-methyl-N-methylsulfonyl)amino-2,2-dimethylchroman

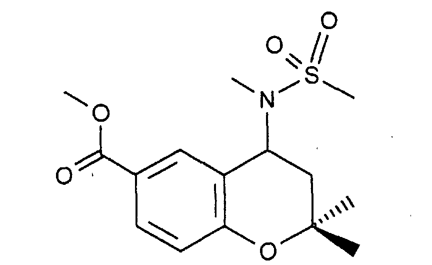

erhält man analog der in Beispiel 2 beschriebenen Vorschrift aus 6-Methoxycarbonyl-4-N-methylsulfonylamino-2,2-dimethylchroman und Methyljodid.
Farblose kristalline Substanz, Schmp. 160 - 164°C.

### Beispiel 54: 6-Carboxy-4-(N-methyl-N-methylsulfonyl)amino-2,2-dimethylchroman

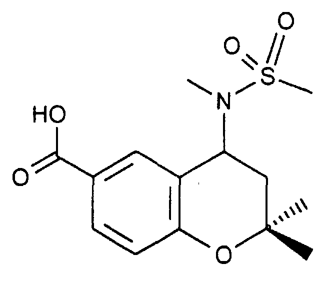

erhält man analog der in Beispiel 23 beschriebenen Vorschrift aus 6-Methoxycarbonyl-4-N-methyl-N-methylsulfonylamino-2,2-dimethylchroman durch alkalische Hydrolyse.
Farblose kristalline Verbindung vom Schmp. 214 - 216°C.

### Beispiel 55: 6-Aminocarbonyl-4-(N-methyl-N-methylsulfonyl)amino-2,2-dimethylchroman

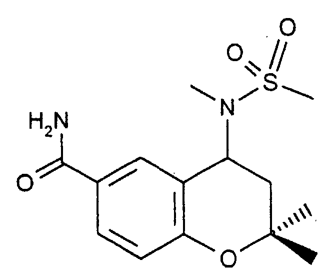

erhält man analog der in Beispiel 24 beschriebenen Vorschrift aus 6-Carboxy-4-(N-methyl-N-methylsulfonyl)amino-2,2-dimethylchroman.
Farblose kristalline Substanz, Schmp. 179 - 182°C.

### Beispiel 56: 6-Cyano-4-(N-methyl-N-methylsulfonyl)amino-2,2-dimethylchroman .

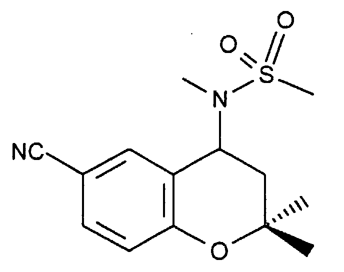

erhält man analog der in Beispiel 25 beschriebenen Vorschrift aus 6-Aminocarbonyl-4-(N-methyl-N-methylsulfonyl)amino-2,2-dimethylchroman. Nach Isolation des Produktes durch Filtration reinigt man durch Chromatographie an Kieselgel mit einem Gemisch aus 10 Teilen Methylenchlorid und 1 Teil Methanol und bringt die Substanz nach Abdestillieren des Lösungsmittels unter Diisopropylether zur Kristallisation.
Farblose Kristalle, Schmp.196 - 200°C.

### Beispiel 57: 4-(N-Ethylsulfonyl-N-ethyl)amino-6-methoxycarbonyl-2,2-dimethylchroman

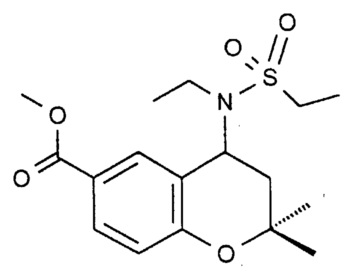

erhält man analog der in Beispiel 2 angegebenen Vorschrift aus 0,0091 M 4-N-Ethylsulfonylamino-6-methoxycarbonyl-2,2-dimethylchroman, 0,013 M NaH (als 80%ige Suspension in Öl) und 0,0126 M Ethyljodid in wasserfreiem DMA.
Farblose kristalline Substanz,
Schmp. 114 - 116°C.

### Beispiel 58: 4-(N-Ethylsulfonyl-N-propyl)amino-6-methoxycarbonyl-2,2-dimethylchroman

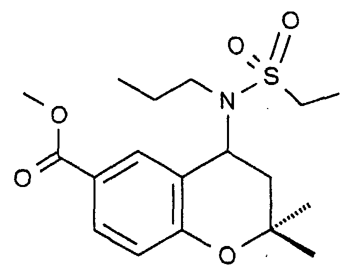

erhält man analog der in Beispiel 2 angegebenen Vorschrift aus 0,0091 M 4-N-Ethylsulfonylamino-6-methoxycarbonyl-2,2-dimethylchroman, 0,013 M NaH (als 80%ige Suspension in Öl) und 0,0126 M 1-Propyliodid in wasserfreiem DMA.
Farblose kristalline Substanz,
Schmp. 106-108°C.

### Beispiel 59: 4-(N-Ethylsulfonyl-N-cyclopropyl)amino-6-methoxycarbonyl-2,2-dimethylchroman

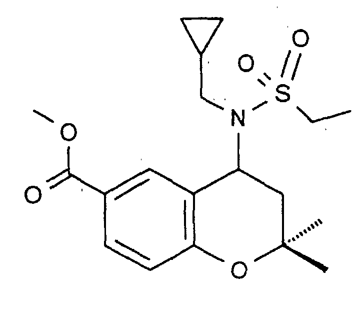

erhält man analog der in Beispiel 2 angegebenen Vorschrift aus 0,0091 M 4-N-Ethylsulfonylamino-6-methoxycarbonyl-2,2-dimethylchroman, 0,013 M NaH (als 80%ige Suspension in Öl) und 0,0126 M Brommethyl-cyclopropan in wasserfreiem DMA.
Farblose kristalline Substanz,
Schmp. 108-110°C.

### Beispiel 60: 4-(N-Ethylsulfonyl-N-1-pentyl)amino-6-methoxycarbonyl-2,2-dimethylchroman

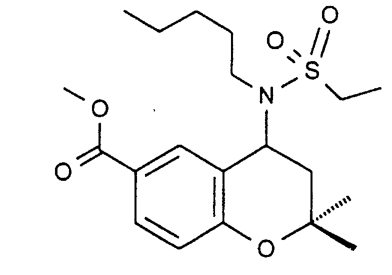

erhält man analog der in Beispiel 2 angegebenen Vorschrift aus 0,0091 M 4-N-Ethylsulfonylamino-6-methoxycarbonyl-2,2-dimethylchroman, 0,013 M NaH (als 80%ige Suspension in Öl) und 0,0126 M Pentyliodid in wasserfreiem DMA.
Öliges amorphes Produkt.

### Beispiel 61: 4-(N-Ethylsulfonyl-N-1-hexyl)amino-6-methoxycarbonyl-2,2-dimethylchroman

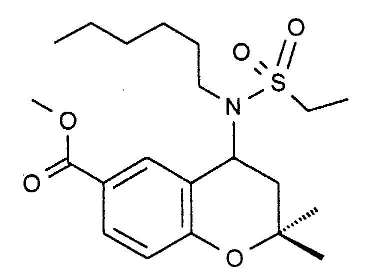

erhält man analog der in Beispiel 2 angegebenen Vorschrift aus 0,0091 M 4-N-Ethylsulfonylamino-6-methoxycarbonyl-2,2-dimethylchroman, 0,013 M NaH (als 80%ige Suspension in Öl) und 0,0126 M Hexyliodid in wasserfreiem DMA.
Öliges amorphes Produkt.

### Beispiel 62: 4-(N-Ethylsulfonyl-N-methyl)amino-6,7-dimethoxy-2,2-dimethylchroman

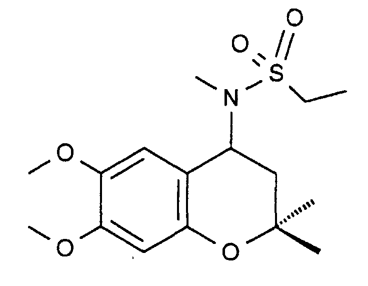

a) 6,7-Dimethoxy-2,2-dimethyl-4-chromanonoxim
   erhält man durch Umsetzung von 0,0189 M 6,7-Dimethoxy-2,2-dimethyl-4-chromanon mit 0,02 M Hydroxylamin-hydrochlorid in einer Mischung aus 20 ml Methanol und 20 ml Pyridin über 20 Stunden bei 60 - 80°C. Nach Abdestillieren des Lösungsmittels erhält man das farblose kristalline Produkt durch Behandeln des Rückstandes mit Wasser.
   Schmp. 110°C.
b) 4-Amino-6,7-dimethoxy-2,2-dimethyl-4-chroman-hydrochlorid
   erhält man analog der in Beispiel 1 b) angegebenen Vorschrift durch katalytische Hydrierung von 6,7-Dimethoxy-2,2-dimethyl-4-chromanonoxim und nachfolgender Aufarbeitung in Gegenwart von Salzsäure. Farblose kristalline
   Substanz vom Schmp. 210-215°C.
c) 4-N-Ethylsulfonylamino-6,7-dimethoxy-2,2-dimethylchroman
   erhält man analog Beispiel 1 c) (Variante 1) aus 4-Amino-6,7-dimethoxy-2,2-dimethyl-4-chroman-hydrochlorid und Ethansulfonsäurechlorid in THF in Gegenwart von Triethylamin.
   Farbloses kristallines Produkt,
   Schmp. 132 - 135°C
d) 4-(N-Ethylsulfonyl-N-methyl)amino-6,7-dimethoxy-2,2-dimethylchroman erhält man analog der in Beispiel 2 angegebenen Vorschrift aus 0,0036 M 4-N-Ethylsulfonylamino-6,7-dimethoxy-2,2-dimethylchroman, 0,00504 M NaH (als 80%ige Suspension in Öl) und 0,0054 M Methyljodid in wasserfreiem DMA.
   Amorphes viskoses Öl.

### Beispiel 63: 7-Chlor-4-(N-ethylsulfonyl-N-methyl)amino-6-fluor-2,2-dimethylchroman

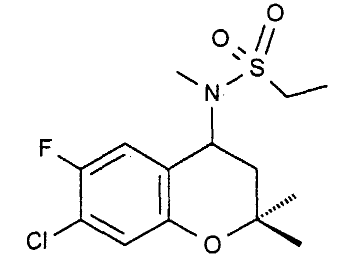

a) 2-Fluor-5-acetoxychlorbenzol
   erhält man durch Umsetzung von 3-Chlor-4-fluorphenol in Acetanhydrid bei 80°C über 6 Stunden.
   Farblose, kristallines Produkt,
   Schmp. 42-46°C.
b) 4-Chlor-5-fluor-2-hydroxyacetophenon
   erhält man durch Erwärmen eines Gemisches aus 0,0705 M 2-Fluor-5-acetoxychlorbenzol mit 0,148 M wasserfreiem Aluminiumchlorid unter mechanischer Rührung auf 120°C über ca. 3 Stunden, Zersetzung des Reaktionsgemisches mit Eiswasser-/Eis-Gemisch und Filtration des Niederschlages. Farblose kristalline Substanz durch Behandlung mit Aktivkohle in Methanol und nach Destillation des Lösungsmittels durch anschließendes Digerieren mit einer Mischung aus n-Heptan und Diisopropylether.
   Farblose Kristalle,
   Schmp. 66 - 71 °C.
c) 7-Chlor-6-fluor-2,2-dimethyl-4-chromanon
   erhält man analog der in Beispiel 18 b) angegebenen Vorschrift aus 4-Chlor-5-fluor-2-hydroxyacetophenon und Aceton in Gegenwart von Pyrrolidin in Acetonitril als Lösungsmittel.
   Farbloser bis schwach gelber amorpher Rückstand.
d) 7-Chlor-6-fluor-2,2-dimethyl-4-chromanonoxim
   erhält man analog der in Beispiel 1 a) angegebenen Vorschrift aus 7-Chlor-6-fluor-2,2-dimethyl-4-chromanon und Hydroxylamin-hydrochlorid.
   Kristallines Produkt,
   Schmp. 120 - 125°C.
e) 7-Chlor-6-fluor-2,2-dimethyl-4-aminochroman-hydrochlorid
   erhält man analog der in Beispiel 1 b) angegebenen Vorschrift durch katalytische Hydrierung von 7-Chlor-6-fluor-2,2-dimethyl-4-chromanonoxim und Aufarbeitung in Gegenwart von Salzsäure.
   Zwei Schmelzpunkte:
   1. Schmp.: 258 - 260°C unter erneuter Kristallisation der Schmelze,
   2. Schmp. > 310°C.
f) 7-Chlor-6-fluor-2,2-dimethyl-4-ethylsulfonylaminochroman
   erhält man analog der in Beispiel 1 c) angegebenen Vorschrift durch Reaktion von 7-Chlor-6-fluor-2,2-dimethyl-4-aminochroman-hydrochlorid mit Ethansulfonsäurechlorid in Gegenwart von TEA in THF
g) 7-Chlor-4-(N-ethylsulfonyl-N-methyl)amino-6-fluor-2,2-dimethylchroman
   erhält man analog der in Beispiel 2 angegebenen Vorschrift durch Umsetzung von 7-Chlor-6-fluor-2,2-dimethyl-4-ethylsulfonylaminochroman mit Natriumhydrid und Methyljodid.
   Farblose kristalline Substanz,
   Schmp. 104 - 107°C.

### Beispiel 64: 4-(N-Ethylsulfonyl-N-methyl)amino-2,2,6-trimethylchroman

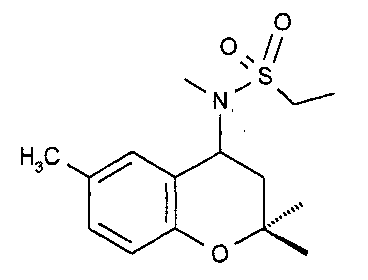

a) 2,2,6-Trimethyl-4-chromanon
   erhält man analog der in Beispiel 18 b) angegebenen Vorschrift aus 5-Methyl-2-hydroxyacetophenon und Aceton in Gegenwart von Pyrrolidin in Acetonitril als Lösungsmittel.
   Amorphes öliges Produkt.
b) 2,2,6-Trimethyl-4-chromanonoxim
   erhält man analog der in Beispiel 1 a) angegebenen Vorschrift aus 2,2,6-Trimethyl-4-chromanon und Hydroxylamin-hydrochlorid.
   Kristallines Produkt, Schmp. 120 - 125°C.
c) 4-Amino-2,2,6-trimethyl-4-aminochroman-hydrochlorid
   erhält man analog der in Beispiel 1 b) angegebenen Vorschrift durch katalytische Hydrierung von 2,2,6-Trimethyl-4-chromanonoxim und Aufarbeitung in Gegenwart von Salzsäure.
   Zwei Schmelzpunkte:
   1. Schmp.: 245 - 248°C unter erneuter Kristallisation der Schmelze,
   2. Schmp. > 310°C.
d) 4-Ethylsulfonylamino -2,2,6-dimethylchroman
   erhält man analog der in Beispiel 1 c) angegebenen Vorschrift durch Reaktion von 4-Amino-2,2,6-trimethylchroman-hydrochlorid mit Ethansulfonsäurechlorid in Gegenwart von TEA in THF.
   Farbloses kristallines Produkt,
   Schmp. 114 - 117 °C.
   4-(N-Ethylsulfonyl-N-methyl)amino-2,2,6-trimethylchroman
   erhält man analog der in Beispiel 2 angegebenen Vorschrift durch Umsetzung von 4-Ethylsulfonylamino -2,2,6-dimethylchroman mit Natriumhydrid und Methyljodid.
   Farblose kristalline Substanz,
   Schmp. 107°C.

### Beispiel 65: 6,7-Dichlor-4-(N-ethylsulfonyl-N-methyl)amino-2,2-dimethylchroman

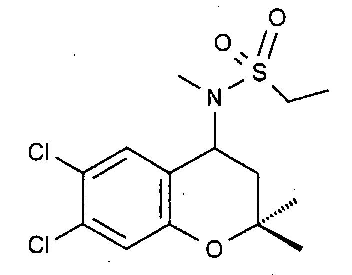

a) 4,5-Dichlor-2-hydroxyacetophenon
   erhält man analog der in Beispiel 63 b) angegebenen Vorschrift aus Essigsäure-(3,4-dichlorphenyl)ester und wasserfreiem, aktiven Aluminiumchlorid.
   Farblose bis schwach gelblich gefärbte kristalline Substanz,
   Schmp. 100 - 103°C. Der verwendete Essigsäure-(3,4-dichlorphenyl)ester wird aus 3,4-Dichlorphenol und Acetanhydrid analog Beispiel 63 a) als braunes Öl erhalten.
b) 6,7-Dichlor-2,2-dimethyl-4-chromanon
   erhält man analog der in Beispiel 18 b) angegebenen Vorschrift aus 4,5-Dichlor-2-hydroxyacetophenon und Aceton in Gegenwart von Pyrrolidin in Acetonitril als Lösungsmittel.
   Amorphes braunes öliges Produkt.
c) 6,7-Dichlor-2,2-dimethyl-4-chromanonoxim
   erhält man analog der in Beispiel 1 a) angegebenen Vorschrift aus 6,7-Dichlor-2,2-dimethyl-4-chromanon und Hydroxylamin-hydrochlorid.
   Kristallines Produkt,
   Schmp. 115 - 121 °C.
d) 4-Amino-6,7-dichlor-2,2-dimethylchroman-hydrochlorid
   erhält man analog der in Beispiel 1 b) angegebenen Vorschrift durch katalytische Hydrierung von 6,7-Dichlor-2,2-dimethyl-4-chromanonoxim und Aufarbeitung in Gegenwart von Salzsäure.
   Zwei Schmelzpunkte:
   1. Schmp.: 260 - 262°C unter erneuter Kristallisation der Schmelze,
   2. Schmp. > 310°C.
e) 6,7-Dichlor-2,2-dimethyl-4-N-ethylsulfonylaminochroman
   erhält man analog der in Beispiel 1 c) angegebenen Vorschrift durch Reaktion von 4-Amino-6,7-dichlor-2,2-dimethylchroman-hydrochlorid und Ethansulfonsäurechlorid in Gegenwart von TEA in THF.
   Farbloses kristallines Produkt, Schmp. 116 - 120°C.
f) 6,7-Dichlor-4-(N-ethylsulfonyl-N-methyl)amino-2,2-dimethylchroman
   erhält man analog der in Beispiel 2 angegebenen Vorschrift durch Umsetzung von 6,7-Dichlor-2,2-dimethyl-4-N-ethylsulfonylaminochroman mit Natriumhydrid und Methyljodid.
   Farblose kristalline Substanz,
   Schmp. 102 - 106°C.

### Beispiel 66: 4-(N-Ethylsulfonyl-N-methyl)amino-6-fluor-7-pyrrolidino-2,2-dimethylchroman (Referenzbeispiel)

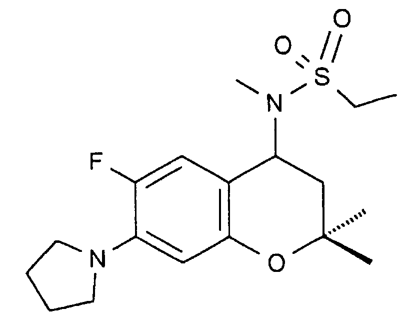

a) 4,5-Difluor-2-hydroxyacetophenon
   erhält man analog der in Beispiel 63 b) angegebenen Vorschrift aus Essigsäure-(3,4-difluorphenyl)ester und wasserfreiem, aktiven Aluminiumchlorid.
   Farblose bis schwach gelblich gefärbte kristalline Substanz,
   Schmp. 43 - 46°C (Kristallisation unter n-Heptan).
   Der verwendete Essigsäure-(3,4-difluorphenyl)ester wird aus 3,4-Difluorphenol und Acetanhydrid analog der in Beispiel 63 a) als helles Öl erhalten.
b) 6-Fluor-7-pyrrolidino-2,2-dimethyl-4-chromanon
   erhält man analog der in Beispiel 18 b) angegebenen Vorschrift 4,5-Difluor-2-hydroxyacetophenon und Aceton in Gegenwart von 1,1 Mol-Äquivalenten Pyrrolidin in Acetonitril als Lösungsmittel, wobei zusätzlich zum Chromanon-Ringschluß das in 7-Position befindliche F-Atom gegen Pyrrolidin ausgetauscht wird. Zur weiteren Reinigung kann das Produkt durch Chromatografie an Kieselgel und einem 8 : 1 - Gemisch aus Toluol / Essigsäureethylester abgetrennt werden. Kristallisation unter n-Heptan.
   Farbloses bis schwach gelbes kristallines Produkt,
   Schmp. 96 - 98 °C.
c) 6-Fluor-7-pyrrolidino-2,2-dimethyl-4-chromanonoxim
   erhält man analog der in Beispiel 1 a) angegebenen Vorschrift aus 6-Fluor-7-pyrrolidino-2,2-dimethyl-4-chromanon und Hydroxylamin-hydrochlorid. Kristallines Produkt,
   Schmp. 148 - 152 °C.
d) 6-Fluor-7-pyrrolidino-2,2-dimethyl-4-aminochroman-dihydrochlorid
   erhält man analog der in Beispiel 1 b) angegebenen Vorschrift durch katalytische Hydrierung von 6-Fluor-7-pyrrolidino-2,2-dimethyl-4-chromanonoxim und Aufarbeitung in Gegenwart von Salzsäure.
   Farbloses kristallines Produkt
   Schmp.: 124 - 137°C unter Zersetzung.
e) 6-Fluor-7-pyrrolidino-2,2-dimethyl-4-N-ethylsulfonylaminochroman
   erhält man analog der in Beispiel 1 c) angegebenen Vorschrift durch Reaktion von 6-Fluor-7-pyrrolidino-2,2-dimethyl-4-aminochroman-dihydrochlorid und Ethansulfonsäurechlorid in Gegenwart von TEA in THF.
   Farbloses kristallines Produkt,
   Schmp. 157 - 159°C (aus Gemisch aus Diisopropylether und Methanol).
f) 4-(N-Ethylsulfonyl-N-methyl)amino-6-fluor-7-pyrrolidino-2,2-dimethylchroman erhält man analog der in Beispiel 2 angegebenen Vorschrift durch Umsetzung von 6-Fluor-7-pyrrolidino-2,2-dimethyl-4-N-ethylsulfonylaminochroman mit Natriumhydrid und Methyliodid.
   Farblose kristalline Substanz,
   Schmp. 136 - 138°C

### Beispiel 67: 4-(N-Ethylsulfonyl-N-methyl)amino-6-fluorchroman

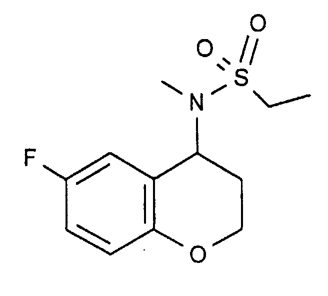

a) 6-Fluor-4-chromanonoxim
   erhält man analog der in Beispiel 1 a) angegebenen Vorschrift 6-Fluor-4-chromanon und Hydroxylamin-hydrochlorid.
   Kristallines Produkt,
   Schmp. 106-107°C.
b) 6-Fluor-4-aminochroman-hydrochlorid
   erhält man analog der in Beispiel 1 b) angegebenen Vorschrift durch katalytische Hydrierung von 6-Fluor-4-chromanonoxim und Aufarbeitung in Gegenwart von Salzsäure.
   Schmp.: 252°C (Zersetzung).
c) 6-Fluor-4-ethylsulfonylaminochroman
   erhält man analog der in Beispiel 1 c) angegebenen Vorschrift durch Reaktion von 6-Fluor-4-aminochroman-hydrochlorid und Ethansulfonsäurechlorid in Gegenwart von TEA in THF.
   Farblose kristalline Substanz,
   Schmp. 107 - 108°C.
d) 4-(N-Ethylsulfonyl-N-methyl)amino-6-fluorchroman
   erhält man analog der in Beispiel 2 angegebenen Vorschrift durch Umsetzung von 6-Fluor-4-ethylsulfonylaminochroman mit Natriumhydrid und Methyliodid.
   Farbloses bis blaßgelbes Öl.

### Beispiel 68: 4-(N-Butyl-N-ethylsulfonyl)amino-6-fluorchroman

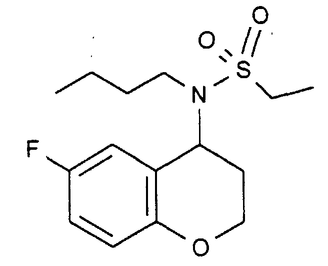

erhält man analog der in Beispiel 2 angegebenen Vorschrift durch Umsetzung 6-Fluor-4-ethylsulfonylaminochroman mit Natriumhydrid und Iodbutan.
Farbloses bis blaßgelbes Öl.

### Beispiel 69: 4-(N-Ethylsulfonyl-N-ethyl)amino-6-fluor-2,2-dimethylchroman

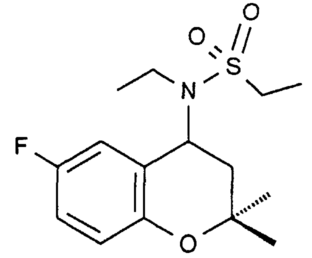

erhält man analog der in Beispiel 2 angegebenen Vorschrift aus 4-N-Ethylsulfonylamino-6-fluor-2,2-dimethylchroman und Ethyliodid.
Amorphes öliges Produkt.

### Beispiel 70: 4-(N-Ethylsulfonyl-N-propyl)amino-6-fluor-2,2-dimethylchroman

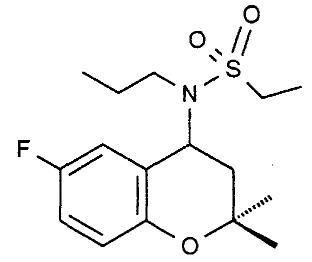

erhält man analog der in Beispiel 2 angegebenen Vorschrift aus 4-N-Ethylsulfonylamino-6-fluor-2,2-dimethylchroman und Propyliodid.
Amorphes öliges Produkt.

### Beispiel 71: 4-(N-Butyl-N-ethylsulfonyl)amino-6-fluor-2,2-dimethylchroman

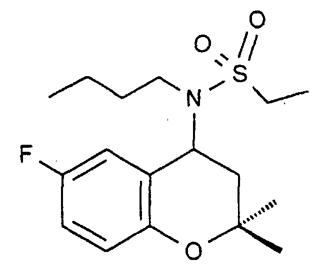

erhält man analog der in Beispiel 2 angegebenen Vorschrift aus 4-N-Ethylsulfonylamino-6-fluor-2,2-dimethylchroman und Butyliodid.
Amorphes öliges Produkt.

### Beispiel 72: 4-[N-Ethylsulfonyl-N-(4,4,4-triffuorbutyl)]amino-6-fluor-2,2-dimethylchroman

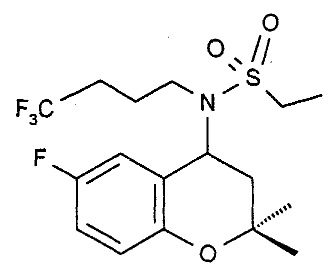

erhält man analog der in Beispiel 2 angegebenen Vorschrift aus 4-N-Ethylsulfonylamino-6-fluor-2,2-dimethylchroman und 4,4,4-Trifluorbutyliodid.
Amorphes öliges Produkt.

### Beispiel 73: 4-(N-Ethylsulfonyl-N-hexyl)amino-6-fluor-2,2-dimethylchroman

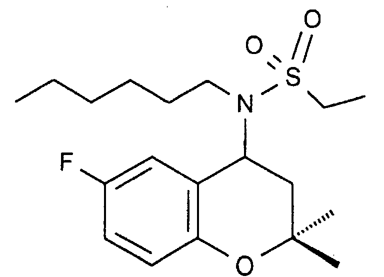

erhält man analog der in Beispiel 2 angegebenen Vorschrift aus 4-N-Ethylsulfonylamino-6-fluor-2,2-dimethylchroman und Hexyliodid.
Amorphes öliges Produkt.

### Beispiel 74: 4-(N-Ethylsulfonyl-N-methyl)amino-6-fluor-2,2-tetramethylenchroman

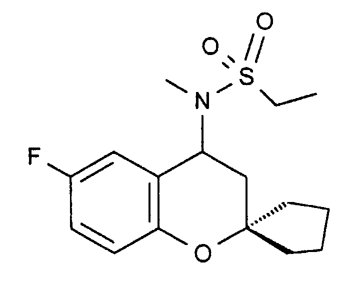

a) 6-Fluor-2,2-tetramethylen-4-chromanon
   erhält man analog der in Beispiel 18 b) angegebenen Vorschrift 5-Fluor-2-hydroxyacetophenon und Cyclopentanon in Gegenwart von Pyrrolidin in Acetonitril als Lösungsmittel.
   Amorphes braunes Produkt.
b) 6-Fluor-2,2-tetramethylen-4-chromanonoxim
   erhält man analog der in Beispiel 1 a) angegebenen Vorschrift aus 6-Fluor-2,2-tetramethylen-4-chromanon und Hydroxylamin-hydrochlorid.
   Farblose bis hellbraun gefärbte kristalline Substanz,
   Schmp. 107 - 110°C.
c) 4-Amino-6-fluor-2,2-tetramethylenchroman-hydrochlorid
   erhält man analog der in Beispiel 1 b) angegebenen Vorschrift durch katalytische Hydrierung 6-Fluor-2,2-tetramethylen-4-chromanonoxim und Aufarbeitung in Gegenwart von Salzsäure,
   Schmp.: 259 - 261 °C unter Zersetzung.
d) 4-Ethylsulfonylamino-6-fluor-2,2-tetramethylenchroman
   erhält man analog der in Beispiel 1 c) angegebenen Vorschrift aus 4-Amino-6-fluor-2,2-tetramethylenchroman-hydrochlorid und Ethansulfonsäurechlorid in Gegenwart von TEA in THF.
   Farblose kristalline Substanz,
   Schmp. 111 - 113°C.
e) 4-(N-Ethylsulfonyl-N-methyl)amino-6-fluor-2,2-tetramethylenchroman
   erhält man analog der in Beispiel 2 angegebenen Vorschrift aus 4-Ethylsulfonylamino-6-fluor-2,2-tetramethylenchroman und Methyliodid.
   Amorphes öliges Produkt.

### Beispiel 75: 4-[N-Ethylsulfonyl-N-(4,4,4-trifluorbutyl)]amino-6-fluor-2,2-tetramethylenchroman

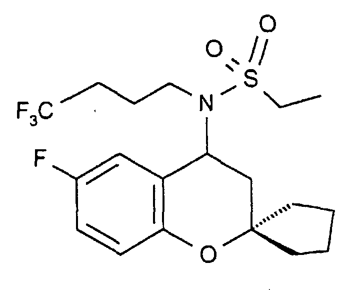

erhält man analog der in Beispiel 2 angegebenen Vorschrift aus 4-Ethylsulfonylamino-6-fluor-2,2-tetramethylenchroman und 4,4,4-Trifluorbutyliodid.
Visköses ölig-amorphes Produkt.

### Beispiel 76: 4-[N-Ethylsulfonyl-N-(4,4,4-trifluorbutyl)]amino-6-fluor-2,2-pentamethylenchroman

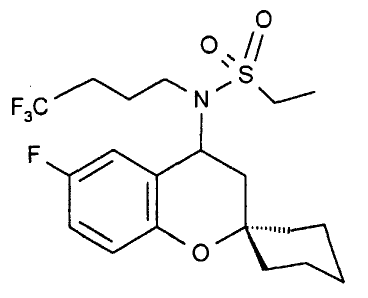

a) 6-Fluor-2,2-pentamethylen-4-chromanon
   erhält man analog der in Beispiel 18 b) angegebenen Vorschrift 5-Fluor-2-hydroxyacetophenon und Cyclohexanon in Gegenwart von Pyrrolidin in Acetonitril als Lösungsmittel.
   Helles amorphes Produkt.
b) 6-Fluor-2,2- pentamethylen-4-chromanonoxim
   erhält man analog der in Beispiel 1 a) angegebenen Vorschrift aus 6-Fluor-2,2-pentamethylen-4-chromanon und Hydroxylamin-hydrochlorid.
   Visköses amorphes Produkt.
c) 4-Amino-6-fluor-2,2- pentamethylenchroman-hydrochlorid
   erhält man analog der in Beispiel 1 b) angegebenen Vorschrift durch katalytische Hydrierung 6-Fluor-2,2- pentamethylen-4-chromanonoxim und Aufarbeitung in Gegenwart von Salzsäure,
   Schmp.: 262-264°C unter Zersetzung.
d) 4-Ethylsulfonylamino-6-fluor-2,2-pentamethylenchroman
   erhält man analog der in Beispiel 1 c) angegebenen Vorschrift durch 4-Amino-6-fluor-2,2-pentamethylenchroman-hydrochlorid und Ethansulfonsäurechlorid in Gegenwart von TEA in THF.
   Visköses amorphes Produkt.
e) 4-[N-Ethylsulfonyl-N-(4,4,4-trifluorbutyl)]amino-6-fluor-2,2-pentamethylenchroman
   erhält man analog der in Beispiel 2 angegebenen Vorschrift aus 4-Ethylsulfonylamino-6-fluor-2,2-pentamethylenchroman und 4,4,4-Trifluorbutyliodid.
   Amorphes öliges Produkt.

### Beispiel 77: 6-Ethyl-4-(N-ethylsulfonyl-N-methyl)amino-2,2-dimethylchroman

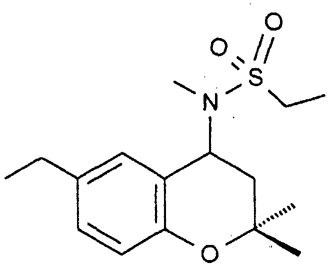

a) 5-Ethyl-2-hydroxyacetophenon
   erhält man analog der in Beispiel 63 b) angegebenen Vorschrift aus Essigsäure-(4-ethylphenyl)ester und wasserfreiem, aktiven Aluminiumchlorid.
   Schwach gelblich gefärbtes Öl.
   Der verwendete Essigsäure-(4-ethylphenyl)ester wird aus 4-Ethylphenol und Acetanhydrid analog der in Beispiel 63 a) ais Öl erhalten.
b) 6-Ethyl-2,2-dimethyl-4-chromanon
   erhält man analog der in Beispiel 18 b) angegebenen Vorschrift 5-Ethyl-2-hydroxyacetophenon und Aceton in Gegenwart von Pyrrolidin in Acetonitril als Lösungsmittel.
   Helles öliges amorphes Produkt.
c) 6-Ethyl-2,2-dimethyl-4-chromanonoxim
   erhält man analog der in Beispiel 1 a) angegebenen Vorschrift aus 6-Ethyl-2,2-dimethyl-4-chromanon und Hydroxylamin-hydrochlorid.
   Visköses öliges amorphes Produkt.
d) 4-Amino-6-ethyl-2,2-dimethylchroman-hydrochlorid
   erhält man analog der in Beispiel 1 b) angegebenen Vorschrift durch katalytische Hydrierung 6-Ethyl-2,2-dimethyl-4-chromanonoxim und Aufarbeitung in Gegenwart von Salzsäure,
   Schmp.: 201-204°C.
e) 6-Ethyl-4-N-ethylsulfonylamino-2,2-dimethylchroman
   erhält man analog der in Beispiel 1 c) angegebenen Vorschrift durch 4-Amino-6-ethyl-2,2-dimethylchroman-hydrochlorid und Ethansulfonsäurechlorid in Gegenwart von TEA in THF.
   Farblose kristalline Substanz,
   Schmp. 104 - 108°C.
f) 6-Ethyl-4-(N-ethylsulfonyl-N-methyl)amino-2,2-dimethylchroman
   erhält man analog der in Beispiel 2 angegebenen Vorschrift aus 6-Ethyl-4-N-ethylsulfonylamino-2,2-dimethylchroman und Methyliodid.
   Farbloses, kristallines Produkt,
   Schmp. 76 - 77°C.

### Beispiel 78: 6-Ethyl-4-[N-ethylsulfonyl-N-(4,4,4-trifluorbutyl)]amino-2,2-dimethylchroman

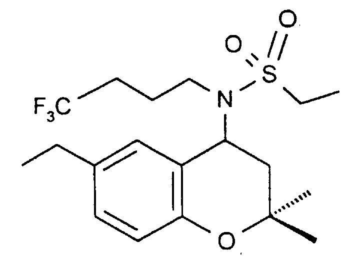

erhâlt man analog der in Beispiel 2 angegebenen Vorschrift aus 6-Ethyl-4-N-ethylsulfonylamino-2,2-dimethylchroman und 4,4,4-Trifluorbutyliodid.
Farbloses bis hellgelb gefärbtes Öl.

### Beispiel 79: 7-Chlor-4-[N-ethylsulfonyl-N-(4,4,4-trifluorbutyl)]amino-6-fluor-2,2-dimethylchroman

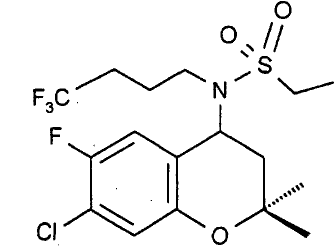

erhält man analog der in Beispiel 2 angegebenen Vorschrift durch Umsetzung von 7-Chlor-6-fluor-2,2-dimethyl-4-ethylsulfonylaminochroman mit Natriumhydrid und 4,4,4-Trifluorbutyliodid.
Visköses, hellgelbes Öl.

### Beispiel 80: 4-[N-Ethylsulfonyl-N-(4',4,4-trifluorbutyl)]amino-2,2,6-trimethylchroman

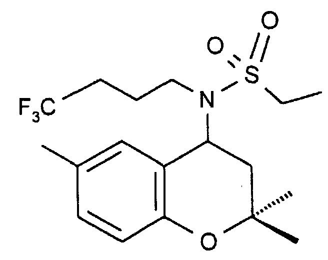

erhält man analog der in Beispiel 2 angegebenen Vorschrift durch Umsetzung von 4-Ethylsulfonylamino-2,2,6-trimethylchroman mit Natriumhydrid und 4,4,4-Trifluorbutyliodid.
Visköses, hellgelbes Öl.

### Beispiel 81: 6,7-Dichlor-4-[N-ethylsulfonyl- N-(4,4,4-trifluorbutyl)]amino -2,2-dimethylchroman

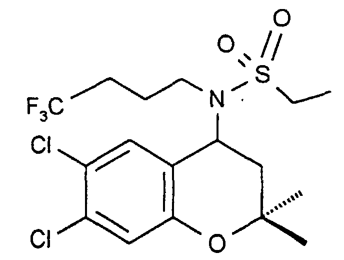

erhält man analog der in Beispiel 2 angegebenen Vorschrift durch Umsetzung 6,7-Dichlor-4-N-ethylsulfonylamino -2,2-dimethylchroman mit Natriumhydrid und 4,4,4-Trifluorbutyliodid.
Visköses, hellbraunes Öl.

### Beispiel 82: 4-(N-Ethylsulfonyl-N-methyl)amino-6-phenyl-2,2-dimethylchroman

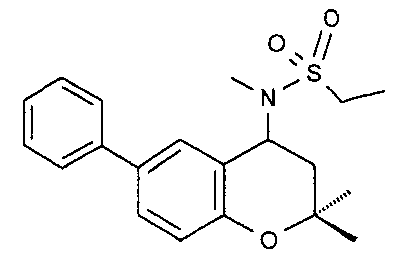

a) 2-Hydroxy-5-phenylacetophenon
   erhält man analog der in Beispiel 63 b) angegebenen Vorschrift aus 4-Acetoxybiphenyl und wasserfreiem, aktiven Aluminiumchlorid. Schwach gelblich gefärbtes Öl, das teilweise kristallisiert. Der verwendete 4-Acetoxybiphenyl wird aus 4-Hydroxybiphenyl und Acetanhydrid analog der in Beispiel 63 a) als farbloser kristalliner Feststoff erhalten.
   Fp 84 - 86°C
b) 2,2-Dimethyl-6-phenyl-4-chromanon
   erhält man analog der in Beispiel 18 b) angegebenen Vorschrift 2-Hydroxy-5-phenylacetophenon und Aceton in Gegenwart von Pyrrolidin in Acetonitril als Lösungsmittel.
   Dunkles öliges amorphes Produkt, das teilweise kristallisiert.
c) 2,2-Dimethyl-6-phenyl-4-chromanonoxim
   erhält man analog der in Beispiel 1 a) angegebenen Vorschrift aus 2,2-Dimethyl-6-phenyl-4-chromanon und Hydroxylamin-hydrochlorid. Kristalliner Feststoff,
   Fp. 130 - 134°C.
d) 4-Amino-2,2-dimethyl-6-phenylchroman-hydrochlorid
   erhält man analog der in Beispiel 1 b) angegebenen Vorschrift durch katalytische Hydrierung 2,2-Dimethyl-6-phenyl-4-chromanonoxim und Aufarbeitung in Gegenwart von Salzsäure,
   Schmp.: 213 - 214°C (Zersetzung).
e) 4-N-Ethylsulfonylamino-2,2-dimethyl-6-phenylchroman
   erhält man analog der in Beispiel 1 c) angegebenen Vorschrift durch 4-Amino-2,2-dimethyl-6-phenylchroman-hydrochlorid und Ethansulfonsäurechlorid in Gegenwart von TEA in THF.
   Farblose kristalline Substanz,
   Schmp. 162 - 164°C.
f) 4-(N-Ethylsulfonyl-N-methyl)amino-6-phenyl-2,2-dimethylchroman
   erhält man analog der in Beispiel 2 angegebenen Vorschrift aus 4-N-Ethylsulfonylamino-2,2-dimethyl-6-phenylchroman und Methyliodid.
   Farbloses, kristallines Produkt,
   Schmp. 184 - 186°C.

### Beispiel 83: 4-[N-ethylsulfonyl- N-(4,4,4-trifluorbutyl)]amino-6-phenyl-2,2-dimethylchroman

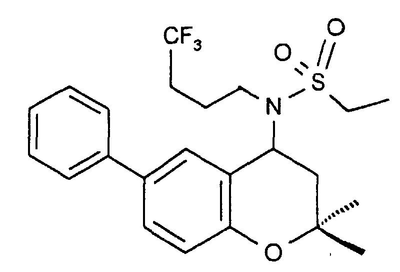

erhält man analog der in Beispiel 2 angegebenen Vorschrift durch Umsetzung 4-N-Ethylsulfonylamino-2,2-dimethyl-6-phenylchroman mit Natriumhydrid und 4,4,4-Trifluorbutyliodid.
Farblose bis hellgelbes kristalline Substanz,
Schmp. 112 - 114°C.

### Beispiel 84: 6,8-Difluor-4-(N-ethylsulfonyl-N-methyl)amino-2,2-dimethylchroman

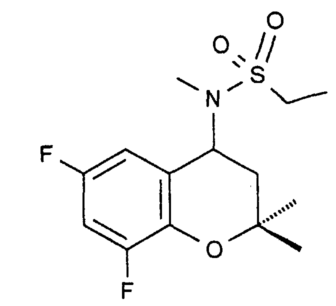

a) 3,5-Difluor-2-hydroxyacetophenon
   erhält man analog der in Beispiel 63 b) angegebenen Vorschrift aus Essigsäure-(2,4-difluorphenyl)ester und wasserfreiem, aktiven Aluminiumchlorid.
   Farbloser kristalliner Feststoff,
   Schmp 80-94°C.
   Der verwendete Essigsäure-(2,4-difluorphenyl)ester wird aus 2,4-Difluorphenol und Acetanhydrid analog der in Beispiel 63 a) beschriebenen Vorschrift als schwach gelblich gefärbte Flüssigkeit erhalten.
b) 6,8-Difluor-2,2-dimethyl-4-chromanon
   erhält man analog der in Beispiel 18 b) angegebenen Vorschrift 3,5-Difluor-2-hydroxyacetophenon und Aceton in Gegenwart von Pyrrolidin in Acetonitril als Lösungsmittel.
   Dunkles öliges amorphes Produkt.
c) 6,8-Difluor-2,2-dimethyl-4-chromanonoxim
   erhält man analog der in Beispiel 1 a) angegebenen Vorschrift aus 6,8-Difluor-2,2-dimethyl-4-chromanon und Hydroxylamin-hydrochlorid.
   Kristalliner Feststoff,
   Fp. 124 - 137°C.
d) 4-Amino-6,8-difluor-2,2-dimethylchroman-hydrochlorid
   erhält man analog der in Beispiel 1 b) angegebenen Vorschrift durch katalytische Hydrierung 6,8-Difluor-2,2-dimethyl-4-chromanonoxim und Aufarbeitung in Gegenwart von Salzsäure,
   Schmp.: > 310°C (Sublimation ab 300°C, 1 atm).
e) 4-N-Ethylsulfonylamino-6,8-difluor-2,2-dimethylchroman
   erhält man analog der in Beispiel 1 c) angegebenen Vorschrift durch 4-Amino-6,8-difluor-2,2-dimethylchroman-hydrochlorid und Ethansulfonsäurechlorid in Gegenwart von TEA in THF.
   Farblose kristalline Substanz.
   Schmp. 124 - 127°C.
f) 6,8-Difluor-4-(N-ethylsulfonyl-N-methyl)amino-2,2-dimethylchroman
   erhält man analog der in Beispiel 2 angegebenen Vorschrift aus 4-N-Ethylsulfonylamino-6,8-difluor-2,2-dimethylchroman und Methyliodid. Farbloses, kristallines Produkt,
   Schmp. 84 - 86°C.

### Beispiel 85: 6,8-Difluor-4-[N-ethylsulfonyl- N-(4,4,4-trifluorbutyl)]amino-2,2-dimethylchroman

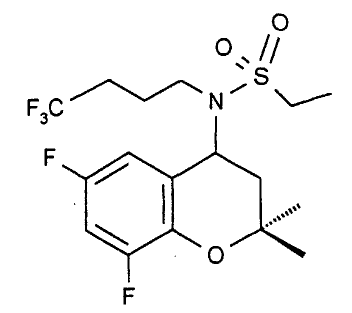

erhält man analog der in Beispiel 2 angegebenen Vorschrift aus 4-N-Ethylsulfonylamino-6,8-difluor-2,2-dimethylchroman und 4,4,4-Trifluorbutyliodid.
Farbloses, kristallines Produkt,
Schmp. 127 - 129°C.

### Beispiel 86:4-[N-Ethylsulfonyl- N-(4,4,4-trifluorbutyl)]amino -6-fluor-7-pyrrolidino-2,2-dimethylchroman (Referenzbeispiel)

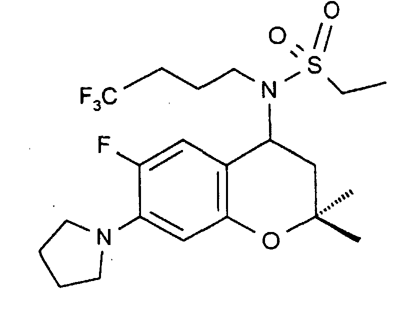

erhält man analog der in Beispiel 2 angegebenen Vorschrift durch Umsetzung von 6-Fluor-7-pyrrolidino-2,2-dimethyl-4-N-ethylsulfonylaminochroman mit Natriumhydrid und 4,4,4-Trifluorbutyliodid.
Farblose kristalline Substanz,
Schmp. 137 - 140°C

### Beispiel 87: 6-Carboxy-4-(N-ethyl-N-ethylsulfonyl)amino-2,2-dimethylchroman

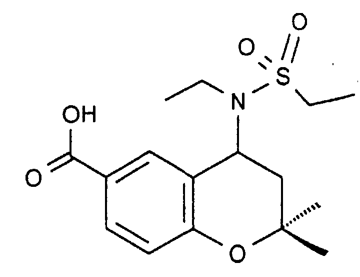

erhält man analog der in Beispiel 23 angegebenen Vorschrift durch Hydrolyse von 4-(N-Ethylsulfonyl-N-ethyl)amino-6-methoxycarbonyl-2,2-dimethylchroman.
Farblose Kristalline Verbindung,
Schmp. 217-220°C.

### Beispiel 88: 6-Carboxy-4-(N-ethylsulfonyl-N-propyl)amino-2,2-dimethylchroman

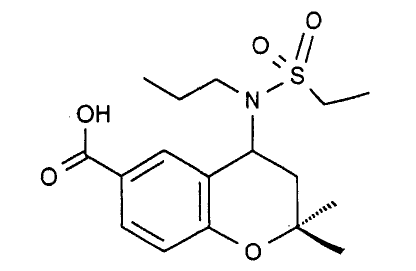

erhält man analog der in Beispiel 23 angegebenen Vorschrift durch Hydrolyse von 4-(N-ethylsulfonyl-N-propyl)amino-6-methoxycarbonyl-2,2-dimethylchroman.
Farblose kristalline Verbindung,
Schmp. 165-169°C.

### Beispiel 89: 6-Carboxy-4-(N-cyclopropylmethyl-N-ethylsulfonyl)amino-2,2-dimethylchroman

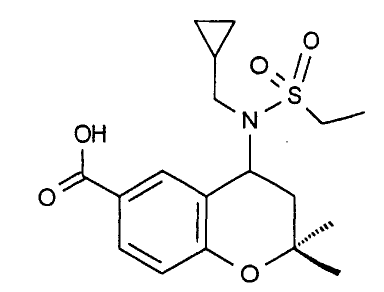

erhält man analog der in Beispiel 23 angegebenen Vorschrift durch Hydrolyse von 4-(N-cyclopropylmethyl-N-ethylsulfonyl)amino -6-methoxycarbonyl-2,2-dimethylchroman.
Farblose kristalline Verbindung,
Schmp. 184 - 188°C.

### Beispiel 90: 6-Carboxy-4-(N-ethylsulfonyl-N-pentyl)amino-2,2-dimethylchroman

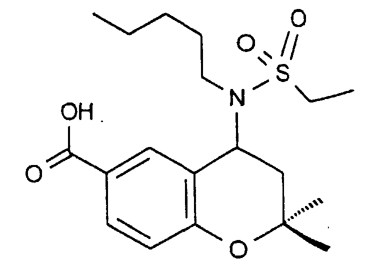

erhält man analog der in Beispiel 23 angegebenen Vorschrift durch Hydrolyse von 4-(N-ethylsulfonyl-N-pentyl)amino-6-methoxycarbonyl-2,2-dimethylchroman.
Farblose kristalline Verbindung,
Schmp. 156-158°C.

### Beispiel 91: 6-Carboxy-4-(N-ethylsulfonyl-N-hexyl)amino-2,2-dimethylchroman

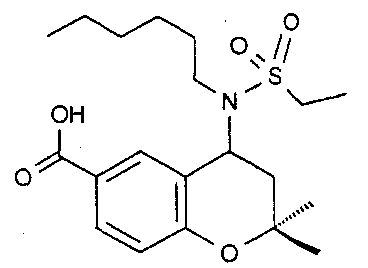

erhält man analog der in Beispiel 23 angegebenen Vorschrift durch Hydrolyse von 4(N-ethylsulfonyl-N- hexyl)amino-6-methoxycarbonyl-2,2-dimethylchroman.
Farblose kristalline Verbindung,
Schmp. 154-158°C.

### Beispiel 92: 6-Carboxamido-4-(N-ethyl-N-ethylsulfonyl)amino-2,2-dimethylchroman

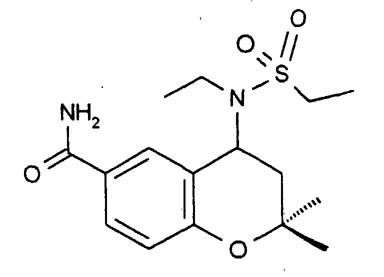

erhält man analog der in Beispiel 24 beschriebenen Vorschrift aus 6-Carboxy-4-(N-ethyl-N-ethylsulfonyl)amino-2,2-dimethylchroman, Carbonyldiimidazol und Ammoniak.
Farblose kristalline Substanz.
Schmp. 173 - 175°C.

### Beispiel 93: 6-Carboxamido-4-(N-ethylsulfonyl-N-propyl)amino-2,2-dimethylchroman

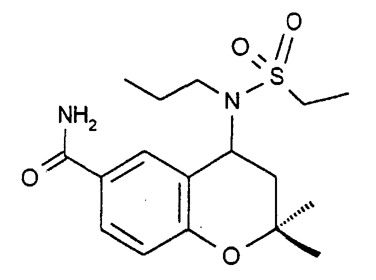

erhält man analog der in Beispiel 24 beschriebenen aus 6-Carboxy-4-(Nethylsulfonyl-N-propyl)amino-2,2-dimethylchroman, Carbonyldiimidazol und Ammoniak.
Farblose kristalline Substanz.
Schmp. 185 - 188°C.

### Beispiel 94: 6-Carboxamido-4-(N-cyclopropylmethyl-N-ethylsulfonyl)amino -2,2-dimethylchroman

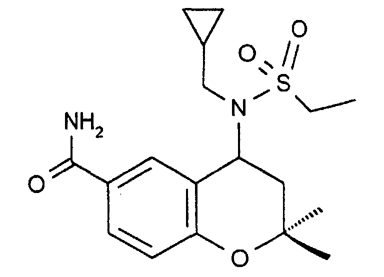

erhält man analog der in Beispiel 24 beschriebenen Vorschrift aus 6-Carboxy-4-(N-cyclopropylmethyl-N-ethylsulfonyl)amino-2,2-dimethylchroman,
Carbonyldiimidazol und Ammoniak.
Farblose kristalline Substanz.
Schmp. 196 - 199°C.

### Beispiel 95: 6-Carboxamido-4-(N-ethylsulfonyl-N-pentyl)amino-2,2-dimethylchroman

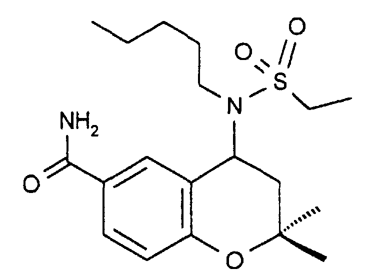

erhält man analog der in Beispiel 24 beschriebenen aus 6-Carboxy-4-(N-ethylsulfonyl-N-pentyl)amino-2,2-dimethylchroman, Carbonyldiimidazol und Ammoniak.
Farblose kristalline Substanz.
Schmp. 168 - 172°C.

### Beispiel 96: 6-Carboxamido-4-(N-ethylsulfonyl-N-hexyl)amino-2,2-dimethylchroman

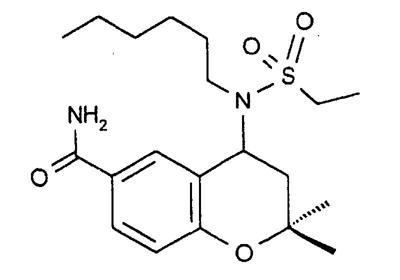

erhält man analog der in Beispiel 24 beschriebenen aus 6-Carboxy-4-(Nethylsulfonyl-N-hexyl)amino-2,2-dimethylchroman, Carbonyldiimidazol und Ammoniak.
Farblose kristalline Substanz.
Schmp. 148 - 152°C.

### Beispiel 97: 6-Cyano-4-(N-ethyl-N-ethylsulfonyl)amino-2,2-dimethylchroman

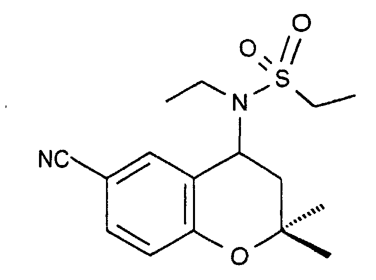

erhält man analog der in Beispiel 25 angegebenen Vorschrift aus 6-Carboxamido-4-(N-ethyl-N-ethylsulfonyl)amino-2,2-dimethylchroman und anschließende Reinigung durch Säulenchromatografie an Kieselgel mit einem Gemisch aus 10 Teilen Methylenchlorid und 1 Teil Methanol als Elutionsmittel.
Farblose bis blaßgelbe kristalline Substanz
Schmp.: 127 - 130°C.

### Beispiel 98: 6-Cyano-4-(N-ethylsulfonyl-N-propyl)amino-2,2-dimethylchroman

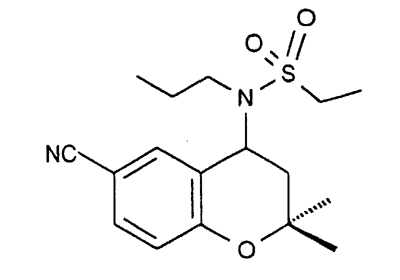

erhält man analog der in Beispiel 25 angegebenen Vorschrift aus 6-Carboxamido-4-(N-ethylsulfonyl-N-propyl)amino-2,2-dimethylchroman und anschließende Reinigung durch Säulenchromatografie an Kieselgel mit einem Gemisch aus 10 Teilen Methylenchlorid und 1 Teil Methanol als Elutionsmittel.
Farblose bis blaßgelbe kristalline Substanz,
Schmp.: 127 - 130°C.

### Beispiel 99: 6-Cyano-4-(N-cyclopropylmethyl-N-ethylsulfonyl)amino-2,2-dimethylchroman

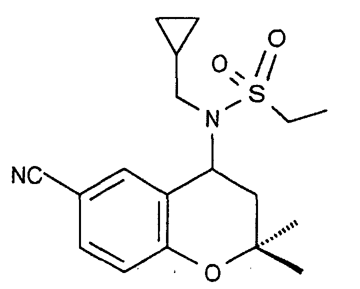

erhält man analog der in Beispiel 25 angegebenen Vorschrift aus 6-Carboxamido-4-(N-cyclopropylmethyl-N-ethylsulfonyl)amino-2,2-dimethylchroman und anschließende Reinigung durch Säulenchromatografie an Kieselgel mit einem Gemisch aus 10 Teilen Methylenchlorid und 1 Teil Methanol als Elutionsmittel.
Farblose bis blaßgelbe kristalline Substanz,
Schmp.: 127-130°C.

### Beispiel 100: 6-Cyano-4-(N-ethylsulfonyl-N-pentyl)amino-2,2-dimethylchroman

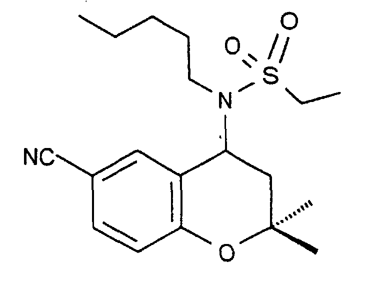

erhält man analog der in Beispiel 25 angegebenen Vorschrift aus 6-Carboxamido-4-(N-ethylsulfonyl-N-pentyl)amino-2,2-dimethylchroman und anschließende Reinigung durch Säulenchromatografie an Kieselgel mit einem Gemisch aus 10 Teilen Methylenchlorid und 1 Teil Methanol als Elutionsmittel.
Viskose ölige Flüssigkeit.

### Beispiel 101: 6-Cyano-4-(N-ethylsulfonyl-N-hexyl)amino-2,2-dimethylchroman

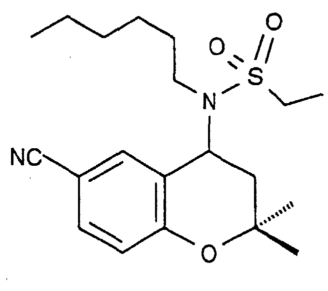

erhält man analog der in Beispiel 25 angegebenen Vorschrift aus 6-Carboxamido-4-(N-ethylsulfonyl-N-hexyl)amino-2,2-dimethylchroman und anschließende Reinigung durch Säulenchromatografie an Kieselgel mit einem Gemisch aus 10 Teilen Methylenchlorid und 1 Teil Methanol als Elutionsmittel.
Viskose gelbe ölige Flüssigkeit.

### Beispiel 102: 4-(N-Ethoxycarbonylmethyl-N-ethylsulfonyl)amino-6-fluor-2,2-dimethylchroman

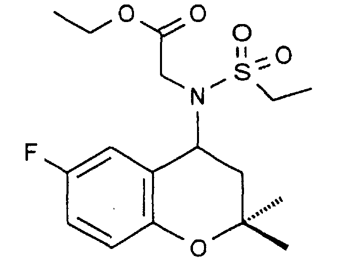

erhält man analog der in Beispiel 2 angegebenen Vorschrift aus 4-N-Ethylsulfonylamino-6-fluor-2,2-dimethylchroman und Bromessigsäureethylester.
Farbloses, kristallines Produkt,
Schmp. 112 - 114°C.

### Beispiel 103 : 4-[N-Ethylsulfonyl-N-methyl]amino-6-fluor-2,2-pentamethylenchroman

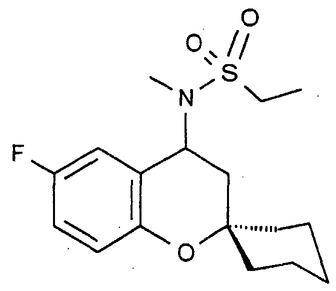

erhält man analog der in Beispiel 2 angegebenen Vorschrift aus 4-Ethylsulfonylamino-6-fluor-2,2-pentamethylenchroman und Methyliodid.
Farblose kristalline Verbindung,
Schmp. 73-74°C

### Beispiel 104: 4-(N-Isopropyloxycarbonylmethyl-N-ethylsulfonyl)amino-6-fluor-2,2-dimethylchroman

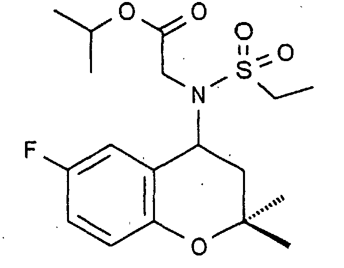

erhält man analog der in Beispiel 2 angegebenen Vorschrift aus 4-N-Ethylsulfonylamino-6-fluor-2,2-dimethylchroman und Bromessigsäureisopropylester.
Farblose bis hellgelbe Flüssigkeit:

### Beispiel 105: 4-(N-Ethylsulfonyl-N-methyl)amino-6-methoxy-2,2-dimethylchroman

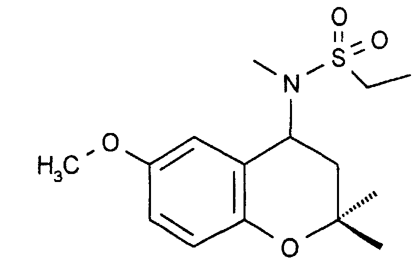

a) 6-Hydroxy-2,2-dimethyl-4-chromanon
   erhält man analog der in Beispiel 18 b) angegebenen Vorschrift 2,5-Dihydroxyacetophenon und Aceton in Gegenwart von Pyrrolidin in Acetonitril als Lösungsmittel.
   Kristalline Verbindung,
   Schmp. 147 - 149°C.
b) 6-Methoxy-2,2-dimethyl-4-chromanon
   Eine Suspension aus 0,03 M 6-Hydroxy-2,2-dimethyl-4-chromanon, 75 ml Aceton und 16,1 g wasserfreiem gepulvertem Kaliumcarbonat wird mit einem Überschuß von 3,6 ml Methyljodid versetzt und das Reaktionsgemisch für 20 Stunden auf 50 - 55°C erwärmt. Das Lösungsmittel wird entfernt durch Vakuumdestillation, der Rückstand mit Wasser versetzt und das sich abscheidende Öl mit Essigsäureethylester extrahiert. Nach Trocknung der organischen Phase über wasserfreiem Natriumsulfat destilliert man das Lösungsmittel ab und erhält 6-Methoxy-2,2-dimethyl-4-chromanon als ölige Flüssigkeit.
c) 6-Methoxy-2,2-dimethyl-4-chromanonoxim
   erhält man analog der in Beispiel 1 a) angegebenen Vorschrift aus 6-Methoxy-2,2-dimethyl-4-chromanon und Hydroxylamin-hydrochlorid.
   Kristalliner Feststoff,
   Fp. 108 - 112°C.
d) 4-Amino-6-methoxy-2,2-dimethylchroman-hydrochlorid
   erhält man analog der in Beispiel 1 b) angegebenen Vorschrift durch katalytische Hydrierung von 6-Methoxy-2,2-dimethyl-4-chromanonoxim und Aufarbeitung in Gegenwart von Salzsäure,
   Schmp.: 250 - 251 °C.
e) 4-N-Ethylsulfonylamino-6-methoxy-2,2-dimethylchroman
   erhält man analog der in Beispiel 1 c) angegebenen Vorschrift aus 4-Amino-6-methoxy-2,2-dimethylchroman-hydrochlorid und Ethansulfonsäurechlorid in Gegenwart von TEA in THF.
   Farblose kristalline Substanz,
   Schmp. 131 - 133°C.
f) 4-(N-Ethylsulfonyl-N-methyl)amino-6-methoxy-2,2-dimethylchroman
   erhält man analog der in Beispiel 2 angegebenen Vorschrift aus 4-N-Ethylsulfonylamino-6-methoxy-2,2-dimethylchroman und Methyliodid.
   Farbloses, kristallines Produkt,
   Schmp. 68 - 70°C.

### Beispiel 106: 4-N-Ethylsulfonylamino-2,2-dimethyl-6-methylsulfonyloxychroman

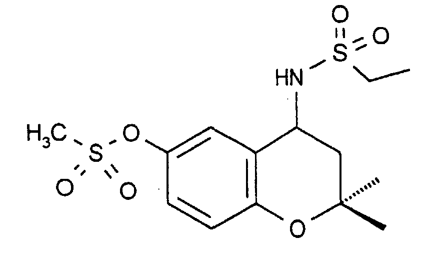

a) 2,2-Dimethyl-6-methylsulfonyloxychromanon
   Eine Mischung aus 0,03 M 6-Hydroxy-2,2-dimethyl-4-chromanon, 16,1 g wasserfreiem gepulverten Kaliumcarbonat und 10 ml Methansulfonsäure in 80 ml wasserfreiem DMF werden für 10 Stunden auf 80°C erwärmt. Sodann destilliert man das Lösungsmittel unter verminderten Druck ab und rührt den Rückstand nach Zugabe von 150 ml Wasser für 2 Stunden. Man filtriert den kristallinen Niederschlag, wäscht mehrfach mit Wasser und trocknet im Luftstrom.
   Farbloser Feststoff,
   Schmp. 108 - 110 °C
b) 2,2-Dimethyl-6-methylsulfonyloxychromanonoxim
   erhält man analog der in Beispiel 1 a) angegebenen Vorschrift aus 2,2-Dimethyl-6-methylsulfonyloxychromanon und Hydroxylamin-hydrochlorid.
   Kristalliner Feststoff,
   Fp. 166 - 167°C.
c) 4-Amino-6-methylsulfonyloxy-2,2-dimethylchroman-hydrochlorid
   erhält man analog der in Beispiel 1 b) angegebenen Vorschrift durch katalytische Hydrierung von 2,2-Dimethyl-6-methylsulfonyloxychromanonoxim und Aufarbeitung in Gegenwart von Salzsäure,
   Schmp.:229 - 231 °C.
e) 4-N-Ethylsulfonylamino-2,2-dimethyl-6-methylsulfonyloxychroman
   erhält man analog der in Beispiel 1 c) angegebenen Vorschrift aus 4-Amino-6-methylsulfonyloxy-2,2-dimethylchroman-hydrochlorid und Ethansulfonsäurechlorid in Gegenwart von TEA in THF.
   Farblose kristalline Substanz,
   Schmp. 97 - 100°C.

### Beispiel 107: 4-(N-Ethylsulfonyl-N-methyl)amino -2,2-dimethyl-6-methylsulfonyloxychroman

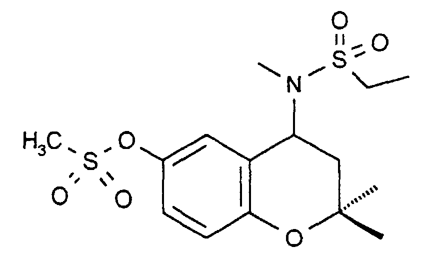

erhält man analog der in Beispiel 2 angegebenen Vorschrift aus 4-N-Ethylsulfonylamino-2,2-dimethyl-6-methylsulfonyloxychroman und Methyliodid. Farbloses,
kristallines Produkt,
Schmp. 137 - 139°C.

### Beispiel 108 : 4-(N-Methylsulfonyl-N-methyl)amino-2,2,6,7-tetramethylchroman

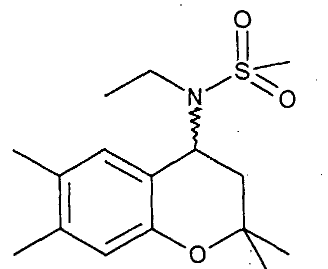

erhält man analog der in Beispiel 2 angegebenen Vorschrift aus 4-N-Methylsulfonylamino-2,2,6,7-tetramethylchroman und Ethyliodid.
Farblose Kristalle,
Schmp.: 119 - 121°C.

Das Hydrochlorid von 4-Amino-2,2,6,7-tetramethylchroman (Schmp. > 270°C)wurde hergestellt durch katalytische Hydrierung von 2,2,6,7-Tetramethyl-4-chromanon-oxime und anschließender Behandlung mit einer Lösung von Chlorwasserstoffgas in Diethylether (Schmp. 162 - 163°C). Das Oxim wurde erhalten aus 2,2,6,7-tetramethylchromanon und Hydroxylaminhydrochlorid analog der obigen beschriebenen Methoden zur Herstellung der Chromanonoxime.
Die Umsetzung von 4-Amino-2,2,6,7-tetramethylchroman mit den entsprechenden Alkylsulfonylchloriden, wie sie in Beispiel 1 (Variante 1) beschrieben ist, führte zu Methylsulfonylamino-2,2,6,7-tetramethylchroman (farbloses öliges Produkt) bzw. Ethylsulfonylamino-2,2,6,7-tetramethylchroman (farbloses öliges Produkt).

### Beispiel 109 : 4-(N-Methylsulfonyl-N-methyl)amino-2,2,6,7-tetramethylchroman

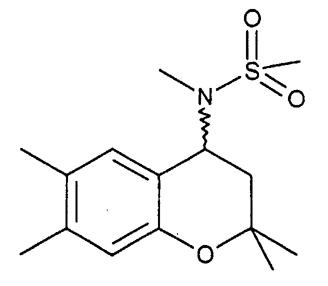

erhält man analog der in Beispiel 2 angegebenen Vorschrift aus 4-N-Methylsulfonylamino-2,2,6,7-tetramethylchroman und Methyliodid.
Farblose Kristalle
Schmp.: 105-107°C.

### Beispiel 110 : 4-(N-Ethylsulfonyl-N-hexyl)amino-2,2,6,7-tetramethylchroman

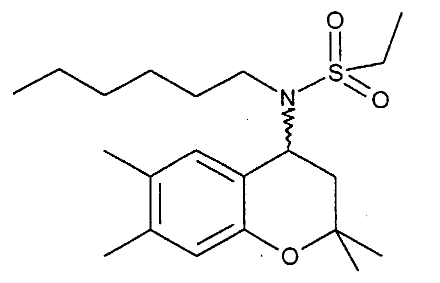

erhält man analog der in Beispiel 2 angegebenen Vorschrift aus 4-N-Ethylsulfonylamino-2,2,6,7-tetramethylchroman und Hexyliodid.
Farbloses, ölig-amorphes Produkt.

### Beispiel 111: 4-(N-Ethylsulfonyl-N-ethyl)amino-2,2,6,7-tetramethylchroman

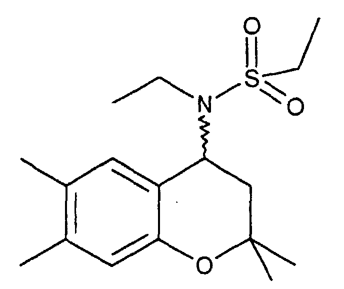

erhält man analog der in Beispiel 2 angegebenen Vorschrift aus 4-N-Ethylsulfonylamino-2,2,6,7-tetramethylchroman und Ethyliodid.
Farblose Kristalle,
Schmp.: 93 - 95°C.

### Beispiel 112: 4-(N-Ethylsulfonyl-N-butyl)amino-2,2,6,7-tetramethylchroman

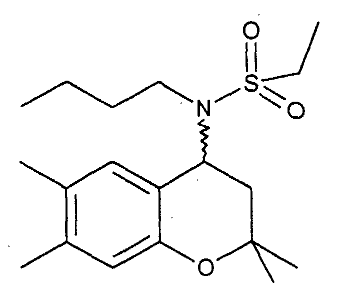

erhält man analog der in Beispiel 2 angegebenen Vorschrift aus 4-N-Ethylsulfonylamino-2,2,6,7-tetramethylchroman und Butyliodid.
Farblose Kristalle,
Schmp.: 81 - 83°C.

### Beispiel 113: 4-(N-Ethylsulfonyl-N-methyl)amino-2,2,6,7-tetramethylchroman

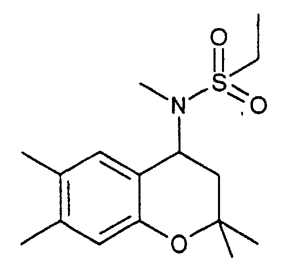

erhält man analog der in Beispiel 2 angegebenen Vorschrift aus 4-N-Ethylsulfonylamino-2,2,6,7-tetramethylchroman und Methyliodid.
Farblose Kristalle,
Schmp.: 132 - 134°C.

### Beispiel 114 : 4-(N-Ethylsulfonyl-N-butyl)amino-7-methoxy-2,2-dimethylchroman

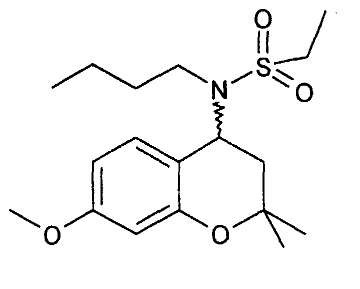

erhält man analog der in Beispiel 2 angegebenen Vorschrift aus 4-N-Ethylsulfonylamino-7-methoxy-2,2-dimethylchroman and Butyliodid.
Farbloses, viskoses Öl.

Das Hydrochlorid von 4-Amino-7-methoxy-2,2-dimethylchroman (Schmp. 239-241 °C) wurde hergestellt durch katalytische Hydrierung von 7-Methoxy-2,2-dimethylchromanoxim (Schmp. 124-126°C) und anschließender Behandlung mit einer Lösung von Chlorwasserstoffgas in Diethylether. Das Oxim wurde erhalten aus 7-Methoxy-2,2-dimethylchroman und Hydroxylaminhydrochlorid analog der obigen beschriebenen Methoden zur Herstellung der Chromanonoxime.
Die Umsetzung von 4-Amino-7-methoxy-2,2-dimethylchroman mit den entsprechenden Alkylsulfonylchloriden, wie sie in Beispiel 1 (Variante 1) beschrieben ist , führte zu Methylsulfonylamino-7-methoxy-2,2-dimethylchroman (farbloses öliges Produkt) bzw und Ethylsulfonylamino-7-methoxy-2,2-dimethylchroman (Schmp. 111 - 113°C).

### Beispiel 115 : 4-(N-Ethylsulfonyl-N-ethyl)amino-7-methoxy-2,2-dimethylchroman

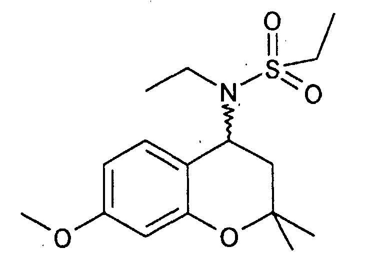

erhält man analog der in Beispiel 2 angegebenen Vorschrift aus 4-N-Ethylsulfonylamino-7-methoxy-2,2-dimethylchroman und Ethyliodid.
Farbloses, viskoses Oel.

### Beispiel 116 : 4-(N-Ethylsulfonyl-N-Methyl)amino-7-methoxy-2,2-dimethylchroman

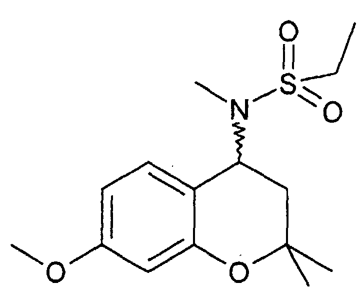

erhält man analog zum Verfahren nach Beispiel 2 aus 4-N-Ethylsulfonylamino-7-methoxy-2,2-dimethylchroman and Methyliodid.
Farbloses Öl.

### Beispiel 117: 4-(N-Ethylsulfonyl-N-methyl)amino-6-(4,4,4-trifluorbutyl)oxy-2,2-dimethylchroman

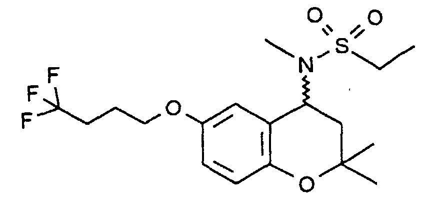

erhält man analog der in Beispiel 2 angegebenen Vorschrift aus 4-N-Ethylsulfonylamino-6-(4,4,4-trifluorbutyl)oxy-2,2-dimethylchroman and Methyliodid. Farblose Kristalle aus einer Mischung von n-Heptane /Diisopropylether. Schmp. 68 - 72 °C

4-N-Ethylsulfonylamino-6-(4,4,4-trifluorbutyl)oxy -2,2-dimethylchroman (Schmp. 84-90°C) erhält man in der Sequenz der einzelnen synthetischen Stufen, wie sie oben für analoge Beispiele angegeben sind ausgehend von 6-Hydroxy-2,2-dimethyl-4-chromanon (erhalten aus 2-Acetoxyhydrochinone und Acetone, Schmp. 147 - 149°C) über 6-(4,4,4-Trifluorbutyl)oxy-2,2-dimethyl-4-chromanon (erhalten aus 6-Hydroxy-2,2dimethyl-4-chromanon und 4,4,4-Trifluorobutyliodide, Schmp. 53-55°C) und 6-(4,4,4-Trifluorbutyl)oxy-2,2-dimethyl-4-chromanonoxime (erhalten aus 6-(4,4,4-Trifluorbutyl)oxy-2,2-dimethyl-4-chromanon und Hydroxylamine Hydrochloride, Schmp. 94 - 97 °C) und 4-Amino-6-(4,4,4-trifluorbutyl)oxy-2,2-dimethylchroman (erhalten aus 6-(4,4,4-Trifluorbutyl)oxy-2,2-dimethyl-4-chromanonoxim und katalytische Hydrierung mit Raney-Nickel, Schmp. 47 - 49°C) und nachfolgende Reaktion des 4-Amino-6-(4,4,4-trifluorbutyl)oxy-2,2-dimethylchroman mit Ethanesulfonylchloride.

### Beispiel 118: 6-(4-Bromophenyl)- 4-(N-ethylsulfonyl-N-methyl)amino-2,2-dimethylchroman

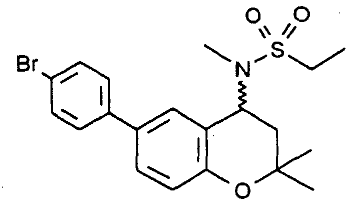

erhält man analog der in Beispiel 2 angegebenen Vorschrift aus 6-(4-Bromophenyl)- 4-N-ethylsulfonylamino-2,2-dimethylchroman und Methyliodid. Farblose Kristalle, Schmp. 160 - 170°C.

6-(4-Bromphenyl)- 4-N-ethylsulfonylamino-2,2-dimethylchroman (Schmp. 122 - 135°C) erhält man in der Sequenz der einzelnen synthetischen Stufen, wie sie oben für analoge Beispiele angegeben sind ausgehend von 3-acetyl-4'bromo-4-hydroxybiphenyl (erhalten aus 4'-Brom-4-acetoxybiphenyl and aluminium chloride in einer Friess Verschiebung, dunkles, braunes Öl) über 6-(4-Bromphenyl)- 2,2-dimethyl-4-chromanon (erhalten aus 3-Acetyl-4'-brom-4-hydroxybiphenyl und Acetone, visköses Öl) und 6-(4-Bromphenyl)- 2,2-dimethyl-4-chromanonoxime (erhalten aus 6-(4-Bromphenyl)- 2,2-dimethyl-4-chromanon, visköses Öl), und 6-(4-Bromphenyl)- 4-amino -2,2-dimethylchroman hydrochloride (erhalten aus 6-(4-Bromphenyl)- 2,2-dimethyl-4-chromanonoxim durch katalytische Hydrierung mit Raney-Nickel uns Behandlung des so erhaltenen Amins mit einer Lösung von HCl-Gas in Diethylether, Schmp. 166 - 170 °C) und nachfolgende Reaktion des 4-Amino-6-(4-bromophenyl)-2,2-dimethylchroman Hydrochlorides mit Ethanesulfonsäurechloride in Anwesenheit von Triethylamine.

### Beispiel 119: 4-(N-Ethylsulfonyl-N-methyl)amino-2,2-dimethyl-6-methoxychroman

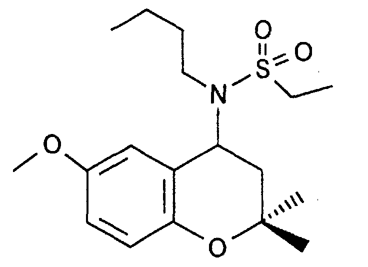

erhält man analog der in Beispiel 2 angegebenen Vorschrift aus 4-N-Ethylsulfonylamino-2,2-dimethyl-6-methoxychroman und Butyliodid. Farbloses, kristallines Produkt, Schmp. 78-80 °C

### Beispiel 120: Die optischen Isomeren (+)-4-(N-Ethylsulfonyl-N-methyl)amino-6-fluoro-2,2-dimethylchroman und (-)-4-(N-Ethylsulfonyl-N-methyl)amino-6-fluoro-2,2-dimethylchroman ([alpha] = -24,5°]

erhält man aus dem racemischen Gemisch von 4-(N-Ethylsulfonyl-N-methyl)amino-6-fluoro-2,2-dimethylchroman durch chirale Chromatographie (CSP Chiralpak AD 250*4.6, Elutions-Lösungsmittel: n-Hexan + Ethanol: 40 + 1).

### Beispiel 121: Die optischen Isomeren (+)-4-(N-Butyl N-ethylsulfonyl)amino-6-fluoro-2,2-dimethylchroman und (-)-4-(N-Butyl N-ethylsulfonyl)amino-6-fluoro-2,2-dimethylchroman ([alpha] = -53.5°]

erhält man aus dem racemischen Gemisch von 4-(N-Butyl N-ethylsulfonyl)amino-6-fluoro-2,2-dimethylchroman durch chirale Chromatographie (CSP Chiralpak AD 250*4.6, Elutionsmittel: n-Hexan + Ethanol: 80 + 1).

### Beispiel 122: 4-(N-Methylsulfonyl-N-isopropyl)amino -2,2,6-trimethylchroman

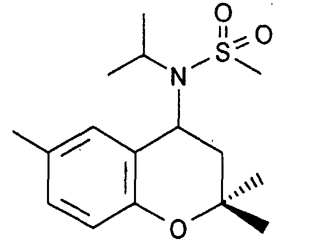

erhält man analog der in Beispiel 2 angegebenen Vorschrift aus 4-N-Methylsulfonylamino-2,2,6-trimethylchroman und Isopropyliodide. Farbloses kristallines Produkt,
Schmp. 140 °C.

### Beispiel 123: 4-[N-Methylsulfonyl-N-(3-methylbutyl)]amino -2,2,6-trimethylchroman

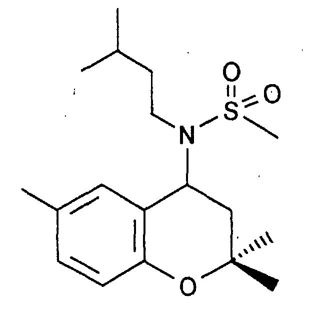

erhält man analog der in Beispiel 2 angegebenen Vorschrift aus 4-N-Methylsulfonylamino-2,2,6-trimethylchroman und 3-Methylbutyliodid. Viskoses Öl.

### Beispiel 124: 4-[N-Ethylsulfonyl-N-(3-ethoxypropyl)]amino -2,2,6-trimethylchroman

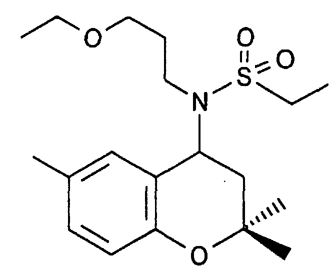

erhält man analog der in Beispiel 2 angegebenen Vorschrift aus 4-N-Ethylsulfonylamino-2,2,6-trimethylchroman und 3-Ethoxypropyliodide. Visköses Öl.

### Pharmakologische Untersuchungen:

I_{sK} Kanäle aus Mensch, Ratte oder Meerschweinchen wurden in Xenopus Oozyten exprimiert. Hierfür wurden zuerst Oozyten aus Xenopus Laevis isoliert und defollikuliert. Anschließend wurden diese Oozyten mit in vitro synthetisierter I_{sK} kodierender RNA injiziert. Nach 2 - 8 Tagen I_{sK} Proteinexpression wurden mit der Zwei-Mikroelektroden Voltage-Clamp Technik I_{sK} Ströme gemessen. I_{sK} Kanäle wurden hierbei in der Regel mit 15 s dauernden Spannungssprüngen auf -10 mV aktiviert und das Bad wurde mit einer Kontrollösung der nachfolgenden Komposition (mM) durchspült: NaCl 96, KCI 2, CaCl₂ 1.8, MgCl₂ 1, HEPES 5 (titriert mit NaOH auf pH 7.5). Diese Experimente wurden bei Raumtemperatur durchgeführt. Die eingesetzte Software zur Datenerhebung und Analyse waren: Geneclamp Verstärker (Axon Instruments, Foster City, USA) und MacLab D/A Umwandler und Software (ADInstruments, Castle Hill, Australia). Chromanole wurden getestet, indem sie in unterschiedlichen Konzentrationen der Kontrollösung zugefügt wurden. Die Effekte der Chromanole wurden als prozentuale Inhibition des I_{sK} Kontrollstromes berechnet. Die Daten wurden anschließend mit der Hill-Gleichung extrapoliert um die IC₅₀ für die jeweiligen Substanzen zu bestimmen.

Die Daten sind gegeben als Durchschnittswerte mit Standardabweichung (S.E.M.). n stellt die Anzahl der durchgeführten Experimente dar. Statistische Signifikanz wurde durch den gepaarten Student's t-test ermittelt.

### Referenzen:

Busch AE, Kopp H-G, Waldegger S, Samarzija I, Süßbrich H, Raber G, Kunzelmann K, Ruppersberg JP and Lang F (1995) Inhibition of both exogenously expressed I_{sK} and endogenous K⁺ channels in Xenopus oocytes by isosorbide dinitrate. J Physiol 491: 735-741
Takumi T, Ohkubo H and Nakanishi S (1989) Cloning of a membrane protein that induces a slow voltage-gated potassium current. Science 242:1042-1045 Varnum MD, Busch AE, Bond CT, Maylie J and Adelman JP (1993) The minK channel underlies the cardiac potassium current and mediates species-specific responses to protein kinase [C Proc Natl Acad Sci USA 90:11528-115321.

| Verbindung | Beispiel-Nr | I_{sK} IC₅₀ [µM/I] |
|---|---|---|
| 6-Cyano-4-[N-ethylsulfonyl-N-(4,4,4-trifuorbutyl)]amino-2,2-dimethylchroman | 33 | 0,42 |
| 4-(N-Butyl-N-ethylsulfonyl)amino-6-cyano-2,2-dimethylchroman | 38 | 0.76 |
| trans-6-Cyano-4-(N-ethylsulfonyl-N-methylamino)-3-hydroxy-2,2-dimethyl-chroman | 293B^{*)} | 6,9 ± 0,4 |
| trans-6-cyano-4-(N-methylsulfonyl-N-methylamino)-3-hydroxy-2,2-dimethyl-chroman | 374B^{*)} | 19,2 ± 1,2 |
| trans-6-Cyano-4-[N-(dimethylamino)sulfonyl-N-methylamino]-3-hydroxy-2,2-dimethyl-chroman | 377B^{*)} | 14,6 ± 0,5 |
| trans-6-Cyano-4-[N-(1-butylsulfonyl)-N-methylamino]-3-hydroxy-2,2-dimethyl-chroman | 360B^{*)} | 58,8 ± 1,8 |

| | | |
|---|---|---|
| ^{*)} E. Lohrmann, I. Burhoff, R.B. Nitschke, H.J. Lang, D. Mania, H.C. Englert, M. Hropot, R. Warth, W. Rohm, M. Bleich, R. Greger, Pflügers-Arch - Eur. J. Physiol (1995) 429: 517 - 530. | | |

### Hemmung der Magensäuresekretion, Antiulkuswirkung:

Methode: Die Hochdruckperfusion des Rattenmagens wurde nach der Beschreibung von Berglindh und Obrink (1) und unter Anwendung einiger Modifikationen, wie sie an anderer Stelle berichtet werden (2), ausgeführt. Kaninchen (männlich und weiblich, 2 - 3 kg) wurden schmerzfrei unter Narkose durch zervikale Dislokation getötet und der Magen wie in der Literatur berichtet perfundiert (1). Die Mukosa des Magenfundus wurde mit einem Schaber entfernt und mittels einer Schere zerkleinert. Die so erhaltenen Mukosafragmente wurden mit_1 mg/ml Kollagenase in einem Medium bestehend aus 100 mM NaCl, 5 mM KCI, 0,5 mM NaH₂PO₄, 1 mM Na₂HPO₄, 1 mM CaCl₂, 1,5 mM MgCl₂, 20 mM NaHCO₃, 20 mM HEPES, 2mg/ml Glukose und 1 mg/ml Kaninchenalbumin für 30 - 45 min bei 37°C behandelt, wobei der pH des Gemisches mit Tris-Puffer auf 7,4 eingestellt war. Die Drüsenschläuche (gastric glands) wurden durch ein Nylonnetz zur Entfernung grober Fragmente filtriert und 3 mal mit Inkubationsmedium gespült. Anschließend wurden die Drüsenschläuche im Medium in einer Konzentration von 2 - 4 mg Trockengewicht/ml suspendiert.

Als Maß für die Fähigkeit der gastrischen Drüsenschläuche zur Säurebildung wurde die Akkumulation von ¹⁴C-Aminopyrin (¹⁴C-AP) bestimmt (3). Hierzu wurden Proben von 1 ml Drüsenschlauchsuspension mit ¹⁴C-AP (1 µM, 200 000 cpm) und der zu testenden Verbindung inkubiert und für 20 - 30 min bei 37°C in einem schüttelnden Wasserbad behandelt. Anschließend wurden Histamin (100 µM), dbcAMP (0.3 oder 1 mM) oder Carbachol (100 µM) zugegeben, gefolgt von einer zweiten Inkubationsdauer von 30 - 45 min.. Die Inkubation wurde anschließend beendet durch Zentrifugiren der Proben für eine halbe Minute. Die überstehende Flüssigkeit wurde entfernt und die erhaltenen pellets in 1 ml NaOH gelöst. Proben der Pellets und des Flüssigkeitsüberstands wurden im Szintillationszähler gemessen. Das AP-Verhältnis der intraglandulären und extraglandulären Radioaktivität wurde nach Sack und Spenney (4) berechnet. Alle Bestimmungen wurden dreifach durchgeführt.

Ergebnis: 6-Cyano-4-(n-ethylsulfonyl-N-methyl)amino-2,2-dimethyl-3-chromanol verursachte eine konzentrationsabhängige Inhibition der stimulierten AP-Akkumulation mit IC₅₀-Werten von 20 µM nach Histamin- und dbcAMP - Stimulation, sowie von 5 µM nach Stimulation mit Carbachol.

### Literaturstellen:

1. Berghlind, T., Obrink, K. J. A method for preparing isolated glands from the rabbit gastric mucosa, Acta Physiol. Scan. 96, 150 159 (1976)
2. Herling, A. W., Becht, M., Kelker, W., Ljungstrom, M., Bickel, M., Inhibition of ¹⁴C-aminopyrine accumulation in isolated rabbit gastric glands by the H₂-receptor antagonist HOE 760 (TZU-0460). Agents and Actions 20: 35 - 39 (1987)
3. Berghlind, T., Helander, H. F., Obrink, K. J., effect of secretagogues on oxygen consumption, aminopyrine accumulation and morphology in isolated gastric glands. Acta Physiol. Scand. 97 401 - 414 (1976)
4. Sack, J., Spenney, J. G., Aminopyrine accumulation by mammalian gastric glands: an analysis of the technique. Am. J. Physiol. 243: G 313 - G 319 (1982).

## Patentansprüche

1. Chromane der Formel I 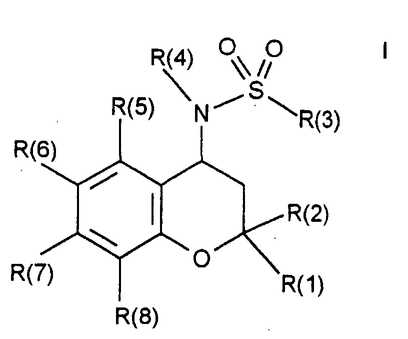 worin bedeuten:
R(1) und R(2)
unabhängig voneinander Wasserstoff, CₚF₂ₚ₊₁, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonylamino und Methylsulfonyl;
p 1, 2 oder 3;
oder
R(1) und R(2)
gemeinsam eine Alkylenkette mit 2, 3, 4, 5, 6, 7, 8, 9 oder 10 C-Atomen;
R(3) R(9)-CₙH₂ₙ[NR(11)]ₘ-;
R(9) Wasserstoff oder Cycloalkyl mit 3, 4, 5, 6, 7 oder 8 C-Atomen;
n Null, 1, 2, 3, 4, 5, 6, 7, 8, 9 oder 10;
m Null oder 1;
R(11) Wasserstoff oder Alkyl mit 1, 2, 3, 4, 5 oder 6 C-Atomen;
oder
R(11) gemeinsam mit R(9) eine Alkylengruppe mit 1, 2, 3, 4, 5, 6, 7 oder 8 C-Atomen;
wobei eine CH₂-Gruppe der Gruppe CₙH₂ₙ durch -O-, -SO_{q} oder -NR(10) ersetzt sein kann;
q Null, 1 oder 2;
R(10) Wasserstoff, Methyl oder Ethyl;
R(4) R(12)-CᵣH₂ᵣ;
R(12)
Wasserstoff, Cycloalkyl mit 3, 4, 5, 6, 7 oder 8 C-Atomen, Piperidyl, 1-Pyrrolidinyl, N-Morpholino, N-Methylpiperazino, CₚF₂ₚ₊₁, Pyridyl, Thienyl, Imidazolyl oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
r 1, 2, 3, 4, 5, 6, 7, 8, 9, 10, 11, 12, 13, 14, 15, 16, 17, 18, 19
oder 20;
wobei eine CH₂-Gruppe der Gruppe CᵣH₂ᵣ ersetzt sein kann durch -O-, -C=C-, -C≡C-, -CO-, -CO-O-, -SOq- oder -NR(10)-;
q Null, 1 oder 2;
R(10) Wasserstoff, Methyl oder Ethyl;
R(5), R(6), R(7) und R(8)
unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, -CN, -CF₃, -C₂F₅, -C₃F₇, -N₃, -NO₂, -CONR(13)R(14), -COOR(15), R(16)-CₛH₂ₛ-Y- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
R(13) und R(14)
unabhängig voneinander Wasserstoff oder Alkyl mit 1, 2 oder 3 C-Atomen;
R(15) Wasserstoff, Methyl, Ethyl, Phenyl oder -CᵤH₂ᵤ-NR(13)R(14);
u 2 oder 3;
R(16) Wasserstoff, Cycloalkyl mit 3, 4, 5, 6, 7 oder 8 C-Atomen, -COOR(15), Thienyl, Imidazolyl, Pyridyl, Chinolyl, Isochinolyl, Piperidyl, 1-Pyrrolidinyl, N-Morpholino, N-Methylpiperazino, CₜF₂ₜ₊₁ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
s Null, 1, 2, 3, 4, 5 oder 6;
t 1, 2 oder 3;
Y SO_{q}, -CO-, -SO₂-NR(10)-, -O-, -NR(10)- oder -CO-NR(10);
wobei jedoch R(6) nicht -OCF₃ oder -OC₂F₅ sein kann;
und deren physiologisch verträgliche Salze.

2. Chromane der Formel I nach Anspruch 1, **dadurch gekennzeichnet, daß** darin bedeuten:
R(1) und R(2)
unabhängig voneinander Wasserstoff, CF₃, Alkyl mit 1, 2 oder 3 C-Atomen, gemeinsam eine Alkylenkette mit 4 oder 5 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;;
R(3) R(9)-CₙH₂ₙ[NR(11)]ₘ-;
R(9) Wasserstoff;
n Null, 1, 2, 3, 4, 5 oder 6;
m Null oder 1;
R(11) Wasserstoff oder Alkyl mit 1, 2, 3 oder 4 C-Atomen;
R(4) R(12)-CᵣH₂ᵣ;
R(12)
Wasserstoff, Cycloalkyl mit 5, 6, 7 oder 8 C-Atomen, CF₃, Pyridyl oder Phenyl,
das unsubstituiert ist oder substituiert mit 1 oder 2 Substituenten ausgewählt aus der Gruppe bestehend aus F, Cl, CF₃, Sulfamoyl oder Methylsulfonyl;
r 1, 2, 3, 4, 5, 6, 7, 8, 9 oder 10;
wobei eine CH₂-Gruppe der Gruppe CᵣH₂ᵣ ersetzt sein kann durch -O-, -CO-, -CO-O- oder -SO_{q}-;
q Null, 1 oder 2;
R(5), R(6), R(7) und R(8)
unabhängig voneinander Wasserstoff, F, Cl, Br, I, Alkyl mit 1 oder 2 C-Atomen, -CN, -CF₃, -NO₂, -CONR(13)R(14), -COOR(15), R(16)-CₛH₂ₛ-Y- oder Phenyl,
das unsubstituiert ist oder substituiert mit einem Substituenten ausgewählt aus der Gruppe bestehend aus F, Cl und CF₃;
R(13) und R(14)
unabhängig voneinander Wasserstoff oder Alkyl mit 1, 2 oder 3 C-Atomen;
R(15) Methyl, Ethyl, Phenyl oder -CᵤH₂ᵤ-NR(13)R(14);
u 2 oder 3;
R(16) Wasserstoff, Cycloalkyl mit 5 oder 6 C-Atomen, CₜF₂ₜ₊₁ oder Phenyl,
das unsubstituiert ist oder substituiert mit einem Substituenten ausgewählt aus der Gruppe bestehend aus F, Cl, Br, CF₃, Methyl, Methoxy, Sulfamoyl oder Methylsulfonyl;
t 1, 2 oder 3;
s Null, 1, 2, 3 oder 4;
Y SO_{q}, -CO-, -SO₂-NR(10)-, -O-, -NR(10)- oder -CO-NR(10);
q Null, 1 oder 2;
R(10) Wasserstoff oder Methyl;
wobei jedoch R(6) nicht -OCF₃ oder -OC₂F₅ sein kann.

3. Chromane der Formel I nach Anspruch 1 oder 2, **dadurch gekennzeichnet, daß** darin bedeuten:
R(1) und R(2)
unabhängig voneinander CF₃, Methyl oder Phenyl,
das unsubstituiert ist oder substituiert durch 1 oder 2 Substituenten ausgewählt aus der Gruppe bestehend aus F, Cl, Br, I, CF₃, Methyl, Methoxy, Sulfamoyl und Methylsulfonyl;
R(3) Alkyl mit 1, 2, 3 oder 4 C-Atomen, Dimethylamino oder Diethylamino;
R(4) R(12)-CᵣH₂ᵣ;
R(12)
Wasserstoff, Cycloalkyl mit 5 oder 6 C-Atomen oder CF₃;
r 1, 2, 3, 4, 5, 6, 7 oder 8;
wobei eine CH₂-Gruppe der Gruppe CᵣH₂ᵣ ersetzt sein kann durch -O-, -CO-, -CO-O- oder -SO_{q}-;
q Null, 1 oder 2;
R(5), R(6), R(7) und R(8)
unabhängig voneinander Wasserstoff, F, Cl, Br, I, Alkyl mit 1 oder 2 C-Atomen, -CN, -NO₂, -COOR(15), R(16)-CₛH₂ₛ-Y- oder Phenyl,
das unsubstituiert ist oder substituiert mit einem Substituenten ausgewählt aus der Gruppe bestehend aus F oder Cl;
R(15) Methyl, Ethyl, Phenyl oder -CᵤH₂ᵤ-NR(13)R(14);
u 2 oder 3;
R(13) und R(14)
unabhängig voneinander Wasserstoff oder Alkyl mit 1, 2 oder 3 C-Atomen;
R(16) Wasserstoff, CF₃ oder Phenyl,
s Null, 1, 2, 3 oder 4;
Y SO_{q}, -CO-, -SO₂-NR(10)-, -O-, -NR(10)- oder -CO-NR(10);
q Null, 1 oder 2;
R(10) Wasserstoff oder Methyl;
wobei jedoch R(6) nicht -OCF₃ sein kann.

4. Chromane der Formel I nach Ansprüchen 1 bis 3, **dadurch gekennzeichnet, daß** sie ausgewählt sind aus der Gruppe:
4-(N-Ethylsulfonyl-N-methyl)amino-6-fluor-2,2-dimethylchroman,
6-Cyano-4-(N-ethylsulfonyl-N-methyl)amino-2,2-dimethylchroman,
4-(N-Ethylsulfonyl-N-methyl)amino-6-methoxycarbonyl-2,2-dimethylchroman,
6-Cyano-4-[N-ethylsulfonyl-N-(4,4,4-trifuorbutyl)]amino-2,2-dimethylchroman,
4-(N-Butyl-N-ethylsulfonyl)amino-6-cyano--2,2-dimethylchroman,
4-(N-Ethylsulfonyl-N-methyl)amino-2,2,6-trimethylchroman,
7-Chlor-4-(N-ethylsulfonyl-N-methyl)amino-6-fluor-2,2-dimethylchroman,
6,7-Dichlor-4-(N-ethylsulfonyl-N-methyl)amino-2,2-dimethylchroman,
4-(N-Butyl-N-ethylsulfonyl)amino-6-fluor-2,2-dimethylchroman,
4-(N-Ethylsulfonyl-N-methyl)amino-6-fluor-2,2-tetramethylenchroman,
4-[N-Ethylsulfonyl-N-(4,4,4-trifluorbutyl)]amino-6-fluor-2,2-dimethylchroman,
4-(N-Ethylsulfonyl-N-hexyl)amino-6-fluor-2,2-dimethylchroman,
6-Ethyl-4-[N-ethylsulfonyl-N-(4,4,4-trifluorbutyl)]amino-2,2-dimethylchroman,
4-(N-Ethoxycarbonylmethyl-N-ethylsulfonyl)amino-6-fluor-2,2-dimethylchroman,
4-(N-Ethylsulfonyl-N-methyl)amino-6-(4,4,4-trifluorbutyl)oxy-2,2-dimethylchroman,
6-(4-Bromophenyl)- 4-(N-ethylsulfonyl-N-methyl)amino-2,2-dimethylchroman,
4-[N-Ethylsulfonyl-N-(3-ethoxypropyl)]amino -2,2,6-trimethylchroman.

5. Verfahren zum Herstellen einer Verbindung der Formel I nach Anspruch 1, **dadurch gekennzeichnet, daß** man
a) eine Verbindung der Formel II 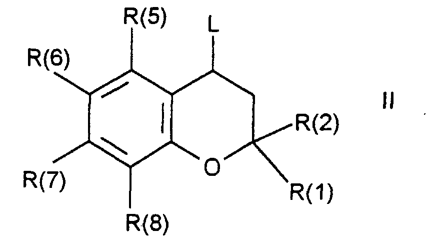 worin R(1), R(2), R(5), R(6), R(7) und R(8) die angegebene Bedeutung besitzen und L die für eine Alkylierung übliche nucleofuge leaving group, insbesondere Cl, Br, I, MeSO₂-O-, einen p-Toluolsulfonyloxy-Rest, bedeutet,
mit einem Sulfonamid oder dessen Salz der Formel III 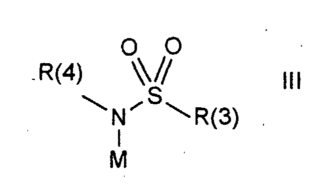 umsetzt, worin R(3) und R(4) die angegebene Bedeutung besitzen und M für Wasserstoff oder vorzugsweise für ein Metallatom, besonders bevorzugt für Lithium, Natrium oder Kalium steht.
oder daß man
b) eine Verbindung der Formel IV 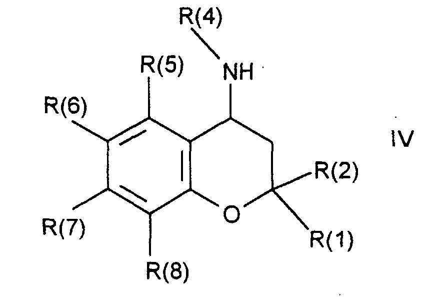 worin R(1), R(2), R(4), R(5), R(6), R(7) und R(8) die angegebene Bedeutung besitzen,
mit einem Sulfonsäure-Derivat der Formel V
R(3) - SO₂-W V
umsetzt, worin R(3) die angegebene Bedeutung besitzt und W eine nucleofuge leaving group, wie Fluor, Brom, 1-Imidazolyl, insbesondere aber Chlor bedeutet,
oder daß man
c) eine Verbindung der Formel VI 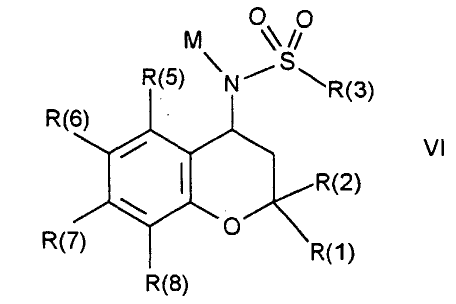 worin R(1), R(2), R(5), R(6), R(7), R(8) und M die angegebene Bedeutung besitzen,
mit einem Alkylierungsmittel der Formel VII
R(4)-L VII
im Sinne einer Alkylierungsreaktion umsetzt, worin R(4) mit Ausnahme von Wasserstoff sowie L die angegebene Bedeutung besitzen;
oder daß man
d) in einer Verbindung der Formel I 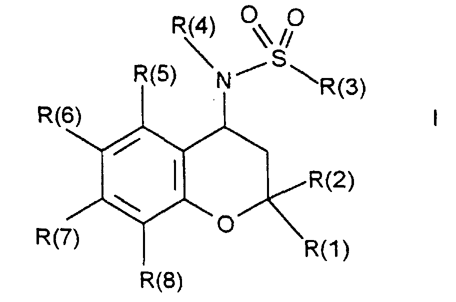
worin R(1) bis R(4) die angegebene Bedeutung besitzen, in mindestens einer Position R(5) bis R(8) eine elektrophile Substitutionsreaktion durchführt, sofern diese Position Wasserstoff bedeutet und die restlichen Substituenten R(5) bis R(8) die angegebene Bedeutung besitzen.

6. Verwendung einer Verbindung der Formel I nach Anspruch 1 zum Herstellen eines Medikaments zum Blockieren des K⁺-Kanals, der durch cyclisches Adenosinmonophosphat (cAMP) geöffnet wird.

7. Verwendung einer Verbindung der Formel I nach Anspruch 1 zum Herstellen eines Medikaments zum Inhibieren Magensäuresekretion.

8. Verwendung einer Verbindung der Formel I nach Anspruch 1 zum Herstellen eines Medikaments zur Behandlung von Ulcera des Magens und des intestinalen Bereiches, insbesondere des Duodenums.

9. Verwendung einer Verbindung der Formel I nach Anspruch 1 zum Herstellen eines Medikaments zur Behandlung der Refluxoesophagitis.

10. Verwendung einer Verbindung der Formel I nach Anspruch 1 zum Herstellen eines Medikaments zur Behandlung von Durchfall-Erkrankungen.

11. Verwendung einer Verbindung der Formel I nach Anspruch 1 zum Herstellen eines Medikaments zur Behandlung und Verhinderung aller Typen von Arrhythmien einschließlich ventrikulärer und supraventrikulärer Arrhythmien.

12. Verwendung einer Verbindung der Formel I nach Anspruch 1 zum Herstellen eines Medikaments zur Kontrolle von Reentry-Arrhythmien und zur Verhinderung des plötzlichen Herztodes infolge von Kammerflimmerns.

13. Heilmittel, enthaltend eine wirksame Menge einer Verbindung der Formel I nach einem der Ansprüche 1 bis 4.

## Claims

1. A chroman of the formula 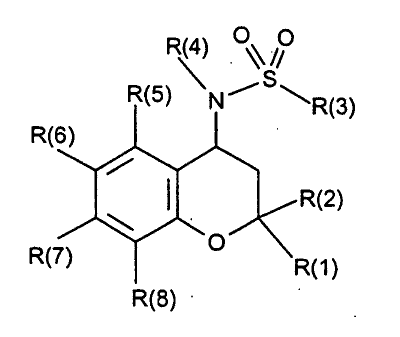 in which:
R(1) and R(2)
independently of one another are hydrogen, CₚF₂ₚ₊₁, alkyl having 1, 2, 3, 4, 5 or 6 carbon atoms or phenyl,
which is unsubstituted or substituted by 1 or 2 substituents selected from the group consisting of F, Cl, Br, I, CF₃, methyl, methoxy, sulfamoyl, methylsulfonylamino and methylsulfonyl;
p is 1, 2 or 3;
or
R(1) and R(2)
together are an alkylene chain having 2, 3, 4, 5, 6, 7, 8, 9 or 10 carbon atoms;
R(3) is R(9)-CₙH₂ₙ(NR(11)]ₘ-;
R(9) is hydrogen or cycloalkyl having 3, 4, 5, 6, 7 or 8 carbon atoms;
n is zero, 1, 2, 3, 4, 5, 6, 7, 8, 9 or 10;
m is zero or 1;
R(11) is hydrogen or alkyl having 1, 2, 3, 4, 5 or 6 carbon atoms; or
R(11) , together with R(9), is an alkylene group having 1, 2, 3, 4, 5, 6, 7 or 8 carbon atoms;
where one CH₂ group of the group CₙH₂ₙ can be replaced by -O-, -SO_{q} or -NR(10);
q is zero, 1 or 2;
R(10) is hydrogen, methyl or ethyl;
R(4) is R(12)-CᵣH₂ᵣ;
R(12)
is hydrogen, cycloalkyl having 3, 4, 5, 6, 7 or 8 carbon atoms, piperidyl, 1-pyrrolidinyl, N-morpholino, N-methylpiperazino, CₚF₂ₚ₊₁, pyridyl, thienyl, imidazolyl or phenyl,
which is unsubstituted or substituted by 1 or 2 substituents selected from the group consisting of F, Cl, Br, I, CF₃, methyl, methoxy, sulfamoyl, methylsulfonyl and methylsulfonylamino;
r is 1, 2, 3, 4, 5, 6, 7, 8, 9, 10, 11, 12, 13, 14, 15, 16, 17, 18, 19 or 20;
where one CH₂ group of the group CᵣH₂ᵣ can be replaced by -O-, -C=C-, -C≡C-, -CO-, -CO-O-, -SO_{q}- or -NR(10)-;
q is zero, 1 or 2;
R(10) is hydrogen, methyl or ethyl;
R(5), R(6), R(7) and R(8)
independently of one another are hydrogen, F, Cl, Br, I, alkyl having 1, 2, 3 or 4 carbon atoms, cycloalkyl having 3, 4, 5, 6, 7 or 8 carbon atoms, -CN, -CF₃, -C₂F₅, -C₃F₇, -N₃, -NO₂, -CONR(13)R(14), -COOR(15), R(16)-CₛH₂ₛ-Y- or phenyl,
which is unsubstituted or substituted by 1 or 2 substituents selected from the group consisting of F, Cl, Br, I, CF₃, methyl, methoxy, sulfamoyl and methylsulfonyl;
R(13) and R(14)
independently of one another are hydrogen or alkyl having 1, 2 or 3 carbon atoms;
R(15) is hydrogen, methyl, ethyl, phenyl or -CᵤH₂ᵤ-NR(13)R(14);
u is 2 or 3;
R(16) is hydrogen, cycloalkyl having 3, 4, 5, 6, 7 or 8 carbon atoms,
-COOR(15), thienyl, imidazolyl, pyridyl, quinolyl, isoquinolyl, piperidyl, 1-pyrrolidinyl, N-morpholino, N-methylpiperazino, CₜF₂ₜ₊₁ or phenyl,
which is unsubstituted or substituted by 1 or 2 substituents selected from the group consisting of F, Cl, Br, I, CF₃, methyl, methoxy, sulfamoyl or methylsulfonyl;
s is zero, 1, 2, 3, 4, 5 or 6;
t is 1, 2 or 3;
Y is SO_{q}, -CO-, -SO₂-NR(10)-, -O-, -NR(10)- or -CO-NR(10);
but where R(6) cannot be -OCF₃ or -OC₂F₅;
or its physiologically tolerable salts.

2. A chroman of the formula I as claimed in claim 1, wherein:
R(1) and R(2)
independently of one another are hydrogen, CF₃, alkyl having 1, 2 or 3 carbon atoms, jointly an alkylene chain having 4 or 5 carbon atoms or phenyl,
which is unsubstituted or substituted by 1 or 2 substituents selected from the group consisting of F, Cl, Br, I, CF₃, methyl, methoxy, sulfamoyl and methylsulfonyl;
R(3) is R(9)-CₙH₂ₙ[NR(11)]ₘ-;
R(9) is hydrogen;
n is zero, 1, 2, 3, 4, 5 or 6;
m is zero or 1;
R(11) is hydrogen or alkyl having 1, 2, 3 or 4 carbon atoms;
R(4) is R(12)-CᵣH₂ᵣ;
R(12)
is hydrogen, cycloalkyl having 5, 6, 7 or 8 carbon atoms, CF₃, pyridyl or phenyl,
which is unsubstituted or substituted by 1 or 2 substituents selected from the group consisting of F, Cl, CF₃, sulfamoyl or methylsulfonyl;
r is 1, 2, 3, 4, 5, 6, 7, 8, 9 or 10;
where one CH₂ group of the group CᵣH₂ᵣ can be replaced by -O-, -CO-, -CO-O- or -SO_{q}-;
q is zero, 1 or 2;
R(5), R(6), R(7) and R(8)
independently of one another are hydrogen, F, Cl, Br, I, alkyl having 1 or 2 carbon atoms, -CN, -CF₃, -NO₂, -CONR(13)R(14),
-COOR(15), R(16)-CₛH₂ₛ-Y- or phenyl,
which is unsubstituted or substituted by a substituent selected from the group consisting of F, Cl and CF₃;
R(13) and R(14)
independently of one another are hydrogen or alkyl having 1, 2 or 3 carbon atoms;
R(15) is methyl, ethyl, phenyl or -CᵤH₂ᵤ-NR(13)R(14);
u is 2 or 3;
R(16) is hydrogen, cycloalkyl having 5 or 6 carbon atoms, CₜF₂ₜ₊₁ or phenyl,
which is unsubstituted or substituted by a substituent selected from the group consisting of F, Cl, Br, CF₃, methyl, methoxy, sulfamoyl or methylsulfonyl;
t is 1, 2 or 3;
s is zero, 1, 2, 3 or 4;
Y is SO_{q}, -CO-, -SO₂-NR(10)-, -O-, -NR(10)- or -CO-NR(10);
q is zero, 1 or 2;
R(10) is hydrogen or methyl;
but where R(6) cannot be -OCF₃ or -OC₂F_{5.}

3. A chroman of the formula I as claimed in claim 1 or 2, wherein:
R(1) and R(2)
independently of one another are CF₃, methyl or phenyl, which is unsubstituted or substituted by 1 or 2 substituents selected from the group consisting of F, Cl, Br, I, CF₃, methyl, methoxy, sulfamoyl and methylsulfonyl;
R(3) is alkyl having 1, 2, 3 or 4 carbon atoms, dimethylamino or diethylamino;
R(4) is R(12)-CᵣH₂ᵣ;
R(12)
is hydrogen, cycloalkyl having 5 or 6 carbon atoms or CF₃;
r is 1, 2, 3, 4, 5, 6, 7 or 8;
where one CH₂ group of the group CᵣH₂ᵣ can be replaced by -O-, -CO-, -CO-O- or -SO_{q}-;
q is zero, 1 or 2;
R(5), R(6), R(7) and R(8)
independently of one another are hydrogen, F, Cl, Br, I, alkyl having 1 or 2 carbon atoms, -CN, -NO₂, -COOR(15), R(16)-CₛH₂ₛ-Y- or phenyl,
which is unsubstituted or substituted by a substituent selected from the group consisting of F or Cl;
R(15) is methyl, ethyl, phenyl or -CᵤH₂ᵤ-NR(13)R(14);
u is 2 or 3;
R(13) and R(14)
independently of one another are hydrogen or alkyl having 1, 2 or 3 carbon atoms;
R(16) is hydrogen, CF₃ or phenyl,
s is zero, 1, 2, 3 or 4;
Y is SO_{q}, -CO-, -SO₂-NR(10)-, -O-, -NR(10)- or -CO-NR(10);
q is zero, 1 or 2;
R(10) is hydrogen or methyl;
but where R(6) cannot be -OCF₃.

4. A chroman of the formula I as claimed in claims 1 to 3, which is selected from the group:
4-(N-ethylsulfonyl-N-methyl)amino-6-fluoro-2,2-dimethylchroman,
6-cyano-4-(N-ethylsulfonyl-N-methyl)amino-2,2-dimethylchroman,
4-(N-ethylsulfonyl-N-methyl)amino-6-methoxycarbonyl-2,2-dimethylchroman,
6-cyano-4-[N-ethylsulfonyl-N-(4,4,4-trifluorobutyl)]amino-2,2-dimethylchroman,
4-(N-butyl-N-ethylsulfonyl)amino-6-cyano-2,2-dimethylchroman,
4-(N-ethylsulfonyl-N-methyl)amino-2,2,6-trimethylchroman,
7-chloro-4-(N-ethylsulfonyl-N-methyl)amino-6-fluoro-2,2-dimethylchroman,
6,7-dichloro-4-(N-ethylsulfonyl-N-methyl)amino-2,2-dirnethylchroman,
4-(N-butyl-N-ethylsulfonyl)amino-6-fluoro-2,2-dimethylchroman,
4-(N-ethylsulfonyl-N-methyl)amino-6-fluoro-2,2-tetramethylenechroman,
4-[N-ethylsulfonyl-N-(4,4,4-trifluorobutyl)]amino-6-fluoro-2,2-dimethylchroman,
4-(N-ethylsulfonyl-N-hexyl)amino-6-fluoro-2,2-dimethylchroman,
6-ethyl-4-[N-ethylsulfonyl-N-(4,4,4-trifluorobutyl)]amino-2,2-dimethylchroman,
4-(N-ethoxycarbonylmethyl-N-ethylsulfonyl)amino-6-fluoro-2,2-dimethylchroman,
4-(N-ethylsulfonyl-N-methyl)amino-6-(4,4,4-trifluorobutyl)oxy-2,2-dimethylchroman,
6-(4-bromophenyl)-4-(N-ethylsulfonyl-N-methyl)amino-2,2-dimethylchroman,
4-[N-ethylsulfonyl-N-(3-ethoxypropyl)]amino-2,2,6-trimethylchroman.

5. A process for preparing a compound of the formula I as claimed in claim 1, which comprises
a) reacting a compound of the formula II 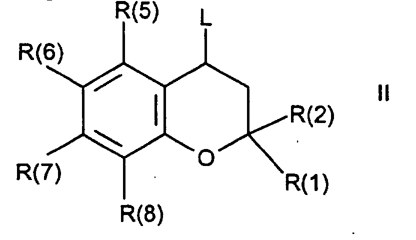 in which R(1), R(2), R(5), R(6), R(7) and R(8) have the meaning indicated and L is the nucleofugic leaving group customary for an alkylation, in particular Cl, Br, I, MeSO₂-O-, a p-toluenesulfonyloxy radical,
with a sulfonamide or its salt of the formula III 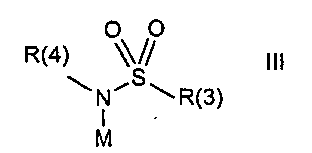 in which R(3) and R(4) have the meaning indicated and M is hydrogen or preferably a metal atom, particularly preferably lithium, sodium or potassium;
or by
b) reacting a compound of the formula IV 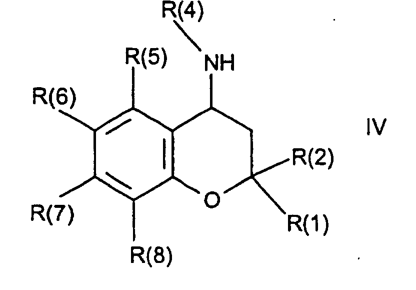 in which R(1), R(2), R(4), R(5), R(6), R(7) and R(8) have the meaning indicated,
with a sulfonic acid derivative of the formula V
R(3) - SO₂-W V
in which R(3) has the meaning indicated and W is a nucleofugic leaving group, such as fluorine, bromine, 1-imidazolyl, but in particular chlorine;
or by
c) reacting a compound of the formula VI 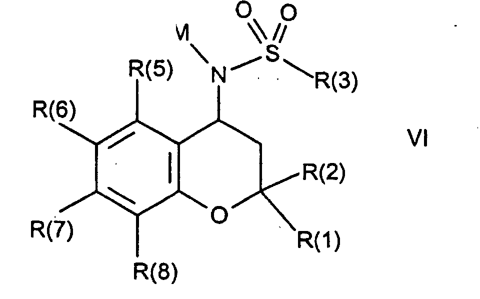 in which R(1), R(2), R(5), R(6), R(7), R(8) and M have the meaning indicated,
with an alkylating agent of the formula VII
R(4)-L VII
in the sense of an alkylation reaction, in which R(4), with the exception of hydrogen, and L have the meaning indicated;
or by carrying out
d) in a compound of the formula I 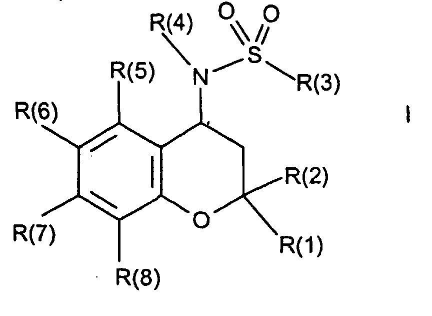 in which R(1) to R(4) have the meaning indicated, an electrophilic substitution reaction in at least one position R(5) to R(8), if this position is hydrogen and the remaining substituents R(5) to R(8) have the meaning indicated.

6. The use of a compound of the formula I as claimed in claim 1 for preparing a medicament for blocking the K⁺ channel which is opened by cyclic adenosine monophosphate (cAMP).

7. The use of a compound of the formula I as claimed in claim 1 for preparing a medicament for inhibiting gastric acid secretion.

8. The use of a compound of the formula I as claimed in claim 1 for preparing a medicament for the treatment of ulcers of the stomach and of the intestinal region, in particular of the duodenum.

9. The use of a compound of the formula I as claimed in claim 1 for preparing a medicament for the treatment of reflux esophagitis.

10. The use of a compound of the formula I as claimed in claim 1 for preparing a medicament for the treatment of diarrheal illnesses.

11. The use of a compound of the formula I as claimed in claim 1 for preparing a medicament for the treatment and prevention of all types of arrhythmias, including ventricular and supraventricular arrhythmias.

12. The use of a compound of the formula I as claimed in claim 1 for preparing a medicament for the control of reentry arrhythmias and for the prevention of sudden heart death as a result of ventricular fibrillation.

13. A therapeutic comprising an efficacious amount of a compound of the formula I as claimed in one of claims 1 to 4.

## Revendications

1. Chromanes de formule I 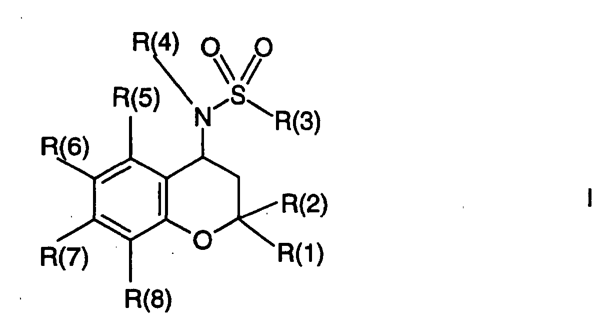 où :
R(1) et R(2) représentent indépendamment l'un de l'autre l'hydrogène, CₚF₂ₚ₊₁, alkyle ayant 1, 2, 3, 4, 5 ou 6 atomes de carbone ou phényle,
qui est non substitué ou substitué par 1 ou 2 substituants choisis dans le groupe consistant en F, CI, Br, I, CF₃, méthyle, méthoxy, sulfamoyle, méthylsulfonylamino et méthylsulfonyle ;
p représente 1, 2 ou 3 ;
ou bien
R(1) et R(2) représentent ensemble une chaîne alkylène ayant 2, 3, 4, 5, 6, 7, 8, 9 ou 10 atomes de carbone ;
R(3) représente R(9)-CₙH₂ₙ[NR(11)]ₘ- ;
R(9) représente l'hydrogène ou cycloalkyle ayant 3, 4, 5, 6, 7 ou 8 atomes de carbone ;
n représente 0, 1, 2, 3, 4, 5, 6, 7, 8, 9 ou 10 ;
m représente 0 ou 1 ;
R(11) représente l'hydrogène ou alkyle ayant 1, 2, 3, 4, 5 ou 6 atomes de carbone ;
ou bien
R(11) représente avec R(9) un groupe alkylène ayant 1, 2, 3, 4, 5, 6, 7 ou 8 atomes de carbone ;
où un groupe CH₂ du groupe CₙH₂ₙ peut être remplacé par -O-, -SOq ou -NR(10) ;
q représente 0, 1 ou 2 ;
R(10) représente l'hydrogène, méthyle ou éthyle ;
R(4) représente R(12)-CᵣH₂ᵣ ;
R(12) représente l'hydrogène, cycloalkyle ayant 3, 4, 5, 6, 7 ou 8 atomes de carbone, pipéridyle, 1-pyrrolidinyle, N-morpholino, N-méthylpipérazino, CₚF₂ₚ₊₁, pyridyle, thiényle, imidazolyle ou phényle,
qui est non substitué ou substitué par 1 ou 2 substituants choisis dans le groupe consistant en F, Cl, Br, I, CF₃, méthyle, méthoxy, sulfamoyle, méthylsulfonyle et méthylsulfonylamino ;
r représente 1, 2, 3, 4, 5, 6, 7, 8, 9, 10, 11, 12, 13, 14, 15, 16, 17, 18, 19 ou 20 ;
où un groupe CH₂ du groupe CᵣH₂ᵣ peut être remplacé par -O-, -C=C-, -C≡C-, -CO-, -CO-O-, -SO_{q}- ou -NR(10)- ;
q représente 0, 1 ou 2 ;
R(10) représente l'hydrogène, méthyle ou éthyle ;
R(5), R(6), R(7) et R(8) représentent indépendamment les uns des autres l'hydrogène, F, Cl, Br, I, alkyle ayant 1, 2, 3 ou 4 atomes de carbone, cycloalkyle ayant 3, 4, 5, 6, 7 ou 8 atomes de carbone, -CN, -CF₃, -C₂F₅, -C₃F₇, -N₃, -NO₂, -CONR(13)R(14), -COOR(15) ; R(16)-CₛH₂ₛ-Y- ou phényle,
qui est non substitué ou substitué par 1 ou 2 substituants choisis dans le groupe consistant en F, CI, Br, I, CF₃, méthyle, méthoxy, sulfamoyle et méthylsulfonyle ;
R(13) et R(14) représentent indépendamment l'un de l'autre l'hydrogène ou alkyle ayant 1, 2 ou 3 atomes de carbone ;
R(15) représente l'hydrogène, méthyle, éthyle, phényle ou -CᵤH₂ᵤ-NR(13)R(14) ;
u représente 2 ou 3 ;
R(16) représente l'hydrogène, cycloalkyle ayant 3, 4, 5, 6, 7 ou 8 atomes de carbone, -COOR(15), thiényle, imidazolyle, pyridyle, quinolyle, isoquinolyle, pipéridyle, 1-pyrrolidinyle, N-morpholino, N-méthylpipérazino, CₜF₂ₜ₊₁ ou phényle,
qui est non substitué ou substitué par 1 ou 2 substituants choisis dans le groupe consistant en F, Cl, Br, I, CF₃, méthyle, méthoxy, sulfamoyle ou méthylsulfonyle ;
s représente 0, 1, 2, 3, 4, 5 ou 6 ;
t représente 1, 2 ou 3 ;
Y représente SO_{q}, -CO-, -SO₂-NR(10)-, -O-, -NR(10)- ou -CO-NR(10) ;
où, cependant, R(6) ne peut pas être -OCF₃ ou -OC₂F₅ ;
et leurs sels physiologiquement acceptables.

2. Chromanes de formule I selon la revendication 1, **caractérisés en ce que** :
R(1) et R(2) représentent indépendamment l'un de l'autre l'hydrogène,
CF₃, alkyle ayant 1, 2 ou 3 atomes de carbone, ensemble une chaîne alkylène ayant 4 ou 5 atomes de carbonyle ou phényle,
qui est non substitué ou substitué par 1 ou 2 substituants choisis dans le groupe consistant en F, Cl, Br, I, CF₃, méthyle, méthoxy, sulfamoyle et méthylsulfonyle ;
R(3) représente R(9)-CₙH₂ₙ[NR(11)]ₘ- ;
R(9) représente l'hydrogène ;
n représente 0, 1, 2, 3, 4, 5 ou 6 ;
m représente 0 ou 1 ;
R(11) représente l'hydrogène ou alkyle ayant 1, 2, 3 ou 4 atomes de carbone ;
R(4) représente R(12)-CᵣH₂ᵣ ;
R(12) représente l'hydrogène, cycloalkyle ayant 5, 6, 7 ou 8 atomes de carbone, CF₃, pyridyle ou phényle,
qui est non substitué ou substitué par 1 ou 2 substituants choisis dans le groupe consistant en F, CI, CF₃, sulfamoyle ou méthylsulfonyle ;
r représente 1, 2, 3, 4, 5, 6, 7, 8, 9 ou 10 ;
où un groupe CH₂ du groupe CᵣH₂ᵣ peut être remplacé par -O-, -CO-, -CO-O- ou SO_{q}- ;
q représente 0, 1 ou 2 ;
R(5), R(6), R(7) et R(8) représentent indépendamment les uns des autres l'hydrogène, F, Cl, Br, I, alkyle ayant 1 ou 2 atomes de carbone, -CN, -CF₃, -NO₂, -CONR(13)R(14), -COOR(15), R(16)-CₛH₂ₛ-Y- ou phényle,
qui est non substitué ou substitué par un substituant choisi dans le groupe consistant en F, Cl et CF₃ ;
R(13) et R(14) représentent indépendamment l'un de l'autre l'hydrogène ou alkyle ayant 1, 2 ou 3 atomes de carbone ;
R(15) représente méthyle, éthyle, phényle ou -CᵤH₂ᵤ-NR(13)R(14) ;
u représente 2 ou 3 ;
R(16) représente l'hydrogène, cycloalkyle ayant 5 ou 6 atomes de carbone, CₜF₂ₜ₊₁ ou phényle,
qui est non substitué ou substitué par un substituant choisi dans le groupe consistant en F, Cl, Br, CF₃, méthyle, méthoxy, sulfamoyle ou méthylsulfonyle ;
t représente 1, 2 ou 3 ;
s représente 0, 1, 2, 3 ou 4 ;
Y représente SO_{q}, -CO-, -SO₂-NR(10)-, -O-, -NR(10)- ou -CO-NR(10) ;
q représente 0, 1 ou 2 ;
R(10) représente l'hydrogène ou méthyle ;
où, toutefois, R(6) ne peut pas être -OCF₃ ou -OC₂F₅.

3. Chromanes de formule I selon la revendication 1 ou 2, **caractérisés en ce que** :
R(1) et R(2) représentent indépendamment l'un de l'autre CF₃, méthyle ou phényle,
qui est non substitué ou substitué par 1 ou 2 substituants choisis dans le groupe consistant en F, CI, Br, I, CF₃, méthyle, méthoxy, sulfamoyle et méthylsulfonyle ;
R(3) représente alkyle ayant 1, 2, 3 ou 4 atomes de carbone, diméthylamino ou diéthylamino ;
R(4) représente R(12)-CᵣH₂ᵣ ;
R(12) représente l'hydrogène, cycloalkyle ayant 5 ou 6 atomes de carbone ou CF₃ ;
r représente 1, 2, 3, 4, 5, 6, 7 ou 8 ;
où un groupe CH₂ du groupe CᵣH₂ᵣ peut être remplacé par -O-, -CO-, -CO-O- ou -SO-_{q} ;
q représente 0, 1 ou 2 ;
R(5), R(6), R(7) et R(8) représentent indépendamment les uns des autres l'hydrogène, F, CI, Br, I, alkyle ayant 1 ou 2 atomes de carbone, -CN, -NO₂, -COOR(15) ; R(16)-CₛH₂ₛ-Y- ou phényle,
qui est non substitué ou substitué par un substituant choisi dans le groupe consistant en F ou Cl ;
R(15) représente méthyle, éthyle, phényle ou -CᵤH₂ᵤ-NR(13)R(14) ;
u représente 2 ou 3 ;
R(13) et R(14) représentent indépendamment l'un de l'autre l'hydrogène ou alkyle ayant 1, 2 ou 3 atomes de carbone ;
R(16) représente l'hydrogène, CF₃ ou phényle,
s représente 0, 1, 2, 3 ou 4 ;
Y représente SO_{q}, -CO-, -SO₂-NR(10)-, -O-, -NR(10)- ou -CO-NR(10) ;
q représente 0, 1 ou 2 ;
R(10) représente l'hydrogène ou méthyle ;
où, cependant, R(6) ne peut pas être -OCF₃.

4. Chromanes de formule I selon les revendications 1 à 3, **caractérisés en ce qu'**ils sont choisis dans le groupe suivant :
4-(N-éthylsulfonyl-N-méthyl)amino-6-fluoro-2,2-diméthylchromane,
6-cyano-4-(N-éthylsulfonyl-N-méthyl)amino-2,2-diméthylchromane,
4-(N-éthylsulfonyl-N-méthyl)amino-6-méthoxycarbonyl-2,2-diméthylchromane,
6-cyano-4-[N-éthylsulfonyl-N-(4,4,4-trifluorobutyl)]amino-2,2-diméthylchromane,
4-(N-butyl-N-éthylsulfonyl)amino-6-cyano-2,2-diméthylchromane,
4-(N-éthylsulfonyl-N-méthyl)amino-2,2,6-triméthylchromane,
7-chloro-4-(N-éthylsulfonyl-N-méthyl)amino-6-fluoro-2,2-diméthylchromane,
6,7-dichloro-4-(N-éthylsulfonyl-N-méthyl)amino-2,2-diméthylchromane,
4-(N-butyl-N-éthylsulfonyl)amino-6-fluoro-2,2-diméthylchromane,
4-(N-éthylsulfonyl-N-méthyl)amino-6-fluoro-2,2-tétraméthylènechromane,
4-[N-éthylsulfonyl-N-(4,4,4-trifluorobutyl)]amino-6-fluoro-2,2-diméthylchromane,
4-(N-éthylsulfonyl-N-hexyl)amino-6-fluoro-2,2-diméthylchromane,
6-éthyl-4-[N-éthylsulfonyl-N-(4,4,4-trifluorobutyl)]amino-2,2-diméthylchromane,
4-(N-éthoxycarbonylméthyl-N-éthylsulfonyl)amino-6-fluoro-2,2-diméthylchromane,
4-(N-éthylsulfonyl-N-méthyl)amino-6-(4,4,4-trifluorobutyl)oxy-2,2-diméthylchromane,
6-(4-bromophényl)-4-(N-éthylsulfonyl-N-méthyl)amino-2,2-diméthylchromane,
4-[N-éthylsulfonyl-N-(3-éthoxypropyl)]amino-2,2,6-triméthylchromane.

5. Procédé de préparation d'un composé de formule I selon la revendication 1, **caractérisé en ce que**
a) on fait réagir un composé de formule II 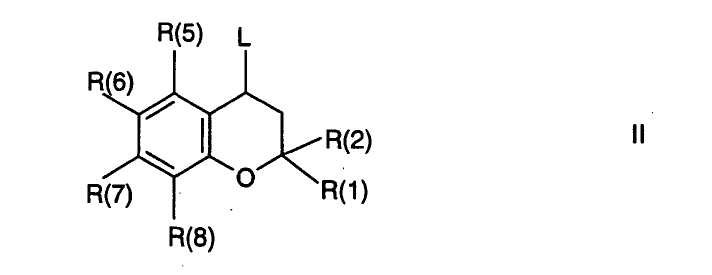 où R(1), R(2), R(5), R(6), R(7) et R(8) possèdent la signification indiquée et L représente un groupe partant nucléofuge courant pour une alkylation, en particulier Cl, Br, I, MeSO₂-O-, un reste p-toluènesulfonyloxy,
avec un sulfonamide ou son sel de formule III 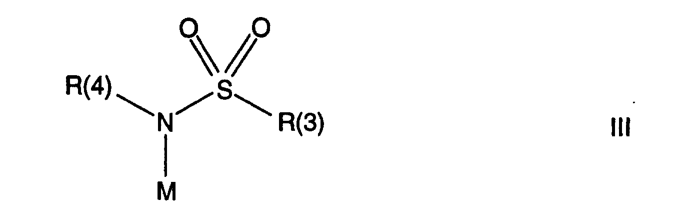 où R(3) et R(4) possèdent la signification indiquée et M représente l'hydrogène ou de préférence un atome de métal, de manière particulièrement préférée le lithium, le sodium ou le potassium,
ou **en ce que**
b) on fait réagir un composé de formule IV 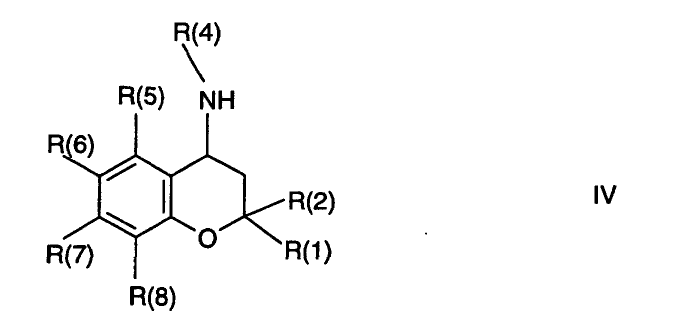 où R(1), R(2), R(4), R(5), R(6), R(7) et R(8) possèdent la signification indiquée avec un dérivé d'acide sulfonique de formule V
R(3)-SO₂-W V
où R(3) possède la signification indiquée et W représente un groupe partant nucléofuge comme le fluor, le brome, 1-imidazolyle, mais en particulier le chlore,
ou **en ce que**
c) on fait réagir un composé de formule VI 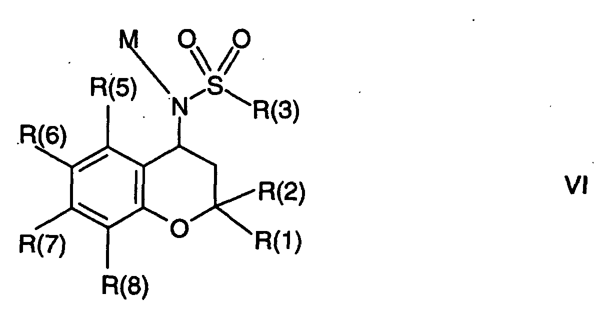 où R(1), R(2), R(5), R(6), R(7), R(8) et M possèdent la signification indiquée,
avec un agent d'alkylation de formule VII
R(4)-L VII
au sens d'une réaction d'alkylation, où R(4), à l'exception de l'hydrogène, et L possèdent la signification indiquée ;
ou **en ce que**
d) dans un composé de formule I 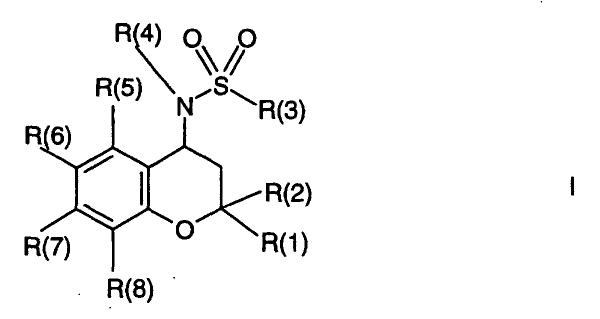
où R(1) à R(4) possèdent la signification indiquée, on réalise une réaction de substitution électrophile dans au moins une position R(5) à R(8), dans la mesure où cette position représente l'hydrogène et les autres substituants R(5) à R(8) possèdent la signification indiquée.

6. Utilisation d'un composé de formule I selon la revendication 1 pour la production d'un médicament pour bloquer le canal K⁺ qui est ouvert par l'adénosine monophosphate cyclique (AMPc).

7. Utilisation d'un composé de formule I selon la revendication 1 pour la production d'un médicament pour inhiber le sécrétion d'acide gastrique.

8. Utilisation d'un composé de formule I selon la revendication 1 pour la production d'un médicament pour le traitement des ulcères de l'estomac et du domaine intestinal, en particulier du duodénum.

9. Utilisation d'un composé de formule I selon la revendication 1 pour la production d'un médicament pour le traitement de l'oesophagite peptique.

10. Utilisation d'un composé de formule I selon la revendication 1 pour la production d'un médicament pour le traitement des maladies diarrhéiques.

11. Utilisation d'un composé de formule I selon la revendication 1 pour la production d'un médicament pour le traitement et la prévention de tous les types d'arythmies, y compris les arythmies ventriculaires et supra-ventriculaires.

12. Utilisation d'un composé de formule I selon la revendication 1 pour la production d'un médicament pour maîtriser les arythmies de réentrée et pour la prévention de la mort cardiaque subite à la suite de fibrillations ventriculaires.

13. Médicament contenant une quantité efficace d'un composé de formule I selon l'une des revendications 1 à 4.
